# EUROPEAN PATENT SPECIFICATION

(11) **EP 2 138 484 B1**
(45) Date of publication and mention of the grant of the patent: **26.08.2015**
(21) Application number: 08739652.9
(22) Date of filing: 02.04.2008
(51) Int. Cl.: C07D 235/12, A61K 31/4184, A61K 31/437, A61P 1/00, A61P 3/00, A61P 7/00, A61P 9/00, A61P 11/06, A61P 15/00, A61P 17/04, A61P 17/06, A61P 17/10, A61P 17/16, C07D 213/82, C07D 277/20, C07D 277/56, C07D 295/14, C07D 417/12, C07D 333/38

(54) **FUSED BICYCLIC HETEROARYL DERIVATIVES**
KONDENSIERTE BICYCLISCHE HETEROARYLDERIVATE
DÉRIVÉS D'HÉTÉRO-ARYLE BICYCLIQUE FUSIONNÉS

(30) Priority: 05.04.2007 JP 2007099413
(43) Date of publication of application: 30.12.2009
(73) Proprietor: Daiichi Sankyo Company, Limited, Tokyo 103-8426 (JP)
(72) Inventor: SHIMADA, Kousei, Tokyo 140-8710 (JP); ONISHI, Yoshiyuki, Tokyo 140-8710 (JP); MORI, Makoto, Tokyo 140-8710 (JP); TOKUMARU, Eri, Tokyo 140-8710 (JP)
(74) Representative: Fairbairn, Angus Chisholm
(86) International application number: PCT/JP2008/056541
(87) International publication number: WO 2008/126732

(56) References cited:
- WO-A1-00/59889
- WO-A1-2004/013109
- JP-A- 2000 351 769
- JP-A- 2002 193 948
- JP-A- 2004 067 629
- JP-A- 2004 315 404
- JP-A- 2005 523 292
- KAPOOR A. ET AL.: 'QSAR and molecular modeling studies in imidazopyridinethiazolidine -2, 4- diones: PPARgamma agonists' MEDICINAL CHEMISTRY RESEARCH vol. 13, no. 8/9, 2004, pages 770 - 780, XP019202360

## Description

### Technical Field

The present invention relates to a medicine, in particular, a novel fused bicyclic heteroaryl derivative or a pharmacologically acceptable salt thereof, which has a hypoglycemic effect or treats and/or prevents the onset of a disorder of carbohydrate or lipid metabolism or a disease mediated by peroxisome proliferator-activated receptor (PPAR) γ.

The present invention also relates to a therapeutic agent and/or prophylactic agent for diabetes (especially type II diabetes), hyperglycemia, hyperlipidemia, adiposity, impaired glucose tolerance, insulin resistance, impaired fasting glucose, cachexia, psoriasis, diabetic complications, arteriosclerosis, atherosclerosis, hypertension, pancreatitis, polycystic ovary syndrome, fatty liver, nonalcoholic steatohepatitis (NASH), gestational diabetes mellitus, inflammatory disease, cancer, osteoporosis, involutional osteoporosis, neurodegenerative disease, Alzheimer's disease, hyperuricemia, metabolic syndrome, or the like, which has an effect of improving carbohydrate or lipid metabolism, an effect of improving insulin resistance, an antiinflammatory effect or an effect of inhibiting the growth of cancer cells, the therapeutic agent and/or prophylactic agent comprising a novel fused bicyclic heteroaryl derivative or a pharmacologically acceptable salt thereof as an active ingredient.

### Background Art

In recent years, the number of patients with metabolic syndrome such as type II diabetes, hyperinsulinemia, dyslipidemia, adiposity, hypertension or atherosclerotic disease has been increasing around the world due to reasons such as changes in lifestyles. Patients with metabolic syndrome have a several-fold increased risk of coronary artery disease, cerebral infarction and cerebral hemorrhage and are further affected with chronic complications such as nephropathy, neuropathy and retinopathy. The increase in the number of patients with complications has been a major cause of rising medical costs (Non-Patent Document 1).

Recent researches have shown that ligands acting on PPARγ are useful for the prevention or improvement of a pathology called metabolic syndrome such as type II diabetes, hyperinsulinemia, dyslipidemia, adiposity, hypertension, atherosclerotic disease or insulin resistance (Non-Patent Document 2). Ligands acting on PPARγ inhibit the production of inflammatory cytokines (Non-Patent Documents 3 and 4) and induce apoptosis to inhibit the growth of cancer cells (Non-Patent Document 5). Therefore, the ligands are also useful for the prevention or improvement of inflammatory disease or cancer. Specific examples of the ligands activating PPARγ include pioglitazone (Non-Patent Document 6) and rosiglitazone (Non-Patent Document 7) classified into thiazolidinedione drugs already medically used in the treatment of type II diabetes. These thiazolidinedione drugs have side effects such as fluid retention, body weight increase and increased risks for heart disease. Therefore, safer pharmaceuticals have been desired to be developed (Patent Document 1). Many researchers have now been researching and developing pharmaceuticals with an aim to prevent or improve insulin resistance, diseases caused by inflammation or the like, or metabolic syndrome through researches of ligands activating or inhibiting PPARα, PPARγ or PPARδ (Non-Patent Document 8).
Non-Patent Document 1: Annual Reports in Medicinal Chemistry, 39, 41-56 (2004).
Non-Patent Document 2: Annual Reviews of Medicine, 53, 409-435 (2002).
Non-Patent Document 3: Nature, 391, 79-82 (1998).
Non-Patent Document 4: Nature, 391, 82-86 (1998).
Non-Patent Document 5: Biochemical and Biophysical Research Communications, 270, 400-405 (2000).
Non-Patent Document 6: Chem. Pharm. Bull., 39, 1440-1445 (1991).
Non-Patent Document 7: Bioorganic and Medicinal Chemistry Letter, 4, 1181-1184 (1994).
Patent Document 1: WO 2004/014308
Non-Patent Document 8: Annual Report in Medicinal Chemistry, 38, 71-80 (2003).

### Disclosure of the Invention

### Problems to be Solved by the Invention

The present inventors have conducted extensive studies to develop therapeutic agents and/or prophylactic agents for disorders of carbohydrate or lipid metabolism or diseases mediated by peroxisome proliferator-activated receptor (PPAR) γ. Thus, the inventors have found that fused bicyclic heteroaryl derivatives having a specific chemical structure have an excellent hypoglycemic effect or have an effect of improving carbohydrate or lipid metabolism, an effect of improving insulin resistance or an effect of improving so-called metabolic syndrome such as arteriosclerosis, hypertension, cardiovascular disorder or complications derived from them or a pathology caused by various inflammations. The inventors have further found that the compounds are ligands acting on PPARγ and therefore have an effect of inhibiting the growth of cancer cells. These findings have led to the completion of the present invention.

Specifically, the present invention provides novel fused bicyclic heteroaryl derivatives or pharmacologically acceptable salts thereof, which are useful as therapeutic agents or prophylactic agents for metabolic syndrome, specifically, diseases such as diabetes (especially type II diabetes), hyperglycemia, hyperlipidemia, adiposity, impaired glucose tolerance (IGT), insulin resistance, impaired fasting glucose (IFG), hypertension, fatty liver, nonalcoholic steatohepatitis (NASH), diabetic complications (such as retinopathy, nephropathy or neuropathy), arteriosclerosis, gestational diabetes mellitus (GDM) or polycystic ovary syndrome (PCOS), inflammatory disease (such as osteoarthritis, pain or inflammatory enteritis), acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease (such as systemic lupus erythematosus, chronic rheumatism, Sjogren's syndrome, systemic sclerosis, mixed connective tissue disease, Hashimoto's disease, Crohn's disease, ulcerative colitis, idiopathic Addison's disease, male sterility, Goodpasture's syndrome, rapidly progressive glomerulonephritis, myasthenia gravis, polymyositis, multiple sclerosis, autoimmune hemolytic anemia, idiopathic thrombocytopenic purpura, Behcet's disease or CREST syndrome), pancreatitis, cachexia, cancer (such as gastric cancer, lung cancer, breast cancer, colon cancer, prostate cancer, pancreatic cancer or liver cancer), leukemia, sarcoma (such as liposarcoma), osteoporosis, involutional osteoporosis, neurodegenerative disease, Alzheimer's disease, hyperuricemia, dry eyes, or the like.

### Means for Solving the Problems

The present invention relates to:
(1) A compound having general formula (I): 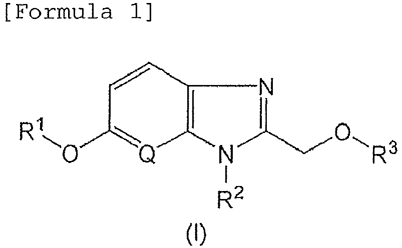 [wherein
   R¹ represents a C₆-C₁₀ aryl group which may be substituted with 1 to 5 group(s) independently selected from Substituent Group a, or a heterocyclic group which may be substituted with 1 to 3 group(s) independently selected from Substituent Group a,
   R² represents a C₁-C₆ alkyl group,
   R³ represents a C₆-C₁₀ aryl group which may be substituted with 1 to 5 group(s) independently selected from Substituent Group a,
   Q represents a group represented by the formula =CH-or a nitrogen atom, and
   Substituent Group a represents a group consisting of a halogen atom, a C₁-C₆ alkyl group, a C₁-C₆ hydroxyalkyl group, a C₁-C₆ halogenated alkyl group, a carboxyl group, a carbamoyl group, a C₂-C₇ alkylcarbonyl group, a C₂-C₇ alkoxycarbonyl group, a hydroxy group, a C₁-C₆ alkoxy group, a C₁-C₆ halogenated alkoxy group, a C₂-C₇ alkylcarbonyloxy group, a C₂-C₇ alkoxycarbonyloxy group, an amino group, a C₂-C₇ alkylcarbonylamino group, a C₂-C₇ alkoxycarbonylamino group, a C₁-C₆ alkylsulfonylamino group, a 4-morpholinyl group and a di-(C₁-C₆ alkyl)amino group]
   or a pharmacologically acceptable salt thereof.
   Preferred embodiments of the present invention include:
(2) The compound or pharmacologically acceptable salt thereof according (1), wherein R¹ is a heterocyclic group and is a pyridyl group, a morpholinyl group, a tetrahydro-2H-pyran group, a tetrahydrofuranyl group, a 2,3-dihydro-1-benzofuran group or a 1,3-benzodioxole group;
(3) The compound or pharmacologically acceptable salt thereof according to (1), wherein R¹ is a phenyl group which may be substituted with 1 to 3 group(s) independently selected from a halogen atom, a C₁-C₆ alkyl group, a C₁-C₆ alkoxy group, a C₁-C₆ halogenated alkoxy group and an amino group, or a 2,3-dihydro-1-benzofuran-6-yl group;
(4) The compound or pharmacologically acceptable salt thereof according to (1), wherein R¹ is a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group, a 3-ethylphenyl group, a 3,4-dimethylphenyl group, a 3-trifluoromethoxyphenyl group, a 3-methoxyphenyl group, a 3-methoxy-4-methylphenyl group, a 4-amino-3,5-dimethylphenyl group or a 2,3-dihydro-1-benzofuran-6-yl group;
(5) The compound or pharmacologically acceptable salt thereof according to (1), wherein R¹ is a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group or a 2,3-dihydro-1-benzofuran-6-yl group;
(6) The compound or pharmacologically acceptable salt thereof according to any one of (1) to (5), wherein R² is a methyl group and Q is a group represented by the formula =CH-;
(7) The compound or pharmacologically acceptable salt thereof according to any one of (1) to (6), wherein R³ is a phenyl group substituted with 1 to 3 fluorine atom(s) and/or carboxyl group(s);
(8) The compound or pharmacologically acceptable salt thereof according to any one of (1) to (6), wherein R³ is a 3-carboxylphenyl group or a 3-carboxyl-5-fluorophenyl group;
(9) The compound according to (1), wherein the compound having the general formula (I) is
   3-{[6-(3-fluorophenoxy)-1-methyl-1H-benzimidazol-2-yl]methoxy}benzoic acid,
   3-[6-(3-chlorophenoxy)-1-methyl-1H-benzimidazol-2-ylmethoxy]benzoic acid,
   3-{[6-(4-chloro-3-fluorophenoxy)-1-methyl-1H-benzimidazol-2-yl]methoxy}benzoic acid,
   3-{[6-(3-chloro-4-fluorophenoxy)-1-methyl-1H-benzimidazol-2-yl]methoxy}benzoic acid,
   3-{[6-(2-fluorophenoxy)-1-methyl-1H-benzimidazol-2-yl]methoxy}benzoic acid,
   3-{[1-methyl-6-(4-methylphenoxy)-1H-benzimidazol-2-yl]methoxy}benzoic acid,
   3-{[6-(2,5-difluorophenoxy)-1-methyl-1H-benzimidazol-2-yl]methoxy}benzoic acid or
   3-{[6-(2,3-dihydro-1-benzofuran-6-yloxy)-1-methyl-1H-benzimidazol-2-yl]methoxy}benzoic acid;
(10) A pharmaceutical composition comprising the compound according to any one of (1) to (9) or pharmacologically acceptable salt thereof as an active ingredient;
(11) The pharmaceutical composition according to (10) for lowering blood glucose;
(12) The pharmaceutical composition according to (10) for the treatment and/or prevention of diabetes;
(13) The pharmaceutical composition according to (10) for the treatment and/or prevention of type II diabetes;
(14) The pharmaceutical composition according to (10) for activating PPARγ;
(15) The pharmaceutical composition according to (10) for improving carbohydrate or lipid metabolism, for improving insulin resistance, for inhibiting inflammation or for inhibiting the growth of cancer cells;
(16) The pharmaceutical composition according to (10) for the treatment and/or prevention of a disease caused by metabolic syndrome;
(17) The pharmaceutical composition according to (10) for the treatment and/or prevention of hyperglycemia, hyperlipidemia, adiposity, impaired glucose tolerance, insulin resistance, impaired fasting glucose, hypertension, fatty liver, nonalcoholic steatohepatitis, diabetic complications, arteriosclerosis, atherosclerosis, gestational diabetes mellitus or polycystic ovary syndrome;
(18) The pharmaceutical composition according to (10) for the treatment and/or prevention of inflammatory disease, cancer, osteoporosis, involutional osteoporosis, neurodegenerative disease, Alzheimer's disease or hyperuricemia;
(19) The pharmaceutical composition according to (10) for the treatment and/or prevention of acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease, pancreatitis, cachexia, leukemia, sarcoma or dry eyes;
(20) A PPARγ activator/modulator comprising the compound according to any one of (1) to (9) or pharmacologically acceptable salt thereof as an active ingredient;
(21) Use of the compound according to any one of (1) to (9) or pharmacologically acceptable salt thereof for producing a pharmaceutical composition;
(22) The use according to (21), wherein the pharmaceutical composition is a composition for lowering blood glucose;
(23) The use according to (21), wherein the pharmaceutical composition is a composition for treatment and/or prevention of diabetes;
(24) The use according to (21), wherein the pharmaceutical composition is a composition for treatment and/or prevention of type II diabetes;
(25) The use according to (21), wherein the pharmaceutical composition is a composition for activating PPARγ;
(26) The use according to (21), wherein the pharmaceutical composition is a composition for improving carbohydrate or lipid metabolism, for improving insulin resistance, for inhibiting inflammation or for inhibiting the growth of cancer cells;
(27) The use according to (21), wherein the pharmaceutical composition is a composition for the treatment and/or prevention of a disease caused by metabolic syndrome;
(28) The use according to (21), wherein the pharmaceutical composition is a composition for the treatment and/or prevention of hyperglycemia, hyperlipidemia, adiposity, impaired glucose tolerance, insulin resistance, impaired fasting glucose, hypertension, fatty liver, nonalcoholic steatohepatitis, diabetic complications, arteriosclerosis, atherosclerosis, gestational diabetes mellitus or polycystic ovary syndrome;
(29) The use according to (21), wherein the pharmaceutical composition is a composition for the treatment and/or prevention of inflammatory disease, cancer, osteoporosis, involutional osteoporosis, neurodegenerative disease, Alzheimer's disease or hyperuricemia;
(30) The use according to (21), wherein the pharmaceutical composition is a composition for the treatment and/or prevention of acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease, pancreatitis, cachexia, leukemia, sarcoma or dry eyes; and
(31) The use according to (21), wherein the pharmaceutical composition is a PPARγ activator/modulator.

The "C₁-C₆ alkyl group" in the present invention is a linear or branched alkyl group having 1 to 6 carbon atom(s). Examples of such a group include a methyl group, an ethyl group, a propyl group, an isopropyl group, a butyl group, an isobutyl group, an s-butyl group, a t-butyl group, a pentyl group, an isopentyl group, a 2-methylbutyl group, a neopentyl group, a 1-ethylpropyl group, a hexyl group, an isohexyl group, a 4-methylpentyl group, a 3-methylpentyl group, a 2-methylpentyl group, a 1-methylpentyl group and a 3,3-dimethylbutyl group. The group is preferably a 1-ethylpropyl group for R¹ and is preferably a linear or branched alkyl group having 1 to 4 carbon atom(s) (C₁-C₄ alkyl group), more preferably a methyl group or an ethyl group (C₁-C₂ alkyl group), and more preferably a methyl group for other substituents.

The "halogen atom" in the present invention is a fluorine atom, a chlorine atom, a bromine atom or an iodine atom. The halogen atom is preferably a fluorine atom or a chlorine atom.

The "C₁-C₆ hydroxyalkyl group" in the present invention is a group in which a hydroxy group is bonded to the above-mentioned "C₁-C₆ alkyl group". The group is, for example, a hydroxymethyl group, a hydroxyethyl group or a hydroxypropyl group, and is preferably a group in which a hydroxy group is bonded to a linear or branched alkyl group having 1 to 4 carbon atom(s) (C₁-C₄ alkyl group substituted with a hydroxy group), more preferably a hydroxymethyl group or a 2-hydroxyethyl group, and still more preferably a hydroxymethyl group.

The "C₁-C₆ halogenated alkyl group" in the present invention is a group in which the same or different 1 to 5 above-mentioned "halogen atom" are bonded to the above-mentioned "C₁-C₆ alkyl group". Examples of such a group include a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group and a 2-fluoroethyl group. The group is preferably a group in which the same or different 1 to 5 above-mentioned "halogen atom" are bonded to the above-mentioned "C₁-C₄ alkyl group" (C₁-C₄ halogenated alkyl group), more preferably a group in which the same or different 1 to 5 above-mentioned "halogen atom" are bonded to the above-mentioned "C₁-C₂ alkyl group" (C₁-C₂ halogenated alkyl group), and still more preferably a trifluoromethyl group.

The "C₂-C₇ alkylcarbonyl group" in the present invention is a group in which the above-mentioned "C₁-C₆ alkyl group" is bonded to a carbonyl group. Examples of such a group include an acetyl group, a propionyl group, a butyryl group, an isobutyryl group, a pentanoyl group, a pivaloyl group, a valeryl group and an isovaleryl group. The group is preferably a group in which the above-mentioned "C₁-C₄ alkyl group" is bonded to a carbonyl group (C₂-C₅ alkylcarbonyl group), more preferably an acetyl group or a propionyl group (C₂-C₃ alkylcarbonyl group), and still more preferably an acetyl group.

The "C₁-C₆ alkoxy group" in the present invention is a group in which the above-mentioned "C₁-C₆ alkyl group" is bonded to an oxygen atom, and is a linear or branched alkoxy group having 1 to 6 carbon atom(s). Examples of such a group include a methoxy group, an ethoxy group, a propoxy group, an isopropoxy group, a butoxy group, an isobutoxy group, an s-butoxy group, a t-butoxy group, a pentoxy group and a 2-methylbutoxy group. The group is preferably a linear or branched alkoxy group having 1 to 4 carbon atom(s) (C₁-C₄ alkoxy group), and more preferably a methoxy group or an isopropoxy group.

The "C₂-C₇ alkoxycarbonyl group" in the present invention is a group in which the above-mentioned "C₁-C₆ alkoxy group" is bonded to a carbonyl group. Examples of such a group include a methoxycarbonyl group, an ethoxycarbonyl group, a propoxycarbonyl group, an isopropoxycarbonyl group, a butoxycarbonyl group, an isobutoxycarbonyl group, an s-butoxycarbonyl group, a t-butoxycarbonyl group and a pentoxycarbonyl group. The group is preferably a group in which the above-mentioned "C₁-C₄ alkoxy group" is bonded to a carbonyl group (C₂-C₅ alkoxycarbonyl group), more preferably a methoxycarbonyl group or an ethoxycarbonyl group (C₂-C₃ alkoxycarbonyl group), and still more preferably a methoxycarbonyl group.

The "C₁-C₆ halogenated alkoxy group" in the present invention is a group in which the same or different 1 to 5 above-mentioned "halogen atom" are bonded to the above-mentioned "C₁-C₆ alkoxy group". Examples of such a group include a trifluoromethoxy group, a trichloromethoxy group, a difluoromethoxy group, a fluoromethoxy group, a 2,2,2-trifluoroethoxy group, a 2,2,2-trichloroethoxy group, a 2-bromoethoxy group, a 2-chloroethoxy group and a 2-fluoroethoxy group. The group is preferably a group in which the same or different 1 to 5 above-mentioned "halogen atom" are bonded to the above-mentioned "C₁-C₄ alkoxy group" (C₁-C₄ halogenated alkoxy group), more preferably a group in which the same or different 1 to 5 above-mentioned "halogen atom" are bonded to the above-mentioned "C₁-C₂ alkoxy group" (C₁-C₂ halogenated alkoxy group), and still more preferably a trifluoromethoxy group.

The "C₂-C₇ alkylcarbonyloxy group" in the present invention is a group in which the above-mentioned "C₂-C₇ alkylcarbonyl group" is bonded to an oxygen atom. Examples of such a group include an acetoxy group, a propionyloxy group, a butyryloxy group and an isobutyryloxy group. The group is preferably a group in which the above-mentioned "C₂-C₅ alkylcarbonyl group" is bonded to an oxygen atom (C₂-C₅ alkylcarbonyloxy group), more preferably an acetoxy group or a propionyloxy group (C₂-C₃ alkylcarbonyloxy group), and still more preferably an acetoxy group.

The "C₂-C₇ alkoxycarbonyloxy group" in the present invention is a group in which the above-mentioned "C₂-C₇ alkoxycarbonyl group" is bonded to an oxygen atom. Examples of such a group include a methoxycarbonyloxy group, an ethoxycarbonyloxy group, a propoxycarbonyloxy group, an isopropoxycarbonyloxy group, a butoxycarbonyloxy group and an isobutoxycarbonyloxy group. The group is preferably a group in which the above-mentioned "C₂-C₅ alkoxycarbonyl group" is bonded to an oxygen atom (C₂-C₅ alkoxycarbonyloxy group), more preferably a methoxycarbonyloxy group or an ethoxycarbonyloxy group (C₂-C₃ alkoxycarbonyloxy group), and still more preferably a methoxycarbonyloxy group.

The "C₂-C₇ alkylcarbonylamino group" in the present invention is a group in which one carbonyl group with the above-mentioned "C₁-C₆ alkyl group" bonded thereto is bonded to an amino group. Examples of such a group include an acetamido group, an ethylcarbonylamino group, a propylcarbonylamino group, an isopropylcarbonylamino group and a butylcarbonylamino group. The group is preferably a group in which one carbonyl group with the above-mentioned "C₁-C₄ alkyl group" bonded thereto is bonded to an amino group (C₂-C₅ alkylcarbonylamino group), and more preferably an acetamido group or an ethylcarbonylamino group (C₂-C₃ alkylcarbonylamino group).

The "C₂-C₇ alkoxycarbonylamino group" in the present invention is a group in which one carbonyl group with the above-mentioned "C₁-C₆ alkoxy group" bonded thereto is bonded to an amino group. Examples of such a group include a methoxycarbonylamino group, an ethoxycarbonylamino group, a propoxycarbonylamino group, an isopropoxycarbonylamino group, a butoxycarbonylamino group, an isobutoxycarbonylamino group and an s-butoxycarbonylamino group. The group is preferably a group in which a carbonyl group with the above-mentioned "C₁-C₄ alkoxy group" bonded thereto is bonded to an amino group (C₂-C₅ alkoxycarbonylamino group), more preferably a methoxycarbonylamino group or an ethoxycarbonylamino group (C₂-C₃ alkoxycarbonylamino group), and still more preferably a methoxycarbonylamino group.

The "C₁-C₆ alkylsulfonylamino group" in the present invention is a group in which one sulfonyl group with the above-mentioned "C₁-C₆ alkyl group" bonded thereto is bonded to an amino group. Examples of such a group include a methylsulfonylamino group, an ethylsulfonylamino group, a propylsulfonylamino group, an isopropylsulfonylamino group and a butylsulfonylamino group. The group is preferably a group in which one sulfonyl group with the above-mentioned "C₁-C₄ alkyl group" bonded thereto is bonded to an amino group (mono-C₁-C₄ alkylsulfonylamino group), more preferably a methylsulfonylamino group or an ethylsulfonylamino group (mono-C₁-C₂ alkylsulfonylamino group), and still more preferably a methylsulfonylamino group.

The "di-(C₁-C₆ alkyl)amino group" in the present invention is a group in which the same or different two above-mentioned "C₁-C₆ alkyl group" are bonded to an amino group. Examples of such a group include a dimethylamino group, a diethylamino group, a dipropylamino group, a diisopropylamino group, a dibutylamino group, a diisobutylamino group, a dipentylamino group, a diisopentylamino group, a dineopentylamino group, a dihexylamino group, an N-ethyl-N-methylamino group, an N-methyl-N-propylamino group, an N-isopropyl-N-methylamino group, an N-butyl-N-methylamino group, an N-isobutyl-N-methylamino group, an N-methyl-N-pentylamino group, an N-isopentyl-N-methylamino group, an N-ethyl-N-propylamino group, an N-ethyl-N-isopropylamino group, an N-butyl-N-ethylamino group and an N-ethyl-N-isopentylamino group. The group is preferably a group in which the same or different two above-mentioned "C₁-C₄ alkyl group" are bonded to an amino group (di-(C₁-C₄ alkyl)amino group), more preferably a dimethylamino group, a diethylamino group or an N-ethyl-N-methylamino group (di-(C₁-C₂ alkyl)amino group), and still more preferably a dimethylamino group.

The "C₆-C₁₀ aryl group" in the present invention is an aromatic hydrocarbon group having 6 to 10 carbon atoms. The group is preferably a phenyl group or a naphthyl group, and more preferably a phenyl group.

The "heterocyclic group" in the present invention is a four- to seven-membered heterocyclic group which contains 1 to 3 sulfur atom(s), oxygen atom(s) or/and nitrogen atom(s) and may further contain 1 or 2 nitrogen atom(s) and in which two oxygen atoms may be bonded to the sulfur atom. Examples of such a group include "aromatic heterocyclic group" such as a furyl group, a thienyl group, a pyrrolyl group, an azepinyl group, a pyrazolyl group, an imidazolyl group, an oxazolyl group, an isoxazolyl group, a thiazolyl group, an isothiazolyl group, a 1,2,3-oxadiazolyl group, a triazolyl group, a tetrazolyl group, a thiadiazolyl group, a pyranyl group, a pyridyl group, a pyridazinyl group, a pyrimidinyl group and a pyrazinyl group; and "partially or completely reduced saturated heterocyclic group" such as a tetrahydropyranyl group, a tetrahydrothienyl group, a morpholinyl group, a thiomorpholinyl group, a pyrrolidinyl group, a pyrrolinyl group, an imidazolidinyl group, a pyrazolidinyl group, a piperidinyl group, a piperazinyl group, an oxazolidinyl group, an isoxazolidinyl group, a thiazolidinyl group, a pyrazolidinyl group, a dioxolanyl group and a dioxanyl group. The above heterocyclic group may be fused with another cyclic group such as a benzene ring ("fused bicyclic heteroaryl group"). Examples of such a group include a benzothienyl group, a benzothiazolyl group, a benzoxazolyl group, an isobenzofuranyl group, a 1,3-dihydroisobenzofuranyl group, a quinolyl group, a 1,3-benzodioxolanyl group, a 1,4-benzodioxanyl group, an indolyl group, an isoindolyl group and an indolinyl group. The group is preferably a six-membered heterocyclic group or a fused bicyclic heteroaryl group containing 1 to 3 sulfur atom(s), oxygen atom(s) or/and nitrogen atom(s), more preferably a pyridyl group, a morpholinyl group, a tetrahydro-2H-pyran group, a 2,3-dihydro-1-benzofuran group or a 1,3-benzodioxole group, still more preferably a 3-pyridyl group, a 4-morpholinyl group, a tetrahydro-2H-pyran-4-yl group, a 2,3-dihydro-1-benzofuran-6-yl group or a 1,3-benzodioxol-5-yl group, and particularly preferably a 2,3-dihydro-1-benzofuran-6-yl group.

The "C₆-C₁₀ aryl group which may be substituted with 1 to 5 group(s) independently selected from Substituent Group a" in the present invention is the aforementioned "C₆-C₁₀ aryl group" which may be substituted with 1 to 5 group(s) independently selected from Substituent Group a. Such a group for R¹ is preferably a phenyl group which may be substituted with 1 to 3 group(s) independently selected from a halogen atom, a C₁-C₆ alkyl group, a C₁-C₆ alkoxy group, a C₁-C₆ halogenated alkoxy group and an amino group, more preferably a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group, a 3-ethylphenyl group, a 3,4-dimethylphenyl group, a 3-trifluoromethoxyphenyl group, a 3-methoxyphenyl group, a 3-methoxy-4-methylphenyl group or a 4-amino-3,5-dimethylphenyl group, and still more preferably a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group or a 4-methylphenyl group. Such a group for R³ is preferably a phenyl group substituted with 1 to 3 fluorine atom(s) and/or carboxyl group(s), and more preferably a 3-carboxylphenyl group or a 3-carboxyl-5-fluorophenyl group.

The "heterocyclic group which may be substituted with 1 to 3 group(s) independently selected from Substituent Group a" in the present invention is the aforementioned "heterocyclic group" which may be substituted with 1 to 3 group(s) independently selected from Substituent Group a. Such a group is preferably a pyridyl group substituted with 1 to 3 group(s) independently selected from a halogen atom and a C₁-C₆ alkoxy group, a pyridyl group, a tetrahydro-2H-pyran-4-yl group, a tetrahydrofuran-3-yl group, a 2,3-dihydro-1-benzofuran-6-yl group or a 1,3-benzodioxol-5-yl group, and more preferably a 2,3-dihydro-1-benzofuran-6-yl group.

In the present invention, R¹ is preferably a 1-ethylpropyl group, a phenyl group which may be substituted with 1 to 3 group(s) independently selected from a halogen atom, a C₁-C₆ alkyl group, a C₁-C₆ alkoxy group, a C₁-C₆ halogenated alkoxy group and an amino group, or a 2,3-dihydro-1-benzofuran-6-yl group. R¹ is more preferably a 1-ethylpropyl group, a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group, a 3-ethylphenyl group, a 3,4-dimethylphenyl group, a 3-trifluoromethoxyphenyl group, a 3-methoxyphenyl group, a 3-methoxy-4-methylphenyl group, a 4-amino-3,5-dimethylphenyl group or a 2,3-dihydro-1-benzofuran-6-yl group. R¹ is still more preferably a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group or a 2,3-dihydro-1-benzofuran-6-yl group.

In the present invention, R² is preferably a methyl group.

In the present invention, R³ is preferably a phenyl group substituted with 1 to 3 fluorine atom(s) and/or carboxyl group(s). R³ is more preferably a 3-carboxylphenyl group or a 3-carboxyl-5-fluorophenyl group.

In the present invention, Q is preferably a group represented by the formula =CH-.

The fused bicyclic heteroaryl derivative or pharmacologically acceptable salt thereof having the general formula (I) according to the present invention includes all isomers (such as a keto-enol isomer, a diastereomer, an optical isomer, a rotamer, etc.). The fused bicyclic heteroaryl derivative or pharmacologically acceptable salt thereof having the general formula (I) according to the present invention has various isomers because asymmetric carbon atom(s) exist in the molecule. These isomers and mixtures of these isomers of the present invention are all represented by a single formula, specifically, the general formula (I). Accordingly, the present invention includes all of these isomers and mixtures of these isomers in arbitrary ratios.

The aforementioned stereoisomers can be obtained by synthesizing the compound of the present invention using an optically active raw material compound or using an asymmetric synthesis or asymmetric induction technique or by isolating the synthesized compound of the present invention by a common optical resolution or separation method if desired.

The "pharmacologically acceptable salt thereof" represents a salt that can be obtained by reacting the fused bicyclic heteroaryl derivative having the general formula (I) according to the present invention having a basic group such as an amino group with an acid or reacting the derivative having an acidic group such as a carboxyl group with a base.

Preferable examples of the salt based on a basic group include hydrohalides such as hydrofluorides, hydrochlorides, hydrobromides and hydroiodides; inorganic acid salts such as nitrates, perchlorates, sulfates and phosphates; alkyl sulfonates such as methanesulfonates and ethanesulfonates; haloalkyl sulfonates such as trifluoromethanesulfonates; aryl sulfonates such as benzenesulfonates and p-toluenesulfonates; and organic acid salts such as acetates, malates, fumarates, succinates, citrates, ascorbates, tartrates, oxalates and maleates.

On the other hand, preferable examples of the salt based on an acidic group include alkali metal salts such as sodium salts, potassium salts and lithium salts; alkali earth metal salts such as calcium salts and magnesium salts; and metal salts such as aluminum salts and iron salts.

The fused bicyclic heteroaryl derivative or pharmacologically acceptable salt thereof having the general formula (I) according to the present invention may absorb moisture or adsorb water to form a hydrate when left to stand in the air or in a purification or preparation step, and such a hydrate is also included in the salt of the present invention.

The fused bicyclic heteroaryl derivative or pharmacologically acceptable salt thereof having the general formula (I) according to the present invention may absorb some other specific solvent(s) to form a solvate, and such a solvate is also included in the salt of the present invention.

Specific examples of the compound having the general formula (I) according to the present invention include compounds shown in the following Table 1; however, the present invention is not limited to these groups.

The abbreviations in the following Table 1 are as follows. Specifically,
Me represents a methyl group,
Et represents an ethyl group,
Ph represents a phenyl group,
3-CO₂H-Ph represents a 3-carboxyphenyl group,
4-Mor represents a 4-morpholinyl group,
5-CO₂H-3-Py represents a 5-carboxy-3-pyridyl group,
Het (A) represents a tetrahydro-2H-pyran-4-yl group,
Het (B) represents a tetrahydrofuran-3-yl group,
Het (C) represents a 2,3-dihydro-1-benzofuran-6-yl group, and
Het (D) represents a 1,3-benzodioxol-5-yl group.

**(Table 1)**

| | | | |
|---|---|---|---|
| 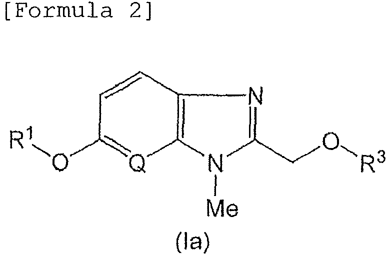 | | | |

| Compound No. | R¹ | Q | R³ |
|---|---|---|---|
| 1-17 | Ph | =CH- | 2-CO₂H-Ph |
| 1-18 | Ph | =CH- | 2-CO₂Et-Ph |
| 1-19 | Ph | =CH- | 3-CO₂H-Ph |
| 1-20 | Ph | =CH- | 3-CO₂Me-Ph |
| 1-21 | Ph | =CH- | 4-CO₂H-Ph |
| 1-22 | Ph | =CH- | 4-CO₂Et-Ph |
| | | | |
| 1-25 | Ph | N | 2-CO₂H-Ph |
| 1-26 | Ph | N | 2-CO₂Et-Ph |
| 1-27 | Ph | N | 3-CO₂H-Ph |
| 1-28 | Ph | N | 3-CO₂Me-Ph |
| 1-29 | Ph | N | 4-CO₂H-Ph |
| 1-30 | Ph | N | 4-CO₂Et-Ph |
| | | | |
| 1-33 | 3-F-Ph | =CH- | 2-CO₂H-Ph |
| 1-34 | 3-F-Ph | =CH- | 2-CO₂Et-Ph |
| 1-35 | 3-F-Ph | =CH- | 3-CO₂H-Ph |
| 1-36 | 3-F-Ph | =CH- | 3-CO₂Me-Ph |
| 1-37 | 3-F-Ph | =CH- | 4-CO₂H-Ph |
| 1-38 | 3-F-Ph | =CH- | 4-CO₂Et-Ph |
| | | | |
| 1-41 | 3-F-Ph | N | 2-CO₂H-Ph |
| 1-42 | 3-F-Ph | N | 2-CO₂Et-Ph |
| 1-43 | 3-F-Ph | N | 3-CO₂H-Ph |
| 1-44 | 3-F-Ph | N | 3-CO₂Me-Ph |
| 1-45 | 3-F-Ph | N | 4-CO₂H-Ph |
| 1-46 | 3-F-Ph | N | 4-CO₂Et-Ph |
| | | | |
| 1-49 | 3-Cl-Ph | =CH- | 2-CO₂H-Ph |
| 1-50 | 3-Cl-Ph | =CH- | 2-CO₂Et-Ph |
| 1-51 | 3-Cl-Ph | =CH- | 3-CO₂H-Ph |
| 1-52 | 3-Cl-Ph | =CH- | 3-CO₂Me-Ph |
| 1-53 | 3-Cl-Ph | =CH- | 4-CO₂H-Ph |
| 1-54 | 3-Cl-Ph | =CH- | 4-CO₂Et-Ph |
| | | | |
| 1-57 | 3-Cl-Ph | N | 2-CO₂H-Ph |
| 1-58 | 3-Cl-Ph | N | 2-CO₂Et-Ph |
| 1-59 | 3-Cl-Ph | N | 3-CO₂H-Ph |
| 1-60 | 3-Cl-Ph | N | 3-CO₂Me-Ph |
| 1-61 | 3-Cl-Ph | N | 4-CO₂H-Ph |
| 1-62 | 3-Cl-Ph | N | 4-CO₂Et-Ph |
| | | | |
| 1-65 | 3-(4-Mor)-Ph | =CH- | 2-CO₂H-Ph |
| 1-66 | 3-(4-Mor)-Ph | =CH- | 2-CO₂Et-Ph |
| 1-67 | 3-(4-Mor)-Ph | =CH- | 3-CO₂H-Ph |
| 1-68 | 3-(4-Mor)-Ph | =CH- | 3-CO₂Me-Ph |
| 1-69 | 3-(4-Mor)-Ph | =CH- | 4-CO₂H-Ph |
| 1-70 | 3-(4-Mor)-Ph | =CH- | 4-CO₂Et-Ph |
| | | | |
| 1-73 | 3-(4-Mor)-Ph | N | 2-CO₂H-Ph |
| 1-74 | 3-(4-Mor)-Ph | N | 2-CO₂Et-Ph |
| 1-75 | 3-(4-Mor)-Ph | N | 3-CO₂H-Ph |
| 1-76 | 3-(4-Mor)-Ph | N | 3-CO₂Me-Ph |
| 1-77 | 3-(4-Mor)-Ph | N | 4-CO₂H-Ph |
| 1-78 | 3-(4-Mor)-Ph | N | 4-CO₂Et-Ph |
| | | | |
| 1-81 | 2,4-Cl₂-Ph | =CH- | 2-CO₂H-Ph |
| 1-82 | 2,4-Cl₂-Ph | =CH- | 2-CO₂Et-Ph |
| 1-83 | 2,4-Cl₂-Ph | =CH- | 3-CO₂H-Ph |
| 1-84 | 2,4-Cl₂-Ph | =CH- | 3-CO₂Me-Ph |
| 1-85 | 2,4-Cl₂-Ph | =CH- | 4 -CO₂H-Ph |
| 1-86 | 2,4-Cl₂-Ph | =CH- | 4-CO₂Et-Ph |
| | | | |
| 1-89 | 2,4-Cl₂-Ph | N | 2-CO₂H-Ph |
| 1-90 | 2,4-Cl₂-Ph | N | 2-CO₂Et-Ph |
| 1-91 | 2,4-Cl₂-Ph | N | 3-CO₂H-Ph |
| 1-92 | 2,4-Cl₂-Ph | N | 3-CO₂Me-Ph |
| 1-93 | 2,4-Cl₂-Ph | N | 4-CO₂H-Ph |
| 1-94 | 2,4-Cl₂-Ph | N | 4-CO₂Et-Ph |
| | | | |
| 1-97 | 4-Cl-3-F-Ph | =CH- | 2-CO₂H-Ph |
| 1-98 | 4-Cl-3-F-Ph | =CH- | 2-CO₂Et-Ph |
| 1-99 | 4-Cl-3-F-Ph | =CH- | 3-CO₂H-Ph |
| 1-100 | 4-Cl-3-F-Ph | =CH- | 3-CO₂Me-Ph |
| 1-101 | 4-Cl-3-F-Ph | =CH- | 4-CO₂H-Ph |
| 1-102 | 4-Cl-3-F-Ph | =CH- | 4-CO₂Et-Ph |
| 1-105 | 4-Cl-3-F-Ph | N | 2-CO₂H-Ph |
| 1-106 | 4-Cl-3-F-Ph | N | 2-CO₂Et-Ph |
| 1-107 | 4-Cl-3-F-Ph | N | 3-CO₂H-Ph |
| 1-108 | 4-Cl-3-F-Ph | N | 3-CO₂Me-Ph |
| 1-109 | 4-Cl-3-F-Ph | N | 4-CO₂H-Ph |
| 1-110 | 4-Cl-3-F-Ph | N | 4-CO₂Et-Ph |
| | | | |
| 1-113 | 3-Cl-4-F-Ph | =CH- | 2-CO₂H-Ph |
| 1-114 | 3-Cl-4-F-Ph | =CH- | 2-CO₂Et-Ph |
| 1-115 | 3-Cl-4-F-Ph | =CH- | 3-CO₂H-Ph |
| 1-116 | 3-Cl-4-F-Ph | =CH- | 3-CO₂Me-Ph |
| 1-117 | 3-Cl-4-F-Ph | =CH- | 4-CO₂H-Ph |
| 1-118 | 3-Cl-4-F-Ph | =CH- | 4-CO₂Et-Ph |
| | | | |
| 1-121 | 3-Cl-4-F-Ph | N | 2-CO₂H-Ph |
| 1-122 | 3-Cl-4-F-Ph | N | 2-CO₂Et-Ph |
| 1-123 | 3-Cl-4-F-Ph | N | 3-CO₂H-Ph |
| 1-124 | 3-Cl-4-F-Ph | N | 3-CO₂Me-Ph |
| 1-125 | 3-Cl-4-F-Ph | N | 4-CO₂H-Ph |
| 1-126 | 3-Cl-4-F-Ph | N | 4-CO₂Et-Ph |
| | | | |
| 1-129 | 4-NH₂-3,5-Me₂-Ph | =CH- | 2-CO₂H-Ph |
| 1-130 | 4-NH₂-3,5-Me₂-Ph | =CH- | 2-CO₂Et-Ph |
| 1-131 | 4-NH₂-3,5-Me₂-Ph | =CH- | 3-CO₂H-Ph |
| 1-132 | 4-NH₂-3,5-Me₂-Ph | =CH- | 3-CO₂Me-Ph |
| 1-133 | 4-NH₂-3,5-Me₂-Ph | =CH- | 4-CO₂H-Ph |
| 1-134 | 4-NH₂-3,5-Me₂-Ph | =CH- | 4-CO₂Et-Ph |
| | | | |
| 1-137 | 4-NH₂-3,5-Me₂-Ph | N | 2-CO₂H-Ph |
| 1-138 | 4-NH₂-3,5-Me₂-Ph | N | 2-CO₂Et-Ph |
| 1-139 | 4-NH₂-3,5-Me₂-Ph | N | 3-CO₂H-Ph |
| 1-140 | 4-NH₂-3,5-Me₂-Ph | N | 3-CO₂Me-Ph |
| 1-141 | 4-NH₂-3,5-Me₂-Ph | N | 4-CO₂H-Ph |
| 1-142 | 4-NH₂-3,5-Me₂-Ph | N | 4-CO₂Et-Ph |
| 1-145 | 3-Py | =CH- | 2-CO₂H-Ph |
| 1-146 | 3-Py | =CH- | 2-CO₂Et-Ph |
| 1-147 | 3-Py | =CH- | 3-CO₂H-Ph |
| 1-148 | 3-Py | =CH- | 3-CO₂Me-Ph |
| 1-149 | 3-Py | =CH- | 4-CO₂H-Ph |
| 1-150 | 3-Py | =CH- | 4-CO₂Et-Ph |
| | | | |
| 1-153 | 3-Py | N | 2-CO₂H-Ph |
| 1-154 | 3-Py | N | 2-CO₂Et-Ph |
| 1-155 | 3-Py | N | 3-CO₂H-Ph |
| 1-156 | 3-Py | N | 3-CO₂Me-Ph |
| 1-157 | 3-Py | N | 4-CO₂H-Ph |
| 1-158 | 3-Py | N | 4 -CO₂Et-Ph |
| | | | |
| 1-164 | 2-F-Ph | =CH- | 3-CO₂H-Ph |
| 1-165 | 4-F-Ph | =CH- | 3-CO₂H-Ph |
| 1-166 | 2-Me-Ph | =CH- | 3-CO₂H-Ph |
| 1-167 | 3-Me-Ph | =CH- | 3-CO₂H-Ph |
| 1-168 | 4-Me-Ph | =CH- | 3-CO₂H-Ph |
| 1-169 | 2-Et-Ph | =CH- | 3-CO₂H-Ph |
| 1-170 | 3-Et-Ph | =CH- | 3-CO₂H-Ph |
| 1-171 | 4-Et-Ph | =CH- | 3-CO₂H-Ph |
| 1-172 | 2-OMe-Ph | =CH- | 3-CO₂H-Ph |
| 1-173 | 3-OMe-Ph | =CH- | 3-CO₂H-Ph |
| 1-174 | 4-OMe-Ph | =CH- | 3-CO₂H-Ph |
| 1-175 | 3-CF₃-Ph | =CH- | 3-CO₂H-Ph |
| 1-176 | 3-OCF₃-Ph | =CH- | 3-CO₂H-Ph |
| 1-177 | 3-NMe₂-Ph | =CH- | 3-CO₂H-Ph |
| 1-178 | 2,4-F₂-Ph | =CH- | 3-CO₂H-Ph |
| 1-179 | 2,5-F₂-Ph | =CH- | 3-CO₂H-Ph |
| 1-180 | 3,4-F₂-Ph | =CH- | 3-CO₂H-Ph |
| 1-181 | 3,5-F₂-Ph | =CH- | 3-CO₂H-Ph |
| 1-182 | 2-F-4-Me-Ph | =CH- | 3-CO₂H-Ph |
| 1-183 | 4-F-2-Me-Ph | =CH- | 3-CO₂H-Ph |
| 1-184 | 2-F-5-Me-Ph | =CH- | 3-CO₂H-Ph |
| 1-185 | 5-F-2-Me-Ph | =CH- | 3-CO₂H-Ph |
| 1-186 | 3-F-4-Me-Ph | =CH- | 3-CO₂H-Ph |
| 1-187 | 4-F-3-Me-Ph | =CH- | 3-CO₂H-Ph |
| 1-188 | 3-F-5-Me-Ph | =CH- | 3-CO₂H-Ph |
| 1-189 | 2-F-4-OMe-Ph | =CH- | 3-CO₂H-Ph |
| 1-190 | 4-F-2-OMe-Ph | =CH- | 3-CO₂H-Ph |
| 1-191 | 2-F-5-OMe-Ph | =CH- | 3-CO₂H-Ph |
| 1-192 | 5-F-2-OMe-Ph | =CH- | 3-CO₂H-Ph |
| 1-193 | 3-F-4-OMe-Ph | =CH- | 3-CO₂H-Ph |
| 1-194 | 4-F-3-OMe-Ph | =CH- | 3-CO₂H-Ph |
| 1-195 | 3-F-5-OMe-Ph | =CH- | 3-CO₂H-Ph |
| 1-196 | 2,4-Me₂-Ph | =CH- | 3-CO₂H-Ph |
| 1-197 | 2,5-Me₂-Ph | =CH- | 3-CO₂H-Ph |
| 1-198 | 3,4-Me₂-Ph | =CH- | 3-CO₂H-Ph |
| 1-199 | 3,5-Me₂-Ph | =CH- | 3-CO₂H-Ph |
| 1-200 | 2-OMe-4-Me-Ph | =CH- | 3-CO₂H-Ph |
| 1-201 | 4-OMe-2-Me-Ph | =CH- | 3-CO₂H-Ph |
| 1-202 | 2-OMe-5-Me-Ph | =CH- | 3-CO₂H-Ph |
| 1-203 | 5-OMe-2-Me-Ph | =CH- | 3-CO₂H-Ph |
| 1-204 | 3-OMe-4-Me-Ph | =CH- | 3-CO₂H-Ph |
| 1-205 | 4-OMe-3-Me-Ph | =CH- | 3-CO₂H-Ph |
| 1-206 | 3-OMe-5-Me-Ph | =CH- | 3-CO₂H-Ph |
| 1-207 | 3-Cl-5-F-Ph | =CH- | 3-CO₂H-Ph |
| 1-208 | Het(A) | =CH- | 3-CO₂H-Ph |
| 1-209 | Het(B) | =CH- | 3-CO₂H-Ph |
| 1-210 | Het(C) | =CH- | 3-CO₂H-Ph |
| 1-211 | Het(D) | =CH- | 3-CO₂H-Ph |
| 1-212 | 3-F-Ph | =CH- | 3-CO₂H-5-F-Ph |
| 1-213 | 4-F-Ph | =CH- | 3-CO₂H-5-F-Ph |
| 1-214 | 3-Cl-Ph | =CH- | 3-CO₂H-5-F-Ph |
| 1-215 | 4-Cl-Ph | =CH- | 3-CO₂H-5-F-Ph |
| 1-216 | 3-Me-Ph | =CH- | 3-CO₂H-5-F-Ph |
| 1-217 | 4-Me-Ph | =CH- | 3-CO₂H-5-F-Ph |
| 1-218 | 3-OMe-Ph | =CH- | 3-CO₂H-5-F-Ph |
| 1-219 | 4-OMe-Ph | =CH- | 3-CO₂H-5-F-Ph |
| 1-220 | 4-Cl-3-F-Ph | =CH- | 3-CO₂H-5-F-Ph |
| 1-221 | 4-Me-Ph | N | 3-CO₂H-Ph |
| 1-222 | 4-Me-Ph | N | 3-CO₂H-5-F-Ph |
| 1-223 | 3,4-Me₂-Ph | N | 3-CO₂H-Ph |
| 1-224 | 3,4-Me₂-Ph | N | 3-CO₂H-5-F-Ph |
| 1-225 | 3,5-Me₂-Ph | N | 3-CO₂H-Ph |
| 1-226 | 3,5-Me₂-Ph | N | 3-CO₂H-5-F-Ph |
| 1-227 | 3-F-4-Me-Ph | N | 3-CO₂H-Ph |
| 1-228 | 3-F-4-Me-Ph | N | 3-CO₂H-5-F-Ph |

In Table 1, preferred compounds are compound Nos. 1-19, 1-27, 1-35, 1-43, 1-50, 1-59, 1-99, 1-107, 1-115, 1-123, 1-131, 1-139, 1-164, 1-168, 1-170, 1-173, 1-175, 1-176, 1-179, 1-188, 1-198, 1-204, 1-210, 1-217, 1-220, 1-221, 1-222, 1-223, 1-224 and 1-227.

More preferred compounds are
3-{[6-(3-fluorophenoxy)-1-methyl-1H-benzimidazol-2-yl]methoxy}benzoic acid (Compound No. 1-35),
3-[6-(3-chlorophenoxy)-1-methyl-1H-benzimidazol-2-ylmethoxy]benzoic acid (Compound No. 1-51),
3-{[6-(4-chloro-3-fluorophenoxy)-1-methyl-1H-benzimidazol-2-yl]methoxy}benzoic acid (Compound No. 1-99),
3-{[6-(3-chloro-4-fluorophenoxy)-1-methyl-1H-benzimidazol-2-yl]methoxy}benzoic acid (Compound No. 1-115),
3-{[6-(4-amino-3,5-dimethylphenoxy)-1-methyl-1H-benzimidazol-2-yl]methoxy}benzoic acid (Compound No. 1-131),
3-{[6-(2-fluorophenoxy)-1-methyl-1H-benzimidazol-2-yl]methoxy}benzoic acid (Compound No. 1-164),
3-{[1-methyl-6-(4-methylphenoxy)-1H-benzimidazol-2-yl]methoxy}benzoic acid (Compound No. 1-168),
3-{[6-(3-ethylphenoxy)-1-methyl-1H-benzimidazol-2-yl]methoxy}benzoic acid (Compound No. 1-170),
3-{[6-(3-methoxyphenoxy)-1-methyl-1H-benzimidazol-2-yl]methoxy}benzoic acid (Compound No. 1-173),
3-({1-methyl-6-[3-(trifluoromethoxy)phenoxy]-1H-benzimidazol-2-yl}methoxy)benzoic acid (Compound No. 1-176),
3-{[6-(2,5-difluorophenoxy)-1-methyl-1H-benzimidazol-2-yl]methoxy}benzoic acid (Compound No. 1-179),
3-{[6-(3,4-dimethylphenoxy)-1-methyl-1H-benzimidazol-2-yl]methoxylbenzoic acid (Compound No. 1-198),
3-{[6-(3-methoxy-4-methylphenoxy)-1-methyl-1H-benzimidazol-2-yl]methoxy}benzoic acid (Compound No. 1-204),
3-{[6-(2,3-dihydro-1-benzofuran-6-yloxy)-1-methyl-1H-benzimidazol-2-yl]methoxylbenzoic acid (Compound No. 1-210),
3-{[3-methyl-5-(4-methylphenoxy)-3H-imidazo[4,5-b]pyridin-2-yl]methoxy}benzoic acid (Compound No. 1-221),
3-fluoro-5-{[3-methyl-5-(4-methylphenoxy)-3H-imidazo[4,5-b]pyridin-2-yl]methoxy}benzoic acid (Compound No. 1-222) and
3-{[5-(3,4-dimethylphenoxy)-3-methyl-3H-imidazo[4,5-b]pyridin-2-yl]methoxy}benzoic acid (Compound No. 1-223).

Still more preferred compounds are
3-{[6-(3-fluorophenoxy)-1-methyl-1H-benzimidazol-2-yl]methoxy}benzoic acid (Compound No. 1-35),
3-[6-(3-chlorophenoxy)-1-methyl-1H-benzimidazol-2-ylmethoxy]benzoic acid (Compound No. 1-51),
3-{[6-(4-chloro-3-fluorophenoxy)-1-methyl-1H-benzimidazol-2-yl]methoxy}benzoic acid (Compound No. 1-99),
3-{[6-(3-chloro-4-fluorophenoxy)-1-methyl-1H-benzimidazol-2-yl]methoxy}benzoic acid (Compound No. 1-115),
3-{[6-(2-fluorophenoxy)-1-methyl-1H-benzimidazol-2-yl]methoxy}benzoic acid (Compound No. 1-164),
3-{[1-methyl-6-(4-methylphenoxy)-1H-benzimidazol-2-yl]methoxy}benzoic acid (Compound No. 1-168),
3-{[6-(2,5-difluorophenoxy)-1-methyl-1H-benzimidazol-2-yl]methoxy}benzoic acid (Compound No. 1-179) and
3-{[6-(2,3-dihydro-1-benzofuran-6-yloxy)-1-methyl-1H-benzimidazol-2-yl]methoxy}benzoic acid (Compound No. 1-210).

### Advantages of the Invention

The fused bicyclic heteroaryl derivatives or pharmacologically acceptable salts thereof having the general formula (I) according to the present invention have been found to have an excellent hypoglycemic effect, an effect of improving carbohydrate or lipid metabolism, an effect of improving insulin resistance or an effect of improving so-called metabolic syndrome such as arteriosclerosis, hypertension, cardiovascular disorder or complications derived from them or a pathology caused by various inflammations. It has also been found that the compounds are ligands acting on PPARγ and therefore have an effect of inhibiting the growth of cancer cells. The compounds are useful in a therapeutic agent or prophylactic agent for metabolic syndrome, specifically, a disease such as diabetes, hyperglycemia, hyperlipidemia, adiposity, impaired glucose tolerance (IGT), insulin resistance, impaired fasting glucose (IFG), hypertension, fatty liver, nonalcoholic steatohepatitis (NASH), diabetic complications (such as retinopathy, nephropathy or neuropathy), arteriosclerosis, gestational diabetes mellitus (GDM) or polycystic ovary syndrome (PCOS), inflammatory disease (such as osteoarthritis, pain or inflammatory enteritis), acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease (such as systemic lupus erythematosus, chronic rheumatism, Sjogren's syndrome, systemic sclerosis, mixed connective tissue disease, Hashimoto's disease, Crohn's disease, ulcerative colitis, idiopathic Addison's disease, male sterility, Goodpasture's syndrome, rapidly progressive glomerulonephritis, myasthenia gravis, polymyositis, multiple sclerosis, autoimmune hemolytic anemia, idiopathic thrombocytopenic purpura, Behcet's disease or CREST syndrome), pancreatitis, cachexia, cancer (such as gastric cancer, lung cancer, breast cancer, colon cancer, prostate cancer, pancreatic cancer or liver cancer), leukemia, sarcoma (such as liposarcoma), osteoporosis, involutional osteoporosis, neurodegenerative disease, Alzheimer's disease, hyperuricemia or dry eyes. The compounds can also be used as a drug for the treatment and/or prevention of the aforementioned diseases.

### Brief Description of the Drawings

Figure 1 is a schematic diagram of a PPARγ expression plasmid which is referred to in Test Example 1;
Figure 2 is a schematic diagram of a PPRE reporter plasmid which is referred to in Test Example 1; and
Figure 3 is a conceptual diagram of a dose-dependent curve which is referred to in Test Example 1.

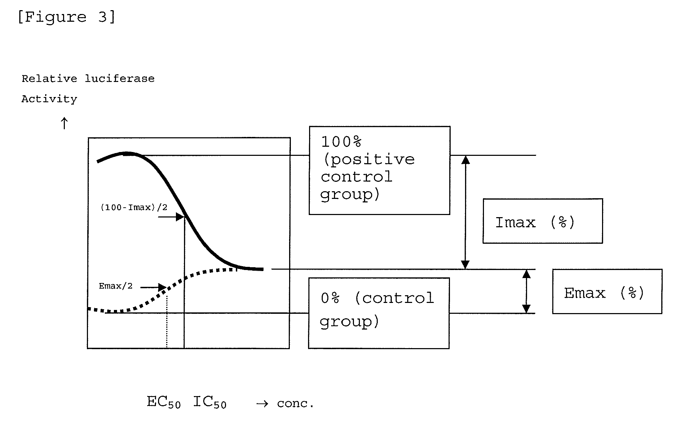

In Figure 3, the luciferase activity of the positive control group is defined as 100% and the luciferase activity of the control group is defined as 0%. The maximum luciferase activity exhibited by the test compound alone is defined as Emax (%) and the maximum inhibition of luciferase activity exhibited by the test compound in the presence of Compound A is defined as Imax (%). The drug concentration of a partial agonist represented by Emax/2 is defined as EC₅₀ and the drug concentration of a partial antagonist represented by (100-Imax)/2 is defined as IC₅₀. ---- indicates a concentration in the presence of Compound A and ..... indicates a concentration in the absence of Compound A.

### Best Mode for Carrying Out the Invention

The compound having the general formula (I) according to the present invention can be produced according to Processes A to C described below.

The solvent used in the reaction in each step of the following Processes A to C is not particularly limited insofar as it does not inhibit the reaction and dissolves the starting material to some extent. The solvent is selected from the following solvent group, for example. The solvent group consists of hydrocarbons such as pentane, hexane, octane, petroleum ether, ligroin and cyclohexane; amides such as formamide, N,N-dimethylformamide, N,N-dimethylacetamide, N-methyl-2-pyrrolidone, N-methyl-2-pyrrolidinone and hexamethylphosphoric triamide; ethers such as diethyl ether, diisopropyl ether, tetrahydrofuran, dioxane, dimethoxyethane and diethylene glycol dimethyl ether; alcohols such as methanol, ethanol, n-propanol, i-propanol, n-butanol, 2-butanol, 2-methyl-1-propanol, t-butanol, isoamyl alcohol, diethylene glycol, glycerol, octanol, cyclohexanol and methyl cellosolve; sulfoxides such as dimethyl sulfoxide; sulfones such as sulfolane; nitriles such as acetonitrile, propionitrile, butyronitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, chlorobenzene, dichlorobenzene, chloroform and carbon tetrachloride; aromatic hydrocarbons such as benzene, toluene and xylene; carboxylic acids such as acetic acid, formic acid, propionic acid, butyric acid and trifluoroacetic acid; water; and mixed solvents thereof.

Examples of the base used in the reaction in each step of the following Processes A to C include inorganic bases such as alkali metal carbonates such as sodium carbonate, potassium carbonate, lithium carbonate and cesium carbonate; alkali metal bicarbonates such as sodium bicarbonate, potassium bicarbonate and lithium bicarbonate; alkali metal hydrides such as lithium hydride, sodium hydride and potassium hydride; alkali metal hydroxides such as sodium hydroxide, potassium hydroxide, barium hydroxide and lithium hydroxide; and alkali metal fluorides such as sodium fluoride and potassium fluoride; alkali metal alkoxides such as sodium methoxide, sodium ethoxide, sodium t-butoxide, potassium methoxide, potassium ethoxide, potassium t-butoxide and lithium methoxide; alkali metal trialkylsilanolates such as sodium trimethylsilanolate, potassium trimethylsilanolate and lithium trimethylsilanolate; alkali metal mercaptans such as sodium methyl mercaptan and sodium ethyl mercaptan; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU); and organometallic bases such as butyllithium, lithium diisopropylamide and lithium bis(trimethylsilyl)amide.

In the reaction in each step of the following Processes A to C, the reaction temperature varies according to the solvent, the starting material, the reagent and the like, and the reaction time varies according to the solvent, the starting material, the reagent, the reaction temperature and the like.

In the reaction in each step of the following Processes A to C, each desired compound is collected from the reaction mixture according to conventional methods after completion of the reaction. The desired compound is obtained as follows, for example. The reaction mixture is appropriately neutralized and insoluble matter, if present, is removed by filtration. Then, water and an immiscible organic solvent such as ethyl acetate are added, and the organic layer containing the desired compound is separated. The organic layer is washed with water or the like and then dried over anhydrous magnesium sulfate, anhydrous sodium sulfate, anhydrous sodium bicarbonate or the like and filtered. Then, the solvent is evaporated. The resulting desired compound may be isolated and purified if necessary by appropriately combining usual methods, for example, methods suitably used for isolation and purification of organic compounds such as recrystallization and reprecipitation and eluting with an appropriate eluent by application of chromatography. The desired compound insoluble in a solvent may be purified by washing the resulting solid crude product with a solvent. The desired compound in each step may also be used as is for the next reaction without purification.

The reaction in each step of Processes A to C will be described below.

Process A is a process for producing a compound having the general formula (I).

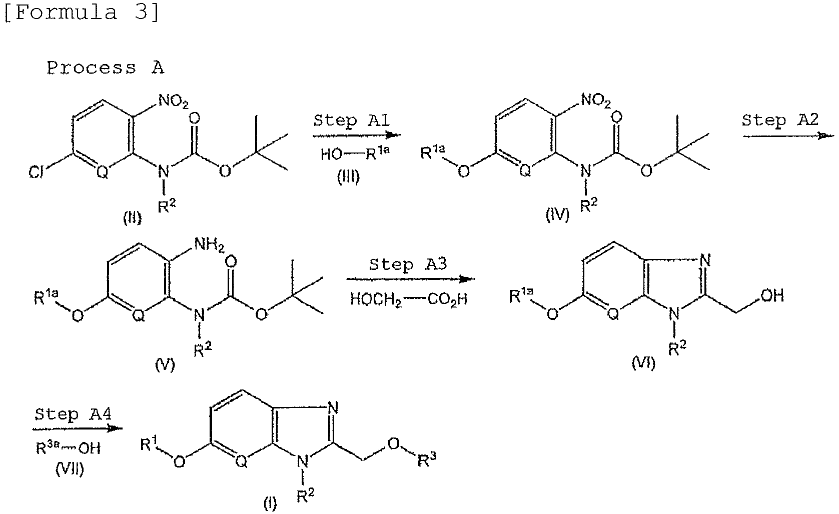

In the present invention, R¹, R², R³ and Q are as defined above, and R^{1a} and R^{3a} are the same groups as R¹ and R³ defined, except that the amino group, the hydroxy group and/or the carboxyl group contained as a substituent(s) in the R¹ and R³ groups are an amino group, a hydroxy group and/or a carboxyl group which may be protected.

### Step A1

This step is a step of producing a compound having the general formula (IV).

This step is carried out by reacting a compound having the general formula (II), which is a known compound or is easily obtained from a known compound as a starting material by a method similar to a known method, with a compound having the general formula (III), which is a known compound or is easily obtained from a known compound as a starting material by a method similar to a known method, in a solvent in the presence of a base.

The solvent used in this step is preferably an amide, and more preferably N,N-dimethylformamide or N-methyl-2-pyrrolidone.

The base used in this step is preferably an alkali metal carbonate or an alkali metal hydride, and more preferably cesium carbonate or sodium hydride.

The reaction temperature in this step is usually 50°C to 150°C, and preferably 80°C to 120°C.

The reaction time in this step is usually 0.5 to 48 hours, and preferably 1 to 30 hours.

### Step A2

This step is a step of producing a compound having the general formula (V).

This step is carried out by reacting the compound having the general formula (IV) with iron in a solvent in the presence of ammonium chloride or by reducing the compound having the general formula (IV) in a solvent in the presence of a palladium catalyst in a hydrogen atmosphere.

The solvent used in this step is preferably an ether, an alcohol or water, more preferably tetrahydrofuran, methanol, ethanol or water, and still more preferably ethanol or a mixed solvent of ethanol and water.

The palladium catalyst used in this step is, for example, a divalent palladium catalyst or a zerovalent palladium catalyst, preferably palladium-active carbon, palladium (II) acetate, palladium (II) trifluoroacetate, palladium black, palladium (II) bromide, palladium (II) chloride, palladium (II) iodide, palladium (II) cyanide, palladium (II) nitrate, palladium (II) oxide, palladium (II) sulfate, dichlorobis(acetonitrile)palladium (II), dichlorobis(benzonitrile)palladium (II), dichloro(1,5-cyclooctadiene)palladium (II), acetylacetone palladium (II), palladium (II) sulfide, tris(dibenzylideneacetone)dipalladium (0), tetrakis(acetonitrile)palladium (II) tetrafluoroborate or an aryl chloride-palladium dimer, and more preferably palladium-active carbon.

The reaction temperature in this step is usually - 20°C to 120°C, and preferably 0°C to 100°C.

The reaction time in this step is usually 1 to 48 hours, and preferably 2 to 24 hours.

### Step A3

This step is a step of producing a compound having the general formula (VI).

This step is carried out by reacting the compound having the general formula (V) with glycolic acid in a solvent in the presence of hydrochloric acid (preferably 4 N hydrochloric acid).

The solvent used in this step is preferably an ether or water, more preferably dioxane or water, and still more preferably a mixed solvent of dioxane and water.

The reaction temperature in this step is usually 50°C to 150°C, and preferably 80°C to 120°C.

The reaction time in this step is usually 0.5 to 48 hours, and preferably 1 to 24 hours.

### Step A4

This step is a step of producing a compound having the general formula (I).

This step is carried out by reacting the compound having the general formula (VI) with a compound having the general formula (VII), which is a known compound or is easily obtained from a known compound as a starting material by a method similar to a known method, in a solvent in the presence of a condensing agent, and then removing the protecting group(s) for the amino group, the hydroxyl group and/or the carboxyl group in R^{1a} and/or R^{3a} as desired.

The solvent used in this step is preferably an aromatic hydrocarbon, and more preferably toluene.

Examples of the condensing agent used in this step include a combination of an azodicarboxylate and a tertiary phosphine, a combination of an azodicarboxylic amide and a tertiary phosphine, and (trialkylphosphoranylidene)acetonitrile. The condensing agent is preferably a combination of an azodicarboxylic amide and a tertiary phosphine, and more preferably a combination of tributylphosphine and 1,1'-(azodicarbonyl)dipiperidine.

The reaction temperature in this step is usually - 78°C to 120°C, and preferably 0°C to 50°C.

The reaction time in this step is usually 0.5 to 24 hours, and preferably 1 to 12 hours.

Process B is another process for producing a compound having the general formula (I).

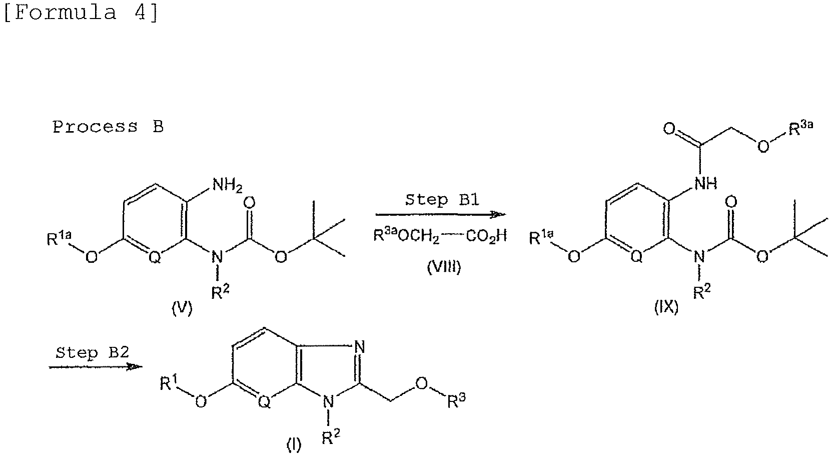

In the present invention, R¹, R², R³ Q, R^{1a} and R^{3a} are as defined above.

### Step B1

This step is a step of producing a compound having the general formula (IX).

This step is carried out by reacting a compound having the general formula (V) with a compound having the general formula (VIII), which is a known compound or is easily obtained from a known compound as a starting material by a method similar to a known method, in a solvent in the presence of a condensing agent and a base. The solvent used in this step is preferably an amide or a halogenated hydrocarbon, and more preferably N,N-dimethylformamide or dichloromethane.

Examples of the condensing agent used in this step include O-(7-azabenzotriazol-1-yl)-N,N,N',N'-tetramethyluronium hexafluorophosphate (HATU), 1-propanephosphonic acid cyclic anhydride (T3P), dicyclohexylcarbodiimide (DCCD), 1-ethyl-3-(3-dimethylaminopropyl)carbodiimide hydrochloride (EDCI), 1-ethyl-3-(3-dimethylaminopropyl)carbodiimide (EDAC), isobutyl chloroformate (IBCF), 1,1'-carbonylbis-1H-imidazole (CDI), diethyl cyanophosphonate (DEPC), diphenylphosphoryl azide (DPPA), N-hydroxysuccinimide, N-hydroxy-5-norbornene-2,3-dicarboxyimide and dipyridyl disulfide. The condensing agent may be used in the presence of 1-hydroxybenzotriazole or 1-hydroxybenzotriazole hydrate (HOBt) as necessary. The condensing agent is preferably EDCI.

The base used in this step is preferably triethylamine, N-methylmorpholine or 4-(N,N-dimethylamino)pyridine.

The reaction temperature in this step is usually - 50°C to 100°C, and preferably -20°C to 60°C.

The reaction time in this step is usually 0.1 to 24 hours, and preferably 0.5 to 10 hours.

### Step B2

This step is a step of producing a compound having the general formula (I).

This step is carried out by reacting the compound having the general formula (IX) with hydrochloric acid and then removing the protecting group(s) for the amino group, the hydroxy group and/or the carboxyl group in R^{1a} and/or R^{3a} as desired.

The reaction temperature in this step is usually - 20°C to 150°C, and preferably 0°C to 100°C.

The reaction time in this step is usually 0.5 to 150 hours, and preferably 1 to 72 hours.

Process C is another process for producing a compound having the general formula (I).

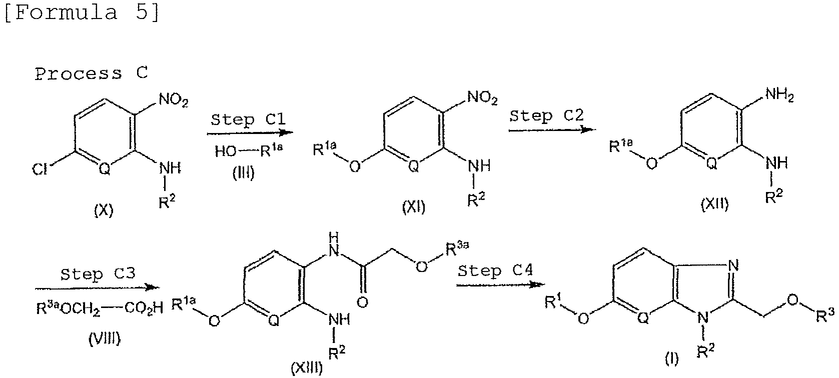

In the present invention, R¹, R², R³, Q, R^{1a} and R^{3a} are as defined above.

### Step C1

This step is a step of producing a compound having the general formula (XI).

This step is carried out by reacting a compound having the general formula (X), which is a known compound or is easily obtained from a known compound as a starting material by a method similar to a known method, with a compound having the general formula (III) in a solvent in the presence of a base.

The solvent used in this step is preferably an amide, and more preferably N,N-dimethylformamide or N-methyl-2-pyrrolidone.

The base used in this step is preferably an alkali metal hydride, and more preferably sodium hydride.

The reaction temperature in this step is usually - 78°C to 150°C, and preferably 0°C to 100°C.

The reaction time in this step is usually 0.5 to 48 hours, and preferably 1 to 24 hours.

### Step C2

This step is a step of producing a compound having the general formula (XII).

This step is carried out in the same manner as in Step A2 of the above Process A by reacting the compound having the general formula (XI) with iron in a solvent in the presence of ammonium chloride or by reducing the compound having the general formula (XI) in a solvent in the presence of a palladium catalyst in a hydrogen atmosphere.

### Step C3

This step is a step of producing a compound having the general formula (XIII).

This step is carried out in the same manner as in Step B1 of the above Process B by reacting the compound having the general formula (XII) with a compound having the general formula (VIII) in a solvent in the presence of a condensing agent and a base.

### Step C4

This step is a step of producing a compound having the general formula (I).

This step is carried out by reacting the compound having the general formula (XIII) with acetic acid in the same manner as in Step B2 of the above Process B and then removing the protecting group(s) for the amino group, the hydroxy group and/or the carboxyl group in R^{1a} and/or R^{3a} as desired.

The raw material compound having the general formula (II), (III), (VII), (VIII) or (X) is a known compound or is easily produced from a known compound as a starting material by a known method or a method similar to the method.

The protecting group for the "amino group which may be protected", "hydroxy group which may be protected" and "carboxyl group which may be protected" as defined above for R^{1a} and R^{3a} refers to a protecting group that can be cleaved by a chemical method such as hydrogenolysis, hydrolysis, electrolysis or photolysis and represents a protecting group generally used in organic synthesis chemistry (see T. W. Greene et al., Protective Groups in Organic Synthesis, 3rd Edition, John Wiley & Sons, Inc. (1999), for example).

The "protecting group" for the "hydroxy group which may be protected" as defined above for R^{1a} and R^{3a} is not particularly limited insofar as it is a protecting group for a hydroxy group used in the field of organic synthesis chemistry; the protecting group is a "general protecting group for a hydroxy group which is an ester", for example. Preferable examples of the protecting group include a formyl group; "alkylcarbonyl groups" such as the above "C₂-C₇ alkylcarbonyl groups", halogenated alkylcarbonyl groups such as chloroacetyl, dichloroacetyl, trichloroacetyl and trifluoroacetyl, alkoxyalkylcarbonyl groups such as methoxyacetyl, and unsaturated alkylcarbonyl groups such as acryloyl, propioloyl, methacryloyl, crotonoyl, isocrotonoyl and (E)-2-methyl-2-butenoyl; "arylcarbonyl groups" such as arylcarbonyl groups such as benzoyl, α-naphthoyl and β-naphthoyl, halogenated arylcarbonyl groups such as 2-bromobenzoyl and 4-chlorobenzoyl, C₁-C₆ alkylated arylcarbonyl groups such as 2,4,6-trimethylbenzoyl and 4-toluoyl, C₁-C₆ alkoxylated arylcarbonyl groups such as 4-anisoyl, nitrated arylcarbonyl groups such as 4-nitrobenzoyl and 2-nitrobenzoyl, C₂-C₇ alkoxycarbonylated arylcarbonyl groups such as 2-(methoxycarbonyl)benzoyl, and arylated arylcarbonyl groups such as 4-phenylbenzoyl; "alkoxycarbonyl groups" such as the above "C₂-C₇ alkoxycarbonyl groups", and C₂-C₇ alkoxycarbonyl groups substituted with halogen or tri-(C₁-C₆ alkyl)silyl group such as 2,2,2-trichloroethoxycarbonyl and 2-trimethylsilylethoxycarbonyl; "tetrahydropyranyl or tetrahydrothiopyranyl groups" such as tetrahydropyran-2-yl, 3-bromotetrahydropyran-2-yl, 4-methoxytetrahydropyran-4-yl, tetrahydrothiopyran-2-yl and 4-methoxytetrahydrothiopyran-4-yl; "tetrahydrofuranyl or tetrahydrothiofuranyl groups" such as tetrahydrofuran-2-yl and tetrahydrothiofuran-2-yl; "silyl groups" such as tri-(C₁-C₆ alkyl)silyl groups such as trimethylsilyl, triethylsilyl, isopropyldimethylsilyl, t-butyldimethylsilyl, methyldiisopropylsilyl, methyldi-t-butylsilyl and triisopropylsilyl, and (C₁-C₆ alkyl)diarylsilyl or di-(C₁-C₆ alkyl)arylsilyl groups such as diphenylmethylsilyl, diphenylbutylsilyl, diphenylisopropylsilyl and phenyldiisopropylsilyl; "alkoxymethyl groups" such as (C₁-C₆ alkoxy)methyl groups such as methoxymethyl, 1,1-dimethyl-1-methoxymethyl, ethoxymethyl, propoxymethyl, isopropoxymethyl, butoxymethyl and t-butoxymethyl, (C₁-C₆ alkoxy)-(C₁-C₆ alkoxy)methyl groups such as 2-methoxyethoxymethyl, and (C₁-C₆ halogenated alkoxy)methyl groups such as 2,2,2-trichloroethoxymethyl and bis(2-chloroethoxy)methyl; "substituted ethyl groups" such as (C₁-C₆ alkoxy)ethyl groups such as 1-ethoxyethyl and 1-(isopropoxy)ethyl, and halogenated ethyl groups such as 2,2,2-trichloroethyl; "aralkyl groups" such as C₁-C₆ alkyl groups substituted with 1 to 3 aryl group(s) such as benzyl, α-naphthylmethyl, β-naphthylmethyl, diphenylmethyl, triphenylmethyl, α-naphthyldiphenylmethyl and 9-anthrylmethyl, and C₁-C₆ alkyl groups substituted with 1 to 3 aryl group(s) having an aryl ring substituted with a C₁-C₆ alkyl, C₁-C₆ alkoxy, nitro, halogen or cyano group such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl and 4-cyanobenzyl; "alkenyloxycarbonyl groups" such as vinyloxycarbonyl and allyloxycarbonyl; and "aralkyloxycarbonyl groups" having an aryl ring which may be substituted with 1 or 2 C₁-C₆ alkoxy or nitro group(s) such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl. Alkylcarbonyl groups, silyl groups or aralkyl groups are more preferable.

The "protecting group" for the "carboxyl group which may be protected" as defined above for R^{1a} and R^{3a} is not particularly limited insofar as it is a protecting group for a carboxyl group used in the field of organic synthesis chemistry; the protecting group is a "general protecting group for a carboxyl group which is an ester", for example. Preferable examples of the protecting group include the above "C₁-C₆ alkyl groups"; "C₂-C₆ alkenyl groups" such as ethenyl, 1-propenyl and 2-propenyl; "C₂-C₆ alkynyl groups" such as ethynyl, 1-propynyl and 2-propynyl; the above "C₁-C₆ halogenated alkyl groups"; the above "C₁-C₆ hydroxyalkyl groups"; (C₂-C₇ alkylcarbonyl)-(C₁-C₆ alkyl) groups such as acetylmethyl; the above "aralkyl groups"; and the above "silyl groups". C₁-C₆ alkyl groups or aralkyl groups are more preferable.

The "protecting group" for the "amino group which may be protected" as defined above for R^{1a} and R^{3a} is not particularly limited insofar as it is a protecting group for an amino group used in the field of organic synthesis chemistry; the protecting group is the same "alkylcarbonyl group"; "arylcarbonyl group"; "alkoxycarbonyl group"; "silyl group"; "aralkyl group"; "alkenyloxycarbonyl group"; or "aralkyloxycarbonyl group" as in the "general protecting group for a hydroxy group which is an ester" or a "substituted methylene group forming a Schiff base" such as N,N-dimethylaminomethylene, benzylidene, 4-methoxybenzylidene, 4-nitrobenzylidene, salicylidene, 5-chlorosalicylidene, diphenylmethylene or (5-chloro-2-hydroxyphenyl)phenylmethylene, for example, and is preferably an alkylcarbonyl group, an arylcarbonyl group or an alkoxycarbonyl group, and more preferably an alkoxycarbonyl group.

The steps involving protection and deprotection are carried out according to known methods (such as a method described in "Protective Groups in Organic Synthesis" (Theodora W. Greene, Peter G. M. Wuts, 1999, published by A Wiley-Interscience Publication)).

The fused bicyclic heteroaryl derivative or pharmacologically acceptable salt thereof having the general formula (I) according to the present invention used as a medicine can be orally administered as tablets, capsules, granules, powder or syrup or parenterally administered as an injection or suppository, for example, alone or in a mixture with an appropriate pharmacologically acceptable excipient, diluent or the like.

These preparations are produced by known methods using additives such as excipients (whose examples include organic excipients such as sugar derivatives such as lactose, sucrose, glucose, mannitol and sorbitol; starch derivatives such as corn starch, potato starch, α-starch and dextrin; cellulose derivatives such as crystalline cellulose; gum arabic; dextran; and pullulan; and inorganic excipients such as silicate derivatives such as light silicic anhydride, synthetic aluminum silicate, calcium silicate and magnesium aluminometasilicate; phosphates such as calcium hydrogenphosphate; carbonates such as calcium carbonate; and sulfates such as calcium sulfate), lubricants (whose examples include stearic acid and stearic acid metal salts such as calcium stearate and magnesium stearate; talc; colloidal silica; waxes such as veegum and spermaceti; boric acid; adipic acid; sulfates such as sodium sulfate; glycol; fumaric acid; sodium benzoate; DL-leucine; fatty acid sodium salts; lauryl sulfates such as sodium lauryl sulfate and magnesium lauryl sulfate; silicic acids such as silicic anhydride and silicic acid hydrate; and the aforementioned starch derivatives), binders (whose examples include hydroxypropylcellulose, hydroxypropylmethylcellulose, polyvinylpyrrolidone, macrogol and the same compounds as the aforementioned excipients), disintegrants (whose examples include cellulose derivatives such as low-substituted hydroxypropylcellulose, carboxymethylcellulose, calcium carboxymethylcellulose and internally crosslinked sodium carboxymethylcellulose; and chemically modified starches such as carboxymethyl starch and sodium carboxymethyl starch), stabilizers (whose examples include parahydroxybenzoic acid esters such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), corrigents (whose examples include commonly used sweeteners, acidulants and flavors) and diluents.

The dose of the preparation varies according to the symptoms, the age and the like of the patient (a warm-blooded animal, in particular, a human). However, the preparation is preferably orally administered at 0.0015 mg/kg body weight (preferably 0.008 mg/kg body weight) per dose per day at the lower limit to 70 mg/kg body weight (preferably 7 mg/kg body weight) per dose per day at the upper limit or intravenously administered at 0.00015 mg/kg body weight (preferably 0.0008 mg/kg body weight) per dose per day at the lower limit to 8.5 mg/kg body weight (preferably 5 mg/kg body weight) per dose per day at the upper limit to an adult once to six times per day according to the symptoms.

### Examples

The present invention will be described in more detail below with reference to Examples, Test Examples and Preparation Examples; however, the scope of the present invention is not limited thereto.

Chromatographic elution in Examples was carried out under observation by TLC (Thin Layer Chromatography). In TLC observation, silica gel 60F₂₅₄ manufactured by Merck & Co., Inc. was used as the TLC plate, the solvent used as the elution solvent in column chromatography was used as the developing solvent, and a UV detector was used as the detection method. Silica gel SK-85 (230 to 400 mesh) or silica gel SK-34 (70 to 230 mesh) also manufactured by Merck & Co., Inc., or Chromatorex NH (200 to 350 mesh) manufactured by Fuji Silysia Chemical Ltd. was used as the column silica gel. An automatic chromatography system manufactured by Biotage AB (SP-1) was appropriately used in addition to a common column chromatography system. The abbrevations used in Examples have the following meanings:
mg: milligram, g: gram, mL: milliliter, MHz: megahertz.

In the following Examples, in nuclear magnetic resonance (hereinafter ¹H NMR) spectra, chemical shifts are described in δ values (ppm) using tetramethylsilane as a reference substance. For splitting patterns, s represents singlet, d represents doublet, t represents triplet, q represents quartet, quint represents quintet, and sep represents septet.

Mass spectrometry (hereinafter MS) was carried out by FAB (Fast Atom Bombardment), EI (Electron Ionization) or ESI (Electron Spray Ionization).

### (Example 1) Methyl 3-{[6-(4-amino-3,5-dimethylphenoxy)-1-methyl-1H-benzimidazol-2-yl]methoxy}benzoate dihydrochloride (dihydrochloride of Compound No. 1-132)

### (1a) [6-(4-tert-Butoxycarbonylamino-3,5-dimethylphenoxy)-1-methyl-1H-benzimidazol-2-yl]methanol

[6-(4-Amino-3,5-dimethylphenoxy)-1-methyl-1H-benzimidazol-2-yl]methanol ((13 g, 43.7 mmol) Japanese Patent Laid-Open No. 2004-123711) and (Boc)₂O (19 g, 87 mmol) were dissolved in 150 mL of isopropanol, followed by stirring overnight. The reaction solution was diluted with ethyl acetate, washed with water and brine, and dried over sodium sulfate. Then, the solvent was evaporated. The residue was subjected to silica gel column chromatography (10% methanol-ethyl acetate). The resulting foam was crystallized from ethyl acetate and hexane to obtain the desired compound (4.5 g, yield: 26%).

¹H-NMR (CDCl₃, 400 MHz) δ: 1.26 (9H, s), 2.21 (6H, s), 3.75 (3H, s), 4.89 (2H, s), 6.67 (2H, s), 6.93 (1H, d, J = 2 Hz), 6.96 (1H, dd, J = 2, 9 Hz), 7.63 (1H, d, J = 9 Hz).

### (1b) Methyl 3-{[6-(4-amino-3,5-dimethylphenoxy)-1-methyl-1H-benzimidazol-2-yl]methoxy}benzoate dihydrochloride

Tri-n-butylphosphine (0.41 g, 2.0 mmol) and 1,1'-(azodicarbonyl)dipiperidine (0.50 g, 2.0 mmol) were added to a solution of {6-[4-(tert-butyloxycarbonylamino)-3,5-dimethylphenoxyl-1-methyl-1H-benzimidazol-2-yl}methanol (0.40 g, 1.0 mmol) and methyl 3-hydroxybenzoate (0.23 g, 1.5 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.32 g, yield: 63%) was obtained by drying under reduced pressure.

¹H-NMR (DMSO-d₆, 400 MHz) δ: 2.25 (6H, s), 3.87 (3H, s), 3.87 (3H, s), 5.60 (2H, s), 6.74 (2H, s), 7.03 (1H, d, J = 8.8 Hz), 7.41 (1H, s), 7.45 (1H, d, J = 8.3 Hz), 7.52 (1H, dd, J = 7.8, 8.3 Hz), 7.63 (1H, d, J = 7.8 Hz), 7.69 (1H, s), 7.72 (1H, d, J = 8.8 Hz).

MS (ESI+) m/z: 432 (M+H)⁺.

HRMS (ESI+) m/Z: 432.19037 (M+H)⁺, calcd 432.19233 (-1.96 mmu).

### (Example 2) 3-{[6-(4-Amino-3,5-dimethylphenoxy)-1-methyl-1H-benzimidazol-2-yl]methoxy}benzoic acid dihydrochloride (dihydrochloride of Compound No. 1-131)

A 1 N sodium hydroxide aqueous solution (10 mL, 10 mmol) was added to a solution of methyl 3-{[6-(4-amino-3,5-dimethylphenoxy)-1-methyl-1H-benzimidazol-2-yl]methoxy}benzoate dihydrochloride synthesized in Example 1 (0.22 g, 0.4 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.12 g, yield: 61%).

Mp 235-239°C,

¹H-NMR (DMSO-d₆, 400 MHz) δ: 2.30 (6H, s), 3.91 (3H, s), 5.65 (2H, s), 6.78 (2H, s), 7.11 (1H, dd, J = 2.0, 8.8 Hz), 7.43 (1H, d, J = 7.8 Hz), 7.49 (1H, dd, J = 7.8, 7.8 Hz), 7.51 (1H, d, J = 2.0 Hz), 7.63 (1H, d, J = 7.8 Hz), 7.76 (1H, d, J = 8.8 Hz).

MS (ESI+) m/z: 418 (M+H)⁺, 440 (M+Na)⁺, 462 (M+2Na-H)⁺.

HRMS (ESI+) m/Z: 418.18023 (M+H)⁺, calcd 418.17668 (3.55 mmu).

### (Example 3) Ethyl 4-{[6-(4-amino-3,5-dimethylphenoxy)-1-methyl-1H-benzimidazol-2-yl]methoxy}benzoate dihydrochloride (dihydrochloride of Compound No. 1-134)

Tri-n-butylphosphine (0.41 g, 2.0 mmol) and 1,1'-(azodicarbonyl)dipiperidine (0.50 g, 2.0 mmol) were added to a solution of {6-[4-(tert-butyloxycarbonylamino)-3,5-dimethylphenoxyl-1-methyl-1H-benzimidazol-2-yl}methanol (0.40 g, 1.0 mmol) and ethyl 4-hydroxybenzoate (0.25 g, 1.5 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.35 g, yield: 67%) was obtained by drying under reduced pressure.

¹H-NMR (DMSO-d₆, 400 MHz) δ: 1.31 (3H, t, J = 7.0 Hz), 2.12 (6H, s), 3.90 (3H, s), 4.29 (2H, q, J = 7.0 Hz), 5.66 (2H, s), 6.71 (2H, s), 7.10 (1H, d, J = 8.8 Hz), 7.29 (2H, d, J = 8.8 Hz), 7.51 (1H, s), 7.75 (1H, d, J = 8.8 Hz), 7.98 (2H, d, J = 8.8 Hz).

MS (ESI+) m/z: 446 (M+H)⁺.

HRMS (ESI+) m/Z: 446.20801 (M+H)⁺, calcd 446.20798 (0.03 mmu).

### (Example 4) 4-{[6-(4-Amino-3,5-dimethylphenoxy)-1-methyl-1H-benzimidazol-2-yl]methoxy}benzoic acid dihydrochloride (dihydrochloride of Compound No. 1-133)

A 1 N sodium hydroxide aqueous solution (10 mL, 10 mmol) was added to a solution of ethyl 4-{[6-(4-amino-3,5-dimethylphenoxy)-1-methyl-1H-benzimidazol-2-yl]methoxy}benzoate dihydrochloride (0.26 g, 0.4 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.15 g, yield: 77%).

¹H-NMR (DMSO-d₆, 400 MHz) δ: 2.30 (6H, s), 3.89 (3H, s), 5.64 (2H, s), 6.77 (2H, s), 7.08 (1H, d, J = 8.8 Hz), 7.25 (2H, d, J = 8.8 Hz), 7.49 (1H, s), 7.75 (1H, d, J = 8.8 Hz), 7.95 (2H, d, J = 8.8 Hz).

MS (ESI+) m/z: 418 (M+H)⁺.

HRMS (ESI+) m/Z: 418.17523 (M+H)⁺, calcd 418.17668 (-1.45 mmu).

### (Example 5) Ethyl 2-{[6-(4-amino-3,5-dimethylphenoxy)-1-methyl-1H-benzimidazol-2-yl]methoxy}benzoate dihydrochloride (dihydrochloride of Compound No. 1-130)

Tri-n-butylphosphine (0.41 g, 2.0 mmol) and 1,1'-(azodicarbonyl)dipiperidine (0.50 g, 2.0 mmol) were added to a solution of {6-[4-(tert-butyloxycarbonylamino)-3,5-dimethylphenoxy]-1-methyl-1H-benzimidazol-2-yl}methanol (0.40 g, 1.0 mmol) and ethyl 2-hydroxybenzoate (0.23 g, 1.5 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.34 g, yield: 65%) was obtained by drying under reduced pressure.

¹H-NMR (DMSO-d₆, 400 MHz) δ: 1.20 (3H, t, J = 7.2 Hz), 2.33 (6H, s), 3.94 (3H, s), 4.23 (2H, q, J = 7.2 Hz), 5.64 (2H, s), 6.79 (2H, s), 7.11 (1H, d, J = 8.8 Hz), 7.13 (1H, dd, J = 7.3, 7.8 Hz), 7.45 (1H, d, J = 8.4 Hz), 7.55 (1H, s), 7.60 (1H, ddd, J = 1.4, 7.3, 8.4 Hz), 7.72 (1H, dd, J = 1.4, 7.8 Hz), 7.77 (1H, d, J = 8.8 Hz).

MS (ESI+) m/z: 446 (M+H)⁺, 468 (M+Na)⁺.

HRMS (ESI+) m/Z: 446.21002 (M+H)⁺, calcd 446.20798 (2.04 mmu) .

### (Example 6) 2-{[6-(4-Amino-3,5-dimethylphenoxy)-1-methyl-1H-benzimidazol-2-yl]methoxy}benzoic acid dihydrochloride (dihydrochloride of Compound No. 1-129)

A 1 N sodium hydroxide aqueous solution (10 mL, 10 mmol) was added to a solution of ethyl 3-{[6-(4-amino-3,5-dimethylphenoxy)-1-methyl-1H-benzimidazol-2-yl]methoxy}benzoate dihydrochloride (0.26 g, 0.4 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.13 g, yield: 66%).

¹H-NMR (DMSO-d₆, 400 MHz) δ: 2.30 (6H, s), 3.92 (3H, s), 5.62 (2H, s), 6.87 (2H, s), 7.10 (1H, m), 7.11 (1H, m), 7.43 (1H, d, J = 8.3 Hz), 7.55 (1H, m), 7.58 (1H, s), 7.69 (1H, dd, J = 1.4, 7.6 Hz), 7.75 (1H, d, J = 8.8 Hz).

MS (ESI+) m/z: 418 (M+H)⁺.

HRMS (ESI+) m/Z: 418.17421 (M+H)⁺, calcd 418.17668 (-2.47 mmu).

### (Example 7) Methyl 3-{[6-(3-fluorophenoxy)-1-methyl-1H-benzimidazol-2-yl]methoxy}benzoate hydrochloride (hydrochloride of Compound No. 1-36)

### (7a) tert-Butyl [5-(3-fluorophenoxy)-2-nitrophenyl]methylcarbamate

Potassium tert-butoxide (3.93 g, 35.0 mmol) was added to a solution of 3-fluorophenol (3.53 g, 31.5 mmol) and tert-butyl (5-chloro-2-nitrophenyl)methylcarbamate (8.60 g, 30.0 mmol) in N,N-dimethylacetamide (15 mL) and tetrahydrofuran (60 mL) under ice-cooling, and the mixture was stirred at 100°C for 30 hours. The reaction solution was concentrated, and ethyl acetate (200 mL) and water (200 mL) were added thereto, followed by extraction. The organic layer was dried and concentrated to obtain the desired title compound (9.21 g, yield: 81%).

### (7b) (6-Fluoro-1-methyl-1H-benzimidazol-2-yl)methanol

Iron powder (7.53 g, 135.0 mmol) was added to a solution of tert-butyl [5-(3-fluorophenoxy)-2-nitrophenyl]methylcarbamate (9.21 g, 25.4 mmol) and ammonium chloride (0.80 g, 15.0 mmol) in water (30 mL) and ethanol (120 mL), and the mixture was stirred at 70°C for nine hours. The reaction solution was concentrated, and 4 N hydrochloric acid (90 mL) was added to the resulting brown solid. The mixture was stirred at 120°C for 30 minutes to obtain a homogeneous solution. Glycolic acid (6.84 g, 90.0 mmol) was added to the solution, and the mixture was stirred at 120°C for four hours. The reaction solution was concentrated, and then made basic by gradually adding a 2 N sodium hydroxide aqueous solution to precipitate a solid. The resulting solid was recrystallized from ethanol to obtain the desired title compound (4.90 g, yield: 71%).

¹H-NMR (DMSO-d₆, 400 MHz) δ: 3.80 (3H, s), 4.71 (2H, d, J = 5.5 Hz), 5.59 (1H, t, J = 5.5 Hz), 6.75-6.82 (2H, m), 6.87-6.97 (2H, m), 7.33-7.42 (2H, m), 7.62 (1H, d, J = 8.6 Hz).

### (7c) Methyl 3-{[6-(3-fluorophenoxy)-1-methyl-1H-benzimidazol-2-yl]methoxy}benzoate hydrochloride

Tri-n-butylphosphine (0.61 g, 3.0 mmol) and 1,1'-(azodicarbonyl)dipiperidine (0.76 g, 3.0 mmol) were added to a solution of [6-(3-fluorophenoxy)-1-methyl-1H-benzimidazol-2-yl]methanol (0.41 g, 1.5 mmol) and methyl 3-hydroxybenzoate (0.34 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.43 g, yield: 64%) was obtained by drying under reduced pressure.

¹H-NMR (DMSO-d₆, 400 MHz) δ: 3.87 (3H, s), 3.90 (3H, s), 5.62 (2H, s), 6.82 (1H, ddd, J = 0.8, 2.4, 8.2 Hz), 6.86 (1H, ddd, J = 2.4, 2.4, 8.2 Hz), 6.96 (1H, dddd, J = 0.8, 2.4, 8.4, 8.4 Hz), 7.14 (1H, dd, J = 2.2, 8.8 hz), 7.41 (1H, ddd, J = 6.9, 8.2, 8.4 Hz), 7.46 (1H, ddd, J = 1.1, 2.6, 8.2 Hz), 7.52 (1H, dd, J = 7.5, 8.2 Hz), 7.59 (1H, d, J = 2.2 Hz), 7.64 (1H, ddd, J = 1.1, 1.5, 7.5 Hz), 7.70 (1H, dd, J = 1.5, 2.6 Hz), 7.78 (1H, d, J = 8.8 Hz).

MS (ESI+) m/z: 407 (M+H)⁺, 429 (M+Na)⁺.

HRMS (ESI+) m/Z: 407.13957 (M+H)⁺, calcd 407.14071 (-1.14 mmu).

### (Example 8) 3-{[6-(3-Fluorophenoxy)-1-methyl-1H-benzimidazol-2-yl]methoxy}benzoic acid hydrochloride (hydrochloride of Compound No. 1-35)

A 1 N sodium hydroxide aqueous solution (15 mL, 15 mmol) was added to a solution of methyl 3-{[6-(3-fluorophenoxy)-1-methyl-1H-benzimidazol-2-yl]methoxy}benzoate hydrochloride synthesized in Example 7 (0.33 g, 0.74 mmol) in 1,4-dioxane, and the mixture was stirred at 60°C for two hours. The reaction solution was treated with concentrated hydrochloric acid (2.5 mL) and then concentrated. The resulting solid was washed with water and ethyl acetate and dried under reduced pressure to obtain the desired title compound (0.22 g, yield: 69%).

¹H-NMR (DMSO-d₆, 400 MHz) δ: 3.95 (3H, s), 5.70 (2H, s), 6.85 (1H, ddd, J = 0.8, 2.4, 8.2 Hz), 6.89 (1H, ddd, J = 2.3, 2.4, 8.2 Hz), 6.99 (1H, dddd, J = 0.8, 2.3, 8.2, 8.4 Hz), 7.24 (1H, dd, J = 2.2, 8.9 Hz), 7.42 (1H, ddd, J = 6.9, 8.2, 8.2 Hz), 7.45 (1H, ddd, J = 1.1, 2.6, 8.2 Hz), 7.51 (1H, dd, J = 7.5, 8.2 Hz), 7.64 (1H, ddd, J = 1.1, 1.3, 7.5 Hz), 7.69 (1H, d, J = 2.23 Hz), 7.71 (1H, dd, J = 1.3, 2.6 Hz), 7.84 (1H, d, J = 8.9 Hz).

MS (ESI+) m/z: 393 (M+H)⁺, 415 (M+Na)⁺, 437 (M+2Na-H)⁺.

HRMS (ESI+) m/Z: 393.12228 (M+H)⁺, calcd 393.12506 (-2.78 mmu).

### (Example 9) Methyl 3-{[6-(4-chloro-3-fluorophenoxy)-1-methyl-1H-benzimidazol-2-yl]methoxy}benzoate hydrochloride (hydrochloride of Compound No. 1-100)

### (9a) tert-Butyl [(4-chloro-3-fluorophenoxy)-2-nitrophenyl]-methyl-carbamate

Sodium hydride (> 56% in oil, 1.31 g, 30.0 mmol) was added to a solution of 4-chloro-3-fluorophenol (4.94 g, 30.0 mmol) and tert-butyl (5-chloro-2-nitrophenyl)-methyl-carbamate (8.60 g, 30.0 mmol) in N,N-dimethylformamide (150 mL) under ice-cooling. The mixture was gradually heated to room temperature and then heated to 80°C, followed directly by stirring for eight hours. The reaction solution was further allowed to stand at room temperature overnight and then stirred at 80°C again for one hour. After leaving to cool to room temperature, water and brine were added to the reaction solution, followed by extraction with ethyl acetate three times. The organic layers were combined and dried over magnesium sulfate, and the solvent was evaporated under reduced pressure. The residue was purified by silica gel chromatography to obtain the desired compound (12.63 g, yield: 100%) as pale yellow needle crystals.

¹H-NMR (CDCl₃, 400 MHz) δ: 1.32 (9H, s), 3.26 (3H, s), 6.85-6.92 (4H, m), 7.43 (1H, t, J = 8.6 Hz), 7.93 (1H, t, J = 8.6 Hz).

### (9b) [6-(4-Chloro-3-fluorophenoxy)-1-methyl-1H-benzimidazol-2-yl]methanol

tert-Butyl [(4-chloro-3-fluorophenoxy)-2-nitrophenyl]-methyl-carbamate (12.62 g, 30.0 mmol) was dissolved in ethanol (150 mL), water (75 mL). Then, iron powder (8.03 g, 150 mmol) and ammonium chloride (803.2 mg, 15.0 mmol) were added and the mixture was heated under reflux for 4.5 hours. The reaction solution was left to cool, and then diluted with water, brine and ethyl acetate and filtered through celite. The filtrate was separated and the organic layer was dried over magnesium sulfate. Then, the solvent was evaporated under reduced pressure to obtain tert-butyl [2-amino-5-(4-chloro-3-fluorophenoxy)-phenyl]-methyl-carbamate as a white solid (12.01 g, yield: 100%). Glycolic acid (3.42 g, 45.0 mmol) and a 4 N hydrochloric acid-1,4-dioxane solution (150 mL) were added thereto, and the mixture was heated under reflux for two hours. The reaction solution was slowly poured into a saturated sodium bicarbonate aqueous solution under ice-cooling. Diisopropyl ether was further added, followed by stirring. After several minutes, a pale yellow powder was generated. The powder was collected by filtration, sequentially washed with a mixed solution of ethyl acetate and n-hexane, and water, and dried to obtain the desired compound (3.85 g, yield: 42%) as a pale yellow powder.

H-NMR (CDCl₃, 400 MHz) δ: 3.78 (3H, s), 4.88 (2H, s), 6.69 (1H, dd, J = 3.9, 10.1 Hz), 6.73 (1H, dd, J = 3.2, 10.1 Hz), 6.93-6.95 (2H, m), 7.28 (1H, t, J = 8.7 Hz), 7.05 (1H, d, J = 8.7 Hz).

### (9c) Methyl 3-{[6-(4-chloro-3-fluorophenoxy)-1-methyl-1H-benzimidazol-2-yl]methoxy}benzoate hydrochloride

Tri-n-butylphosphine (0.61 g, 3.0 mmol) and 1,1'-(azodicarbonyl)dipiperidine (0.76 g, 3.0 mmol) were added to a solution of [6-(4-chloro-3-fluorophenoxy)-1-methyl-1H-benzimidazol-2-yl]methanol (0.46 g, 1.5 mmol) and methyl 3-hydroxybenzoate (0.34 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.44 g, yield: 61%) was obtained by drying under reduced pressure.

¹H-NMR (DMSO-d₆, 400 MHz) δ: 3.87 (3H, s), 3.92 (3H, s), 5.66 (2H, s), 6.88 (1H, ddd, J = 1.3, 2.8, 8.9 Hz), 7.15 (1H, dd, J = 2.8, 10.8 Hz), 7.21 (1H, dd, J = 2.2, 8.8 Hz), 7.47 (1H, ddd, J = 1.1, 2.6, 8.3 Hz), 7.53 (1H, dd, J = 7.5, 8.3 Hz), 7.59 (1H, dd, J = 8.8, 8.9 Hz), 7.64 (1H, ddd, J = 1.1, 1.5, 7.5 Hz), 7.65 (1H, d, J = 2.2 Hz), 7.71 (1H, dd, J = 1.5, 2.6 Hz), 7.81 (1H, d, J = 8.8 Hz).

MS (ESI+) m/z: 441 (M+H)⁺, 443 (M+H+2)⁺, 463 (M+Na)⁺, 465 (M+Na+2)⁺.

HRMS (ESI+) m/Z: 441.10171 (M+H)⁺, calcd 441.10174 (-0.03 mmu).

### (Example 10) 3-{[6-(4-Chloro-3-fluorophenoxy)-1-methyl-1H-benzimidazol-2-yl]methoxy}benzoic acid hydrochloride (hydrochloride of Compound No. 1-99)

A 1 N sodium hydroxide aqueous solution (10 mL, 10 mmol) was added to a solution of methyl 3-{[6-(4-chloro-3-fluorophenoxy)-1-methyl-1H-benzimidazol-2-yl]methoxy}benzoate hydrochloride synthesized in Example 9 (0.33 g, 0.69 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.23 g, yield: 72%).

Mp 197-201°C.

¹H-NMR (DMSO-d₆, 400 MHz) δ: 3.94 (3H, s), 5.68 (2H, s), 6.88 (1H, ddd, J = 1.3, 2.8, 8.9 Hz), 7.16 (1H, dd, J = 2.8, 10.7 Hz), 7.23 (1H, dd, J = 2.2, 8.8 Hz), 7.44 (1H, ddd, J = 1.1, 2.6, 8.3 Hz), 7.50 (1H, dd, J = 7.6, 8.3 Hz), 7.59 (1H, dd, J = 8.8, 8.9 Hz), 7.63 (1H, ddd, J = 1.1, 1.3, 7.6 Hz), 7.68 (1H, d, J = 2.2 Hz), 7.70 (1H, dd, J = 1.3, 2.6 Hz), 7.82 (1H, d, J = 8.8 Hz).

MS (ESI+) m/z: 427 (M+H)⁺, 429 (M+H+2)⁺, 449 (M+Na)⁺, 451 (M+Na+2)⁺.

HRMS (ESI+) m/Z: 427.08529 (M+H)⁺, calcd 427.08609 (-0.80 mmu).

### (Example 11) Methyl 3-{[6-(3-chloro-4-fluorophenoxy)-1-methyl-1H-benzimidazol-2-yl]methoxy}benzoate hydrochloride (hydrochloride of Compound No. 1-116)

### (11a) tert-Butyl [(3-chloro-4-fluorophenoxy)-2-nitrophenyl]-methyl-carbamate

The desired compound (15.50 g, yield: 100%) was obtained as pale yellow needle crystals by synthesis from 3-chloro-4-fluorophenol (5.97 g, 36.7 mmol), tert-butyl (5-chloro-2-nitrophenyl)-methyl-carbamate (10.40 g, 36.3 mmol), sodium hydride (> 56% in oil, 1.58 g, 36.3 mmol) and N,N-dimethylformamide (200 mL) in the same manner as in Example 7a.

¹H-NMR (CDCl₃, 400 MHz) δ: 1. 32 (9H, s), 3.26 (3H, s), 6.79-6.85 (2H, m), 6.95-6.97 (1H, m), 7.15-7.18 (2H, m), 7.91-7.93 (1H, m).

### (11b) tert-Butyl [2-amino-5-(3-chloro-4-fluorophenoxy)-phenyl]-methyl-carbamate

The desired compound was obtained as pale brown crystals (6.98 g, yield: 93%) by synthesis from tert-butyl [(3-chloro-4-fluorophenoxy)-2-nitrophenyl]-methyl-carbamate (7.51 g, 18.1 mmol), iron powder (4.84 g, 90.5 mmol), ammonium chloride (0.48 g, 9.05 mmol), ethanol (100 mL) and water (50 mL) in the same manner as in Example 7b.

¹H-NMR (CDCl₃, 400 MHz) δ: 1.58 (9H, s), 3.14 (3H, s), 3.72 (1H, broad), 6.74-6.82 (4H, m), 6.90-6.99 (1H, m), 7.05 (1H, t, J = 8.8 Hz).

### (11c) [6-(3-Chloro-4-fluorophenoxy)-1-methyl-1H-benzimidazol-2-yl]methanol

The desired compound was obtained as pale brown crystals (2.42 g, yield: 48%) by synthesis from tert-butyl [2-amino-5-(3-chloro-4-fluorophenoxy)-phenyl]-methyl-carbamate (6.89 g, 16.6 mmol), glycolic acid (1.89 g, 24.9 mmol) and a 4 N hydrochloric acid-1,4-dioxane solution (120 mL) in the same manner as in Example 7b.

H-NMR (CDCl₃, 400 MHz) δ: 3.78 (3H, s), 4.89 (2H, s), 6.85-7.00 (4H, m), 7.09 (1H, t, J = 8.6 Hz), 7.05 (1H, d, J = 8.8 Hz).

### (11d) Methyl 3-{[6-(3-chloro-4-fluorophenoxy)-1-methyl-1H-benzimidazol-2-yl]methoxy}benzoate hydrochloride

Tri-n-butylphosphine (0.61 g, 3.0 mmol) and 1,1'-(azodicarbonyl)dipiperidine (0.76 g, 3.0 mmol) were added to a solution of [6-(3-chloro-4-fluorophenoxy)-1-methyl-1H-benzimidazol-2-yl]methanol (0.46 g, 1.5 mmol) and methyl 3-hydroxybenzoate (0.34 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.48 g, yield: 67%) was obtained by drying under reduced pressure.

¹H-NMR (DMSO-d₆, 400 MHz) δ: 3.87 (3H, s), 3.92 (3H, s), 5.66 (2H, s), 7.06 (1H, ddd, J = 3.0, 3.9, 9.1 Hz), 7.19 (1H, dd, J = 2.2, 8.9 Hz), 7.28 (1H, dd, J = 3.0, 6.3 Hz), 7.46 (1H, dd, J = 8.9, 9.1 Hz), 7.47 7 (1H, ddd, J = 1.1, 2.2, 8.2 Hz), 7.53 (1H, dd, J = 7.6, 8.2 Hz), 7.59 (1H, d, J = 2.2 Hz), 7.65 (1H, ddd, J = 1.1, 1.5, 7.6 Hz), 7.71 (1H, dd, J = 1.5, 2.2 Hz), 7.79 (1H, d, J = 8.9 Hz).

MS (ESI+) m/z: 441 (M+H)⁺, 443 (M+H+2)⁺, 463 (M+Na)⁺, 465 (M+Na+2)⁺.

HRMS (ESI+) m/Z: 441.10182 (M+H)⁺, calcd 441.10174 (0.08 mmu).

### (Example 12) 3-{[6-(3-Chloro-4-fluorophenoxy)-1-methyl-1H-benzimidazol-2-yl]methoxy}benzoic acid hydrochloride (hydrochloride of Compound No. 1-115)

A 1 N sodium hydroxide aqueous solution (10 mL, 10 mmol) was added to a solution of methyl 3-{[6-(3-chloro-4-fluorophenoxy)-1-methyl-1H-benzimidazol-2-yl]methoxy}benzoate hydrochloride synthesized in Example 11 (0.34 g, 0.7 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.23 g, yield: 70%).

¹H-NMR (DMSO-d₆, 400 MHz) δ: 3.94 (3H, s), 5.69 (2H, s), 7.07 (1H, ddd, J = 3.0, 3.9, 9.1 Hz), 7.22 (1H, dd, J = 2.2, 8.9 Hz), 7.29 (1H, dd, J = 3.0, 6.2 Hz), 7.44 (1H, ddd, J = 1.1, 2.7, 8.2 Hz), 7.46 (1H, dd, J = 9.1, 9.1 Hz), 7.50 (1H, dd, J = 7.6, 8.2 Hz), 7.63 (1H, d, J = 2.2 Hz), 7.64 (1H, ddd, J = 1.1, 1.3, 7.6 Hz), 7.70 (1H, dd, J = 1.3, 2.7 Hz), 7.82 (1H, d, J = 8.9 Hz).

MS (ESI+) m/z: 427 (M+H)⁺, 429 (M+H+2)⁺, 449 (M+Na)⁺, 451 (M+Na+2)⁺.

HRMS (ESI+) m/Z: 427.08535 (M+H)⁺, calcd 427.08609 (-0.74 mmu).

### (Example 13) Methyl 3-[(1-methyl-6-phenoxy-1H-benzimidazol-2-yl)methoxy]benzoate hydrochloride (hydrochloride of Compound No. 1-20)

### (13a) (6-Phenoxy-1-methyl-1H-benzimidazol-2-yl)methanol

tert-Butyl 2-nitro-4-chlorophenyl(methyl)carbamate (22.5 g, 78.6 mmol) and phenol (7.5 g, 78.6 mmol) were dissolved in tetrahydrofuran (180 mL) and DMF (20 mL). Sodium hydride (3.4 g, 78.6 mmol) was added and the mixture was stirred at 80°C for 10 hours. The reaction solution was poured into ice water, followed by extraction with ethyl acetate. The organic layer was washed with water and brine and dried over sodium sulfate. Then, the solvent was evaporated under reduced pressure. The residue was dissolved in 250 mL of ethanol, and 10% palladium carbon (8 g) was added. The mixture was stirred in a hydrogen atmosphere at 60°C for four hours. The catalyst was removed through celite, and the solvent was evaporated under reduced pressure. The residue was dissolved in 1,4-dioxane (80 mL) and 4 N hydrochloric acid-dioxane (80 mL). Glycolic acid (8.7 g, 115 mmol) was added and the mixture was heated under reflux for two hours. The reaction solution was cooled and then neutralized with saturated sodium bicarbonate. The generated crystals were filtered and washed with water and ethyl acetate to obtain 14 g of the desired compound (yield: 73%).

¹H-NMR (DMSO-d₆, 400 MHz) δ: 3.77 (3H, s), 4.70 (2H, s), 6. 85-6. 97 (3H, m), 7.05-7.11 (1H, m), 7.23-7.28 (3H, m), 7.58-7.61 (1H, m).

### (13b) Methyl 3-[(1-methyl-6-phenoxy-1H-benzimidazol-2-yl)methoxy]benzoate hydrochloride

Tri-n-butylphosphine (0.61 g, 3.0 mmol) and 1,1'-(azodicarbonyl)dipiperidine (0.76 g, 3.0 mmol) were added to a solution of (1-methyl-6-phenoxy-1H-benzimidazol-2-yl)methanol (0.38 g, 1.5 mmol) and methyl 3-hydroxybenzoate (0.34 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.42 g, yield: 66%) was obtained by drying under reduced pressure.

¹H-NMR (DMSO-d₆, 400 MHz) δ: 3.88 (3H, s), 3.93 (3H, s), 5.69 (2H, s), 7.02 (2H, d, J = 8.7 Hz), 7.12 (1H, dd, J = 2.2, 8.8 Hz), 7.15 (1H, t, J = 7.5 Hz), 7.41 (2H, dd, J = 7.5, 8.7 Hz), 7.48 (1H, ddd, J = 1.3, 2.6, 8.2 Hz), 7.54 (1H, dd, J = 7.5, 8.2 Hz), 7.59 (1H, d, J = 2.2 Hz), 7.66 (1H, ddd, J = 1.3, 1.5, 7.5 Hz), 7.72 (1H, dd, J = 1.5, 2.6 Hz), 7.80 (1H, d, J = 8.8 Hz).
MS (ESI+) m/z: 389 (M+H)⁺, 441 (M+Na)⁺.

HRMS (ESI+) m/Z: 389.15176 (M+H)⁺, calcd 389.15013 (1.63 mmu).

### (Example 14) 3-[(1-Methyl-6-phenoxy-1H-benzimidazol-2-yl)methoxy]benzoic acid hydrochloride (hydrochloride of Compound No. 1-19)

A 1 N sodium hydroxide aqueous solution (10 mL, 10 mmol) was added to a solution of methyl 3-[(1-methyl-6-phenoxy-1H-benzimidazol-2-yl)methoxy]benzoate hydrochloride synthesized in Example 13 (0.28 g, 0.7 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.19 g, yield: 70%).

¹H-NMR (DMSO-d₆, 400 MHz) δ: 3.95 (3H, s), 5.72 (2H, s), 7.04 (2H, d, J = 8.8 Hz), 7.16 (1H, t, J = 7.5 Hz), 7.21 (1H, dd, J = 2.4, 8.8 Hz), 7.41 (2H, dd, J = 7.5, 8.8 Hz), 7.46 (1H, ddd, J = 1.1, 2.6, 8.2 Hz), 7.50 (1H, dd, J = 7.5, 8.2 Hz), 7.62 (1H, d, J = 2.2 Hz), 7.64 (1H, ddd, J = 1.1, 1.5, 7.5 Hz), 7.71 (1H, dd, J = 1.5, 2.6 Hz), 7.82 (1H, d, J = 8.8 Hz).

MS (ESI+) m/z: 375 (M+H)⁺, 397 (M+Na)⁺, 419 (M+2Na-H)⁺.

HRMS (ESI+) m/Z: 375.13441 (M+H)⁺, calcd 375.13448 (-0.07 mmu).

### (Example 15) Methyl 3-[(3-methyl-5-phenoxy-3H-imidazo[4,5-b]pyridin-2-yl)methoxy]benzoate dihydrochloride (dihydrochloride of Compound No. 1-28)

### (15a) (3-Methyl-5-phenoxy-3H-imidazo[4,5-b]pyridin-2-yl)methanol

Phenol (12.08 g, 128 mmol) was dissolved in THF (200 mL), and sodium hydride (60%, 5.12 g, 128 mmol) was added. Subsequently, 6-chloro-N-methyl-3-nitropyridin-2-amine (20 g, 107 mmol) was added and the mixture was stirred at 80°C for four hours. The reaction solution was poured into water, followed by extraction with ethyl acetate. The organic layer was sequentially washed with water, a 1 N potassium hydroxide solution, water and brine, dried and then concentrated. The residue was dissolved in THF (50 mL)-ethanol (50 mL), and palladium hydroxide (500 mg) was added. The mixture was stirred in a hydrogen atmosphere overnight. The catalyst was removed through celite, and the solvent was evaporated under reduced pressure. The residue was dissolved in 1,4-dioxane (150 mL) and 4 N hydrochloric acid-dioxane (150 mL). Glycolic acid (24.4 g, 321 mmol) was added and the mixture was heated under reflux for 10 hours. The reaction solution was cooled and then neutralized with a saturated sodium bicarbonate aqueous solution, followed by extraction with ethyl acetate. The organic layer was sequentially washed with water and brine and dried. Then, the solvent was evaporated under reduced pressure. The generated crystals were thoroughly washed with diisopropyl ether to obtain 21.8 g of the desired compound (yield: 80%).

¹H-NMR (DMSO-d₆, 400 MHz) δ: 3.67 (3H, s, J = 7 Hz), 4.69 (2H, d, J = 5 Hz), 5.61 (1H, t, J = 5 Hz), 6.84 (1H, d, J = 8 Hz), 7.12-7.23 (3H, m), 7.39-7.45 (2H, m), 8.06 (1H, d, J = 8 Hz).

### (15b) Methyl 3-[(3-methyl-5-phenoxy-3H-imidazo[4,5-b]pyridin-2-yl)methoxy]benzoate dihydrochloride

Tri-n-butylphosphine (0.61 g, 3.0 mmol) and 1,1'-(azodicarbonyl)dipiperidine (0.76 g, 3.0 mmol) were added to a solution of (3-methyl-5-phenoxy-3H-imidazo[4,5-b]pyridin-2-yl)methanol (0.38 g, 1.5 mmol) and methyl 3-hydroxybenzoate (0.34 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.40 g, yield: 58%) was obtained by drying under reduced pressure.

¹H-NMR (DMSO-d₆, 400 MHz) δ: 3.72 (3H, s), 3.86 (3H, s), 5.54 (2H, s), 6.96 (1H, d, J = 8.6 Hz), 7.18 (1H, d, J = 7.5 Hz), 7.22 (1H, t, J = 7.3 Hz), 7.43 (2H, m), 7.45 (1H, dd, J = 7.6, 8.2 Hz), 7.61 (1H, dd, J = 1.5, 7.6 Hz), 7.66 (1H, dd, J = 1.5, 2.3 Hz), 8.18 (1H, d, J = 8.6 Hz).

MS (ESI+) m/z: 390 (M+H)⁺, 412 (M+Na)⁺.

HRMS (ESI+) m/Z: 390.14693 (M+H)⁺, calcd 390.14538 (1.55 mmu).

### (Example 16) 3-[(3-Methyl-5-phenoxy-3H-imidazo[4,5-b]pyridin-2-yl)methoxy]benzoic acid dihydrochloride (dihydrochloride of Compound No. 1-27)

A 1 N sodium hydroxide aqueous solution (10 mL, 10 mmol) was added to a solution of methyl 3-[(3-methyl-5-phenoxy-3H-imidazo[4,5-b]pyridin-2-yl)methoxy]benzoate dihydrochloride synthesized in Example 15 (0.25 g, 0.6 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.16 g, yield: 65%).

¹H-NMR (DMSO-d₆, 400 MHz) δ: 3.73 (3H, s), 5.53 (2H, s), 6.96 (1H, d, J = 8.6 Hz), 7.18 (1H, d, J = 8.7 Hz), 7.22 (1H, t, J = 7.5 Hz), 7.38 (1H, ddd, J = 1.1, 2.6, 8.2 Hz), 7.42 (2H, m), 7.46 (1H, dd, J = 7.6, 8.2 Hz), 7.59 (1H, ddd, J = 1.1, 1.3, 7.6 Hz), 7.64 (1H, dd, J = 1.3, 2.6 Hz), 8.18 (1H, d, J = 8.6 Hz).

MS (ESI+) m/z: 376 (M+H)⁺, 398 (M+Na)⁺, 420 (M+2Na-H)⁺.

HRMS (ESI+) m/Z: 376.12947 (M+H)⁺, calcd 376.12973 (-0.26 mmu).

### (Example 19) Methyl 3-{[1-methyl-6-(3-morpholin-4-ylphenoxy)-1H-benzimidazol-2-yl]methoxy}benzoate dihydrochloride (dihydrochloride of Compound No. 1-68)

### (19a) tert-Butyl methyl-[5-(3-morpholin-4-yl-phenoxy)-2-nitro-phenyl]-carbamate

The desired crude compound was obtained from tert-butyl (5-chloro-2-nitro-phenyl)-methyl-carbamate (5.0 g, 17 mmol), sodium hydride (761 mg, 17 mmol) and 3-morpholin-4-yl-phenol (3.2 g, 17 mmol) in the same manner as in Example 7a. The compound was purified by silica gel column chromatography to obtain the desired compound (6.5 g, yield: 86%) as a yellow foam.

¹H-NMR (CDCl₃, 400 MHz) δ: 1.32 (9H, s), 3.15-3.22 (4H, m), 3.25 (3H, s), 3.81-3.88 (4H, m), 6.57 (1H, t, J = 7.3 Hz), 6.62 (1H, s), 6.79 (1H, d, J = 7.8 Hz), 6.82-6.91 (2H, m), 7.24-7.33 (1H, m), 7.92 (1H, d, J = 8.8 Hz).

### (19b) tert-Butyl [2-amino-5-(3-morpholin-4-yl-phenoxy)-phenyl]-methyl-carbamate

tert-Butyl methyl-[5-(3-morpholin-4-yl-phenoxy)-2-nitro-phenyl]-carbamate synthesized in Example 19a (6.5 g, 15 mmol) and palladium-carbon (10%, 1.0 g) were suspended in ethyl acetate (100 mL), and the suspension was stirred in a hydrogen atmosphere for two hours. The catalyst was removed by filtration, and the filtrate was concentrated under reduced pressure to obtain the desired compound (5.8 g, yield: 96%) as a pale orange solid.

¹H-NMR (CDCl₃, 400 MHz) δ: 1.38 (9H, s), 3.09-3.16 (7H, m), 3.80-3.84 (4H, m), 6.38 (1H, br s), 6.51 (1H, s), 6.56 (1H, d, J = 7.4 Hz), 6.70-6.80 (3H, m), 7.13 (1H, t, J = 8.0 Hz).

### (19c) [1-Methyl-6-(3-morpholin-4-yl-phenoxy)-1H-benzimidazol-2-yl]-methanol

tert-Butyl [2-amino-5-(3-morpholin-4-yl-phenoxy)-phenyl]-methyl-carbamate synthesized in Example 19b (5.8 g, 15 mmol) and glycolic acid (1.66 g, 22 mmol) were dissolved in a mixed solvent of 4 N hydrochloric acid (60 mL) and 1,4-dioxane (60 mL), followed by heating under reflux. The organic solvent was evaporated from the reaction solution under reduced pressure. The remaining aqueous solution was washed with methylene chloride, neutralized with an excess of sodium bicarbonate, diluted with ethyl acetate and stirred. The generated solid was filtered to obtain the desired compound (2.8 g, yield: 57%) as a pale brown solid.

¹H-NMR (DMSO-d₆, 400 MHz) δ: 3.03-3.08 (4H, m), 3.67-3.71 (4H, m), 3.76 (3H, s), 4.66-4.69 (2H, m), 6.30 (1H, dd, J = 2.0, 7.8 Hz), 6.55 (1H, t, J = 2.35), 6.65 (1H, dd, J = 2.2, 8.0 Hz), 6.86 (1H, dd, J = 2.4, 8.6 Hz), 7.14 (1H, t, J = 8.2 Hz), 7.22 (1H, d, J = 2.4 Hz), 7.55 (1H, d, J = 8.6 Hz).

### (19d) Methyl 3-{[1-methyl-6-(3-morpholin-4-ylphenoxy)-1H-benzimidazol-2-yl]methoxy}benzoate dihydrochloride

Tri-n-butylphosphine (0.61 g, 3.0 mmol) and 1,1'-(azodicarbonyl)dipiperidine (0.76 g, 3.0 mmol) were added to a solution of [1-methyl-6-(3-morpholin-4-ylphenoxy)-1H-benzimidazol-2-yl]methanol (0.51 g, 1.5 mmol) and methyl 3-hydroxybenzoate (0.34 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.49 g, yield: 60%) was obtained by drying under reduced pressure.

¹H-NMR (DMSO-d₆, 400 MHz) δ: 3.13 (4H, t, J = 4.7 Hz), 3.72 (4H, t, J = 4.7 Hz), 3.87 (3H, s), 3.97 (3H, s), 5.76 (2H, s), 6.42 (1H, dd, J = 2.0, 7.8 Hz), 6.70 (1H, dd, J = 2.0, 2.0 Hz), 6.80 (1H, dd, J = 2.0, 8.2 Hz), 7.23 (1H, dd, J = 7.8, 8.2 Hz), 7.23 (1H, dd, J = 2.3, 8.6 Hz), 7.50 (1H, ddd, J = 1.2, 2.4, 8.3 Hz), 7.54 (1H, dd, J = 7.1, 8.3 Hz), 7.64 (1H, d, J = 2.3 Hz), 7.65 (1H, ddd, J = 1.2, 1.6, 7.1 Hz), 7.72 (1H, dd, J = 1.6, 2.4 Hz), 7.82 (1H, d, J = 8.6 Hz).

MS (ESI+) m/z: 474 (M+H)⁺, 496 (M+Na)⁺.

HRMS (ESI+) m/Z: 474.20084 (M+H)⁺, calcd 474.20290 (-2.06 mmu).

### (Example 20) 3-{[1-Methyl-6-(3-morpholin-4-ylphenoxy)-1H-benzimidazol-2-yl]methoxy}benzoic acid 1/2 hydrochloride (1/2 hydrochloride of Compound No. 1-67)

A 1 N sodium hydroxide aqueous solution (10 mL, 10 mmol) was added to a solution of methyl 3-{[1-methyl-6-(3-morpholin-4-ylphenoxy)-1H-benzimidazol-2-yl]methoxy}benzoate dihydrochloride (0.34 g, 0.7 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.18 g, yield: 59%).

¹H-NMR (DMSO-d₆, 400 MHz) δ: 3.69 (4H, t, J = 4.7 Hz), 3.80 (4H, t, J = 4.7 Hz), 5.45 (2H, s), 6.32 (1H, dd, J = 2.0, 8.2 Hz), 6.57 (1H, dd, J = 2.0, 2.4 Hz), 6.66 (1H, dd, J = 2.4, 8.2 Hz), 6.90 (1H, dd, J = 2.4, 8.6 Hz), 7.15 (1H, dd, J = 8.2, 8.2 Hz), 7.28 (1H, d, J = 2.4 Hz), 7.36 (1H, ddd, J = 8.2 Hz), 7.43 (1H, dd, J = 7.4, 8.2 Hz), 7.55 (1H, ddd, J = 7.4 Hz), 7.61 (1H, br), 7.62 (1H, d, J = 8.6 Hz).

MS (ESI+) m/z: 460 (M+H)⁺, 482 (M+Na)⁺.

HRMS (ESI+) m/Z: 460.18678 (M+H)⁺, calcd 460.18725 (-0.47 mmu).

### (Example 21) Ethyl 4-{[1-methyl-6-(3-morpholin-4-ylphenoxy)-1H-benzimidazol-2-yl]methoxy}benzoate dihydrochloride (dihydrochloride of Compound No. 1-70)

Tri-n-butylphosphine (0.61 g, 3.0 mmol) and 1,1'-(azodicarbonyl)dipiperidine (0.76 g, 3.0 mmol) were added to a solution of [1-methyl-6-(3-morpholin-4-ylphenoxy)-1H-benzimidazol-2-yl]methanol (0.51 g, 1.5 mmol) and ethyl 4-hydroxybenzoate (0.37 g, 2.3 mmol) in toluene, followed by stirring for 10 hours. The reaction solution was concentrated and then purified by silica gel column chromatography (elution solvent: hexane/ethyl acetate = 1/2). Then, a 4 N hydrogen chloride/1,4-dioxane solution (10 mL) was added, followed by stirring for two hours. The precipitated solid was collected by filtration and washed with ethyl acetate and ether. The desired title compound (0.55 g, yield: 65%) was obtained by drying under reduced pressure.

¹H-NMR (DMSO-d₆, 400 MHz) δ: 1.31 (3H, t, J = 7.0 Hz), 3.11 (4H, t, J = 4.7 Hz), 3.71 (4H, t, J = 4.7 Hz), 3.94 (3H, s), 4.28 (2H, q, J = 7.04 Hz), 5.73 (2H, s), 6.40 (1H, dd, J = 2.0, 7.8 Hz), 6.66 (1H, dd, J = 2.0, 2.0 Hz), 6.76 (1H, dd, J = 2.0, 8.2 Hz), 7.18 (1H, dd, J = 2.4, 9.0 Hz), 7.22 (1H, dd, J = 7.8, 8.2 Hz) 7.29 (2H, d, J = 9.0 Hz), 7.59 (1H, d, J = 2.4), 7.78 (1H, d, J = 9.0 Hz), 7.97 (2H, d, J = 9.0 Hz).

MS (ESI+) m/z: 488 (M+H)⁺, 510 (M+Na)⁺.

HRMS (ESI+) m/Z: 488.21667 (M+H)⁺, calcd 488.21855 (-1.87 mmu).

### (Example 22) 4-{[1-Methyl-6-(3-morpholin-4-ylphenoxy)-1H-benzimidazol-2-yl]methoxy}benzoic acid dihydrochloride (dihydrochloride of Compound No. 1-69)

A 1 N sodium hydroxide aqueous solution (10 mL, 10 mmol) was added to a solution of ethyl 4-{[1-methyl-6-(3-morpholin-4-ylphenoxy)-1H-benzimidazol-2-yl]methoxy}benzoate dihydrochloride (0.43 g, 0.8 mmol) in 1,4-dioxane, and the mixture was stirred at 60°C for two hours. The reaction solution was treated with concentrated hydrochloric acid (1.5 mL) and then concentrated. The resulting solid was washed with water and ethyl acetate and dried under reduced pressure to obtain the desired title compound (0.24 g, yield: 59%).

¹H-NMR (DMSO-d₆, 400 MHz) δ: 3.11 (4H, t, J = 4.7 Hz), 3.71 (4H, t, J = 4.7 Hz), 3.94 (3H, s), 5.72 (2H, s), 6.39 (1H, dd, J = 2.3, 7.8 Hz), 6.65 (1H, dd, J = 2.0, 2.3 Hz), 6.76 (1H, dd, J = 2.0, 8.2 Hz), 7.18 (1H, dd, J = 2.4, 9.0 Hz), 7.22 (1H, dd, J = 7.8, 8.2 Hz), 7.26 (2H, d, J = 9.0 Hz), 7.58 (1H, d, J = 2.3 Hz), 7.78 (1H, d, J = 9.0 Hz), 7.94 (2H, d, J = 9.0 Hz).

MS (ESI+) m/z: 460 (M+H)⁺, 482 (M+Na)⁺.

HRMS (ESI+) m/Z: 460.18703 (M+H)⁺, calcd 460.18725 (-0.22 mmu).

### (Example 25) Methyl 3-{[1-methyl-6-(pyridin-3-yloxy)-1H-benzimidazol-2-yl]methoxy}benzoate dihydrochloride (dihydrochloride of Compound No. 1-148)

### (25a) [1-Methyl-6-(pyridin-3-yloxy)-1H-benzimidazol-2-yl]methanol

Glycolic acid (2.75 g, 36.2 mmol) was added to a mixed solution of tert-butyl 2-amino-5-(pyridin-3-yloxy)phenyl(methyl)carbamate (7.61 g, 24.1 mmol) [US6432993 B1] in 1,4-dioxane (75 mL) and a 4 N hydrochloric acid solution (75 mL). The mixture was stirred at 50°C for 30 minutes and heated under reflux for seven hours. The reaction solution was neutralized with a saturated sodium bicarbonate aqueous solution, followed by extraction with ethyl acetate. The organic layer was washed with brine and then dried over anhydrous sodium sulfate. The solvent was evaporated under reduced pressure, and the resulting residue was purified by silica gel column chromatography (elution solvent: ethyl acetate/methanol = 5/1) to obtain the desired title compound (4.31 g, 16.9 mmol).

1H-NMR (CDCl₃, 400 MHz) δ: 3.78 (3H, s), 4.90 (2H, s), 6.96-7.01 (2H, m), 7.25-7.28 (2H, m), 7.65-7.68 (1H, m), 8.34-8.42 (2H, m).

### (25b) Methyl 3-{[1-methyl-6-(pyridin-3-yloxy)-1H-benzimidazol-2-yl]methoxy}benzoate dihydrochloride

[1-Methyl-6-(pyridin-3-yloxy)-1H-benzimidazol-2-yl]methanol synthesized in Example 25a (1.0 g, 3.92 mmol) was dissolved in toluene (17 mL). Methyl 3-hydroxybenzoate (895 mg, 5.88 mmol), 1,1'-(azodicarbonyl)dipiperidine (2.97 g, 9.65 mmol) and n-tributylphosphine (2.90 mL, 11.8 mmol) were added and the mixture was stirred at room temperature overnight. The solvent was evaporated under reduced pressure, and the resulting residue was purified by silica gel column chromatography (elution solvent: ethyl acetate/methanol = 5/1) to obtain methyl 3-{[1-methyl-6-(pyridin-3-yloxy)-1H-benzimidazol-2-yl]methoxy}benzoate (1.5 g, 3.85 mmol). Methyl 3-{[1-methyl-6-(pyridin-3-yloxy)-1H-benzimidazol-2-yl]methoxy}benzoate (681 mg, 1.75 mmol) was dissolved in ethyl acetate (15 mL). 4 N hydrochloric acid (solution in 1,4-dioxane, 5 mL) was added and the mixture was stirred at room temperature for 10 minutes. The solid was collected by filtration and recrystallized from ethanol to obtain the desired title compound (627 mg, 1.36 mmol).

Mp 195-208°C.

IR (KBr) νmax 1238, 1272, 1489, 1546, 1707.

1H-NMR (DMSO-d₆, 400 MHz) δ: 3.88 (3H, s), 3.97 (3H, s), 5.75 (2H, s), 7.36 (1H, dd, J = 8.8, 2.2 Hz), 7.50-7.56 (2H, m), 7.65-7.66 (1H, m), 7.73-7.82 (3H, m), 7.86-7.91 (2H, m), 8.57 (1H, dd, J = 5.1, 1.3 Hz), 8.63 (1H, d, J = 2.4 Hz).

MS (FAB) m/z: 390 (M+H)⁺.

Anal. calcd for C₂₂H₁₉N₃O₄+2HCl: C, 57.15; H, 4.58; Cl, 15.34; N, 9.09. Found C, 53.52; H, 4.99; Cl, 14.73; N, 8.48.

### (Example 26) 3-{[1-Methyl-6-(pyridin-3-yloxy)-1H-benzimidazol-2-yl]methoxy}benzoic acid (Compound No. 1-147)

A 1 N sodium hydroxide aqueous solution (2.26 mL, 2.26 mmol) was added to a solution of the intermediate methyl 3-{[1-methyl-6-(pyridin-3-yloxy)-1H-benzimidazol-2-yl]methoxy}benzoate synthesized in Example 25b (800 mg, 2.05 mmol) in 1,4-dioxane (5 mL), and the mixture was stirred at 70°C for one hour. The reaction solution was adjusted to pH 7 with a 1 N hydrochloric acid solution. The precipitated solid was collected by filtration and washed with ethyl acetate to obtain the desired title compound (332 mg, 0.885 mmol).

Mp 240-245°C.

IR (KBr) νmax 1219, 1290, 1421, 1478, 1586, 1697.

1H-NMR (DMSO-d₆, 400 MHz) δ: 3.83 (3H, s), 5.48 (2H, s), 7.00 (1H, dd, J = 8.6, 2.4 Hz), 7.34-7.47 (5H, m), 7.57-7.59 (1H, m), 7.63-7.70 (2H, m), 8.32 (1H, dd, J = 4.0, 1.5 Hz), 8.38 (1H, dd, J = 2.8, 0.9 Hz).

MS (FAB) m/z: 376 (M+H)⁺.

Anal. calcd for C₂₁H₁₇N₃O₄: C, 67.19; H, 4.56; N, 11.19. Found C, 67.14; H, 4.71; N, 11.04.

### (Example 27) 3-{[6-(4-Fluorophenoxy)-1-methyl-1H-benzimidazol-2-yl]methoxy}benzoic acid (Compound No. 1-165)

### (27a) tert-Butyl [5-(4-fluorophenoxy)-2-nitrophenyl]methylcarbamate

The title substance (3.2 g, yield: 88%) was obtained as a yellow solid by synthesis from 4-fluorophenol (1.1 g, 10 mmol), tert-butyl (5-chloro-2-nitrophenyl)methylcarbamate (2.9 g, 10 mmol), sodium hydride (> 56% in oil, 0.38 g, 10 mmol) and N,N-dimethylformamide (40 mL) in the same manner as in Example (28a) and crystallization from hexane.

¹H-NMR (CDCl₃, 400 MHz) δ: 1.33 (6H, s), 1.50 (3H, s), 3.26 (3H, s), 6.81 (1H, dd, J = 2.7, 9.0 Hz), 6.85 (1H, br s), 7.07-7.17 (4H, m), 7.93-7.97 (1H, m).

### (27b) tert-Butyl [2-amino-5-(4-fluorophenoxy)phenyl]methylcarbamate

The synthesis was carried out in the same manner as in Example (28b) using tert-butyl [5-(4-fluorophenoxy)-2-nitrophenyl]methylcarbamate produced in Example (27a) (3.2 g, 8.8 mmol), iron powder (2.4 g, 12 mmol), ammonium chloride (0.24 g, 1.2 mmol), ethanol (40 mL) and water (20 mL). The resulting oil was directly used for the next reaction.

### (27c) [6-(4-Fluorophenoxy)-1-methyl-1H-benzimidazol-2-yl]methanol

The synthesis was carried out in the same manner as in Example (28c) using tert-butyl [2-amino-5-(4-fluorophenoxy)phenyl]methylcarbamate produced in Example (27b) (2.9 g, 8.8 mmol), glycolic acid (1.0 g, 13 mmol) and a 4 N hydrochloric acid-1,4-dioxane solution (40 mL). The resulting dark brown oil was directly used for the next reaction.

### (27d) Methyl 3-{[6-(4-fluorophenoxy)-1-methyl-1H-benzimidazol-2-yl]methoxy}benzoate

The reaction and post-treatment were carried out according to Example (28d) using [6-(4-fluorophenoxy)-1-methyl-1H-benzimidazol-2-yl]methanol produced in Example (27c) (0.30 g, 1.1 mmol), methyl 3-hydroxybenzoate (0.25 g, 1.7 mmol), tri-n-butylphosphine (0.55 mL, 2.2 mmol), 1,1'-(azodicarbonyl)dipiperidine (0.56 g, 2.2 mmol) and dichloromethane (6.0 mL) to obtain the desired compound (0.36 g, yield: 81%).

¹H-NMR (CDCl₃, 500 MHz) δ: 3.82 (3H, s), 3.92 (3H, s), 5.39 (2H, s), 6.94-7.05 (5H, m), 7.29 (1H, br s), 7.38 (1H, t, J = 7.8 Hz), 7.69 (1H, d, J = 7.8 Hz), 7.71-7.74 (2H, m).

MS (FAB) m/z: 407 (M+H)⁺.

### (27e) 3-{[6-(4-Fluorophenoxy)-1-methyl-1H-benzimidazol-2-yl]methoxy}benzoic acid

The reaction and post-treatment were carried out according to Example (28e) using methyl 3-{[6-(4-fluorophenoxy)-1-methyl-1H-benzimidazol-2-yl]methoxy}benzoate produced in Example (27d) (0.34 g, 0.84 mmol), a 1 N sodium hydroxide aqueous solution (1.3 mL, 1.3 mmol) and 1,4-dioxane to obtain the desired compound (0.10 g, yield: 37%) as a white solid.

¹H-NMR (DMSO-d₆, 500 MHz) δ: 3.81 (3H, s), 5.46 (2H, s), 6.93 (1H, dd, J = 2.4, 8.8 Hz), 7.01-7.04 (2H, m), 7.19 (2H, t, J = 8.8 Hz), 7.30 (1H, d, J = 2.4 Hz), 7.37-7.39 (1H, m), 7.45 (1H, t, J = 7.8 Hz), 7.57 (2H, d, J = 7.8 Hz), 7.66 (1H, d, J = 8.8 Hz), 7.63 (1H, s), 13.03 (1H, br. s).

MS (FAB) m/z: 393 (M+H)⁺.

Anal. calcd for C₂₂H₁₇FN₂O₄+0.14H₂O: C, 66.91; H, 4.41; N, 7.09; F, 4.81. Found C, 66.85; H, 4.46; N, 7.21; F, 4.81.

### (Example 28) 3-{[6-(2-Fluorophenoxy)-1-methyl-1H-benzimidazol-2-yl]methoxy}benzoic acid (Compound No. 1-164)

### (28a) tert-Butyl [5-(2-fluorophenoxy)-2-nitrophenyl]methylcarbamate

2-Fluorophenol (0.16 mL, 1.7 mmol) was dissolved in N,N-dimethylformamide (10 mL) in a nitrogen atmosphere. tert-Butyl (5-chloro-2-nitrophenyl)methylcarbamate (500 mg, 1.7 mmol) and sodium hydride (> 56% in oil, 0.84 g, 1.9 mmol) were added and the mixture was stirred at 80°C for seven hours. The reaction solution was concentrated and a sodium bicarbonate aqueous solution was added, followed by extraction with diethyl ether twice. Then, the organic layers were washed with water and brine and dried over anhydrous magnesium sulfate. The solvent was evaporated under reduced pressure, and the resulting yellow oil was directly used for the next reaction.

### (28b) tert-Butyl [2-amino-5-(2-fluorophenoxy)phenyl]methylcarbamate

Iron powder (0.47 g, 8.8 mmol) and ammonium chloride (0.047 g, 0.88 mmol) were added to a mixture of tert-butyl [5-(2-fluorophenoxy)-2-nitrophenyl]methylcarbamate produced in Example (28a) (0.74 g, 1.7 mmol), ethanol (8.0 mL) and water (4.0 mL), and the resulting mixture was heated under reflux for two hours. The insoluble matter was filtered off through celite. Water was added to the concentrated filtrate, followed by extraction with ethyl acetate twice. Then, the organic layers were washed with brine and dried over anhydrous magnesium sulfate. The solvent was evaporated under reduced pressure, and the resulting oil was directly used for the next reaction.

### (28c) [6-(2-Fluorophenoxy)-1-methyl-1H-benzimidazol-2-yl]methanol

tert-Butyl [2-amino-5-(2-fluorophenoxy)phenyl]methylcarbamate produced in Example (28b) (0.58 g, 1.749 mmol) was dissolved in a 4 N hydrochloric acid-1,4-dioxane solution (10 mL). Glycolic acid (0.20 g, 2.6 mmol) was added and the mixture was heated under reflux for 1.5 days. The reaction solution was concentrated and a sodium bicarbonate aqueous solution was added, followed by extraction with ethyl acetate twice. Then, the organic layers were washed with water and brine and dried over anhydrous magnesium sulfate. The solvent was evaporated under reduced pressure, and the resulting residue was subjected to simple purification by silica gel column chromatography (elution solvent: hexane/ethyl acetate = 5/1 -> only ethyl acetate -> methanol/dichloromethane = 1/5). The solvent was evaporated under reduced pressure, and the resulting dark brown oil was directly used for the next reaction.

### (28d) Methyl 3-{[6-(2-fluorophenoxy)-1-methyl-1H-benzimidazol-2-yl]methoxy}benzoate

Tri-n-butylphosphine (0.36 mL, 1.4 mmol) and 1,1'-(azodicarbonyl)dipiperidine (0.36 g, 1.4 mmol) were added to a solution of [6-(2-fluorophenoxy)-1-methyl-1H-benzimidazol-2-yl]methanol produced in Example (28c) (0.20 g, 0.7 mmol) and methyl 3-hydroxybenzoate (0.16 g, 1.1 mmol) in dichloromethane, followed by stirring for 12 hours. The reaction solution was concentrated and then suspended in a mixed solvent of hexane/ethyl acetate (= 3/2). After ultrasonic treatment, the precipitated solid was separated by filtration. The filtrate was concentrated and then purified by silica gel column chromatography (elution solvent: hexane/ethyl acetate = 5/1 -> 1/1). The desired compound (0.16 g, yield: 58%) was obtained by drying under reduced pressure.

¹H-NMR (CDCl₃, 400 MHz) δ: 3.81 (3H, s), 3.92 (3H, s), 5.39 (2H, s), 6.96 (1H, s), 7.00-7.05 (2H, m), 7.07-7.13 (2H, m), 7.20 (1H, t, J = 9.5 Hz), 7.29 (1H, d, J = 7.3 Hz), 7.37 (1H, t, J = 7.3 Hz), 7.69 (1H, d, J = 7.8 Hz), 7.70-7.74 (2H, m).

MS (FAB) m/z: 407 (M+H)⁺.

### (28e) 3-{[6-(2-Fluorophenoxy)-1-methyl-1H-benzimidazol-2-yl]methoxy}benzoic acid

A 1 N sodium hydroxide aqueous solution (0.54 mL, 0.54 mmol) was added to a solution of methyl 3-{[6-(2-fluorophenoxy)-1-methyl-1H-benzimidazol-2-yl]methoxy}benzoate produced in Example (28d) (0.16 g, 0.36 mmol) in 1,4-dioxane, and the mixture was stirred at 70°C for 1.5 hours. The reaction solution was concentrated and water was added. The mixture was neutralized by dropwise addition of a 1 N hydrochloric acid solution. The precipitated solid was collected by filtration to obtain the desired compound (0.096 g, yield: 68%) as a white solid.

¹H-NMR (DMSO-d₆, 400 MHz) δ: 3.81 (3H, s), 5.47 (2H, s), 6.94 (1H, dd, J = 2.5, 8.8 Hz), 7.04-7.09 (1H, m), 7.18 (2H, ddd, J = 3.1, 3.3, 6.1 Hz), 7.30 (1H, d, J = 2.4 Hz), 7.35-7.42 (2H, m), 7.45 (1H, t, J = 7.8 Hz), 7.58 (1H, d, J = 7.4 Hz), 7.66 (1H, d, J = 8.6 Hz), 7.63 (1H, s), 13.03 (1H, br. s).

MS (FAB) m/z: 393 (M+H)⁺.

Anal. calcd for C₂₂H₁₇FN₂O₄+0.14H₂O: C, 66.91; H, 4.41; N, 7.09; F, 4.81. Found C, 66.86; H, 4.48; N, 7.08; F, 4.80.

### (Example 29) 3-{[6-(3-Methoxyphenoxy)-1-methyl-1H-benzimidazol-2-yl]methoxy}benzoic acid (Compound No. 1-173)

### (29a) tert-Butyl [5-(3-methoxyphenoxy)-2-nitrophenyl]methylcarbamate

The synthesis was carried out in the same manner as in Example (28a) using 3-methoxyphenol (0.19 mL, 1.7 mmol), tert-butyl (5-chloro-2-nitrophenyl)methylcarbamate (0.50 g, 1.7 mmol), sodium hydride (> 56% in oil, 0.84 g, 1.9 mmol) and N,N-dimethylformamide (10 mL). The resulting yellow oil was directly used for the next reaction.

### (29b) tert-Butyl [2-amino-5-(3-methoxyphenoxy)phenyl]methylcarbamate

The synthesis was carried out in the same manner as in Example (28b) using tert-butyl [5-(3-methoxyphenoxy)-2-nitrophenyl]methylcarbamate produced in Example (29a) (0.65 g, 1.7 mmol), iron powder (0.47 g, 8.7 mmol), ammonium chloride (0.047 g, 0.87 mmol), ethanol (8.0 mL) and water (4.0 mL). The resulting oil was directly used for the next reaction.

### (29c) [6-(3-Methoxyphenoxy)-1-methyl-1H-benzimidazol-2-yl]methanol

The synthesis was carried out in the same manner as in Example (28c) using tert-butyl [2-amino-5-(3-methoxyphenoxy)phenyl]methylcarbamate produced in Example (29b) (0.60 g, 1.7 mmol), glycolic acid (0.20 g, 2.6 mmol) and a 4 N hydrochloric acid-1,4-dioxane solution (10 mL). The resulting dark brown oil was directly used for the next reaction.

### (29d) Methyl 3-{[6-(3-methoxyphenoxy)-1-methyl-1H-benzimidazol-2-yl]methoxy}benzoate

The reaction and post-treatment were carried out according to Example (28d) using [6-(3-methoxyphenoxy)-1-methyl-1H-benzimidazol-2-yl]methanol produced in Example (29c) (0.24 g, 0.85 mmol), methyl 3-hydroxybenzoate (0.20 g, 1.3 mmol), tri-n-butylphosphine (0.43 mL, 1.7 mmol), 1,1'-(azodicarbonyl)dipiperidine (0.43 g, 1.7 mmol) and dichloromethane (6.0 mL) to obtain the desired compound (0.23 g, yield: 65%).

¹H-NMR (CDCl₃, 400 MHz) δ: 3.77 (3H, s), 3.89 (3H, s), 5.57 (2H, s), 6.55-6.58 (2H, m), 6.61-6.69 (1H, m), 7.00-7.07 (2H, m), 7.19-7.25 (2H, m), 7.38 (1H, t, J = 7.8 Hz), 7.84 (1H, d, J = 8.6 Hz), 7.78 (1H, d, J = 7.4 Hz), 8.03 (1H, s).

### (29e) 3-{[6-(3-Methoxyphenoxy)-1-methyl-1H-benzimidazol-2-yl]methoxy}benzoic acid

The reaction and post-treatment were carried out according to Example (28e) using methyl 3-{[6-(3-methoxyphenoxy)-1-methyl-1H-benzimidazol-2-yl]methoxy}benzoate produced in Example (29d) (0.23 g, 0.56 mmol), a 1 N sodium hydroxide aqueous solution (0.83 mL, 0.83 mmol) and 1,4-dioxane to obtain the desired compound (0.20 g, yield: 89%) as a white solid.

¹H-NMR (CDCl₃, 400 MHz) δ: 3.77 (3H, s), 3.89 (3H, s), 5.57 (2H, s), 6.55-6.58 (2H, m), 6.61-6.69 (1H, m), 7.00-7.07 (2H, m), 7.19-7.25 (2H, m), 7.38 (1H, t, J = 7.8 Hz), 7.84 (1H, d, J = 8.6 Hz), 7.78 (1H, d, J = 7.4 Hz), 8.03 (1H, s).

MS (FAB) m/z: 405 (M+H)⁺,

Anal. calcd for C₂₃H₂₀N₂O₅+0.33H₂O: C, 67.31; H, 5.08; N, 6.83. Found C, 67.47; H, 4.94; N, 6.92.

### (Example 30) 3-{[6-(4-Methoxyphenoxy)-1-methyl-1H-benzimidazol-2-yl]methoxy}benzoic acid (Compound No. 1-174)

### (30a) tert-Butyl [5-(4-methoxyphenoxy)-2-nitrophenyl]methylcarbamate

The synthesis was carried out in the same manner as in Example (28a) using 4-methoxyphenol (0.20 g, 1.7 mmol), tert-butyl (5-chloro-2-nitrophenyl)methylcarbamate (0.50 g, 1.7 mmol), sodium hydride (> 56% in oil, 0.84 g, 1.9 mmol) and N,N-dimethylformamide (10 mL). The resulting yellow oil was directly used for the next reaction.

### (30b) tert-Butyl [2-amino-5-(4-methoxyphenoxy)phenyl]methylcarbamate

The synthesis was carried out in the same manner as in Example (28b) using tert-butyl [5-(4-methoxyphenoxy)-2-nitrophenyl]methylcarbamate produced in Example (30a) (0.65 g, 1.7 mmol), iron powder (0.47 g, 8.7 mmol), ammonium chloride (0.047 g, 0.87 mmol), ethanol (8.0 mL) and water (4.0 mL). The resulting oil was directly used for the next reaction.

### (30c) [6-(4-Methoxyphenoxy)-1-methyl-1H-benzimidazol-2-yl]methanol

The synthesis was carried out in the same manner as in Example (28c) using tert-butyl [2-amino-5-(4-methoxyphenoxy)phenyl]methylcarbamate produced in Example (30b) (0.60 g, 1.7 mmol), glycolic acid (0.40 g, 5.2 mmol) and a 4 N hydrochloric acid-1,4-dioxane solution (20 mL). The resulting dark brown oil was directly used for the next reaction.

### (30d) Methyl 3-{[6-(4-methoxyphenoxy)-1-methyl-1H-benzimidazol-2-yl]methoxy}benzoate

The reaction and post-treatment were carried out according to Example (28d) using [6-(4-methoxyphenoxy)-1-methyl-1H-benzimidazol-2-yl]methanol produced in Example (30c) (0.23 g, 0.79 mmol), methyl 3-hydroxybenzoate (0.18 g, 1.2 mmol), tri-n-butylphosphine (0.40 mL, 2.0 mmol), 1,1'-(azodicarbonyl)dipiperidine (0.39 g, 2.0 mmol) and dichloromethane (4.0 mL) to obtain the desired compound (0.25 g, yield: 75%) as a pale brown oil.

¹H-NMR (CDCl₃, 400 MHz) δ: 3.79 (3H, s), 3.81 (3H, s), 3.92 (3H, s), 5.38 (2H, s), 6.88-6.91 (3H, m), 6.97-7.01 (3H, m), 7.27-7.31 (1H, m), 7.37 (1H, t, J = 7.8 Hz), 7.66-7.73 (3H, m).

### (30e) 3-{[6-(4-Methoxyphenoxy)-1-methyl-1H-benzimidazol-2-yl]methoxy}benzoic acid

The reaction and post-treatment were carried out according to Example (28e) using methyl 3-{[6-(4-methoxyphenoxy)-1-methyl-1H-benzimidazol-2-yl]methoxy}benzoate produced in Example (30d) (0.25 g, 0.59 mmol), a 1 N sodium hydroxide aqueous solution (0.89 mL, 0.89 mmol) and 1,4-dioxane to obtain the desired compound (0.21 g, yield: 86%) as a white solid.

¹H-NMR (CDCl₃, 500 MHz) δ: 3.79 (3H, s), 3.81 (3H, s), 5.36 (2H, br. s.), 6.87-6.93 (3H, m) 6.98 (3H, d, J = 8.3 Hz), 7.20-7.29 (1H, m), 7.36 (1H, s), 7.65-7.76 (3H, m).

MS (FAB) m/z: 405 (M+H)⁺.

Anal. calcd for C₂₃H₂₀N₂O₅+1.5H₂O: C, 64.03; H, 5.37; N, 6.49. Found C, 63.96; H, 5.30; N, 6.52.

### (Example 31) 3-[6-(3-Chlorophenoxy)-1-methyl-1H-benzimidazol-2-ylmethoxy]benzoic acid hydrochloride (hydrochloride of Compound No. 1-51)

### (31a) tert-Butyl [5-(3-fluorophenoxy)-2-nitrophenyl]methylcarbamate

Sodium hydride (56%, 0.38 g, 10 mmol) was added to a solution of 3-chlorophenol (1.29 g, 10 mmol) and tert-butyl (5-chloro-2-nitrophenyl)methylcarbamate (2.87 g, 10 mmol) in N,N-dimethylformamide (20 mL) under ice-cooling. The reaction mixture was stirred at 80°C for six hours. After leaving to cool, water (100 mL) was added to the reaction mixture, followed by extraction with ethyl acetate (100 mL). Then, the organic layer was washed with water (100 mL) twice and dried over anhydrous sodium sulfate. After concentration under reduced pressure, the residue was purified by silica gel chromatography (hexane:ethyl acetate, 6:1) to obtain the title compound (3.79 g, yield: 99%) as a yellow oil.

¹H-NMR (CDCl₃, 400 MHz) δ: 1.33 (6H, s), 1.50 (3H, s), 3.27 (3H, s), 6.87 (1H, dd, J = 2.7, 8.6 Hz), 6.89 (1H, br s), 7.01 (1H, d, J = 8.1 Hz), 7.12 (1H, t, J = 2.0 Hz), 7.24-7.26 (1H, m), 7.38 (1H, t, J = 8.2 Hz), 7.96 (1H, d, J = 9.0 Hz).

### (31b) tert-Butyl [2-amino-5-(3-chlorophenoxy)phenyl]methylcarbamate

A solution of tert-butyl [5-(3-chlorophenoxy)-2-nitrophenyl]methylcarbamate obtained in Example (31a) (3.79 g, 10 mmol), ammonium chloride (0.27 g, 5.0 mmol) and iron powder (2.79 g, 50 mmol) in ethanol (50 mL) and water (25 mL) was stirred with heating under reflux for one hour. After leaving to cool, the reaction mixture was filtered through celite. The filtrate was concentrated and then water (100 mL) was added, followed by extraction with ethyl acetate (100 mL). Then, the organic layer was dried over anhydrous sodium sulfate. After concentration under reduced pressure, the residue was purified by silica gel chromatography (hexane:ethyl acetate, 5:1) to obtain the title compound (3.49 g, yield: 99%) as a yellow oil.

¹H-NMR (CDCl₃, 400 MHz) δ: 1. 41 (9H, s), 3.16 (3H, s), 3.70 (2H, br s), 6.77 (1H, d, J = 8.6 Hz), 6.82-6.90 (4H, m), 6.99-7.01 (1H, m), 7.20 (1H, t, J = 8.2 Hz).

### (31c) [6-(3-Chlorophenoxy)-1-methyl-1H-benzimidazol-2-yl]methanol

A solution of tert-butyl [2-amino-5-(3-chlorophenoxy)phenyl]methylcarbamate (3.49 g, 10 mmol) obtained in Example (31b) and glycolic acid (1.52 g, 20 mmol) in 4 M hydrochloric acid-dioxane (10 mL) was stirred with heating under reflux for nine hours. After leaving to cool, the reaction mixture was poured into a saturated sodium bicarbonate aqueous solution (100 mL), followed by extraction with ethyl acetate (100 mL). Then, the organic layer was dried over anhydrous sodium sulfate. After concentration under reduced pressure, the residue was purified by silica gel chromatography (methylene chloride:methanol, 95:5) to obtain the title compound (0.66 g, yield: 23%) as a pale brown powder.

¹H-NMR (CDCl₃, 400 MHz) δ: 3.79 (3H, s), 4.91 (2H, s), 6.88 (1H, d, J = 8.6 Hz), 6.95 (1H, s), 6.98-7.01 (2H, m), 7.05 (1H, d, J = 8.2 Hz), 7.24 (1H, d, J = 8.2 Hz), 7.69 (1H, d, J = 9.4 Hz).

### (31d) Methyl 3-{[6-(3-chlorophenoxy)-1-methyl-1H-benzimidazol-2-yl]methoxy}benzoate

A solution of [6-(3-chlorophenoxy)-1-methyl-1H-benzimidazol-2-yl]methanol obtained in Example (31c) (660 mg, 2.3 mmol), methyl 3-hydroxybenzoate (522 mg, 3.4 mmol), tri-n-butylphosphine (925 mg, 4.6 mmol) and 1,1'-(azodicarbonyl)dipiperidine (1.15 g, 4.6 mmol) in methylene chloride (5 mL) was stirred for three hours. The reaction mixture was concentrated and then purified by silica gel column chromatography (hexane:ethyl acetate, 2:1) to obtain the title compound (935 mg, yield: 97%) as a white amorphous solid.

¹H-NMR (CDCl₃, 400 MHz) δ: 3.85 (3H, s), 3.93 (3H, s), 5.41 (2H, s), 6.89 (1H, ddd, J = 0.8, 3.1, 8.2 Hz), 6.97 (1H, t, J = 2.0 Hz), 7.01-7.07 (3H, m), 7.24 (1H, d, J = 8.2 Hz), 7.29-7.32 (1H, m), 7.39 (1H, t, J = 7.8 Hz), 7.70 (1H, dt, J = 1.1, 7.4 Hz), 7.73-7.74 (1H, m), 7.77 (1H, d, J = 9.4 Hz).

### (31e) 3-[6-(3-Chlorophenoxy)-1-methyl-1H-benzimidazol-2-ylmethoxy]benzoic acid hydrochloride

A solution of methyl 3-{[6-(3-chlorophenoxy)-1-methyl-1H-benzimidazol-2-yl]methoxy}benzoate obtained in Example (31d) (933 mg, 2.21 mmol) in a 2 M sodium hydroxide aqueous solution (5 mL) and dioxane (10 mL) was stirred with heating under reflux for two hours. After leaving to cool, 5 M hydrochloric acid (20 mL) was added to the reaction mixture. The precipitated solid was collected by filtration to obtain the title compound (829 mg, yield: 84%) as a white powder.

¹H-NMR (DMSO-d₆, 400 MH) δ: 3.91 (3H, s), 5.62 (2H, s), 6.98 (1H, dd, J = 3.1, 9.0 Hz), 7.04 (1H, t, J = 2.0 Hz), 7.17 (1H, dd, J = 2.4, 9.0 Hz), 7.18-7.20 (1H, m), 7.40 (1H, d, J = 8.2 Hz), 7.42-7.44 (1H, m), 7.49 (1H, t, J = 7.8 Hz), 7.61 (1H, s), 7.62 (1H, d, J = 7.4 Hz), 7.69 (1H, s), 7.79 (1H, d, J = 9.0 Hz).

MS (FAB+) m/z: 409 (M+H)⁺.

Mp: 218-222°C.

### (Example 32) 3-{[1-Methyl-6-(3-methylphenoxy)-1H-benzimidazol-2-yl]methoxy}benzoic acid (Compound No. 1-167)

### (32a) tert-Butyl methyl[5-(3-methylphenoxy)-2-nitrophenyl]carbamate

The synthesis was carried out in the same manner as in Example (28a) using m-cresol (0.36 mL, 3.5 mmol), tert-butyl (5-chloro-2-nitrophenyl)-methyl-carbamate (1.0 g, 3.5 mmol), sodium hydride (> 56% in oil, 0.17 g, 3.8 mmol) and N,N-dimethylformamide (20 mL). The resulting yellow oil was directly used for the next reaction.

### (32b) tert-Butyl [2-amino-5-(3-methylphenoxy)phenyl]methylcarbamate

The synthesis was carried out in the same manner as in Example (28b) using tert-butyl methyl[5-(3-methylphenoxy)-2-nitrophenyl]-carbamate produced in Example (32a) (1.2 g, 3.5 mmol), iron powder (0.93 g, 17 mmol), ammonium chloride (0.093 g, 1.7 mmol), ethanol (16 mL) and water (8.0 mL). The resulting oil was directly used for the next reaction.

### (32c) [1-Methyl-6-(3-methylphenoxy)-1H-benzimidazol-2-yl]methanol

tert-Butyl [2-amino-5-(3-methylphenoxy)phenyl]methylcarbamate produced in Example (32b) (1.1 g, 3.5 mmol) was dissolved in a 4 N hydrochloric acid-1,4-dioxane solution (20 mL). Glycolic acid (0.80 g, 10 mmol) was added and the mixture was heated under reflux overnight. The solvent was evaporated under reduced pressure and then a sodium bicarbonate aqueous solution (100 mL) was added, followed by extraction with ethyl acetate (100 mL x 2). The resulting organic layer was washed with brine (80 mL) and then dried over anhydrous magnesium sulfate. The solvent was evaporated under reduced pressure. The resulting solid was washed with diisopropyl ether to obtain the desired compound (0.59 g, yield: 63%).

¹H-NMR (CDCl₃, 400 MHz) δ: 2.32 (3H, s), 3.75 (3H, s), 4.90 (2H, s), 6.80 (2H, s), 6.90 (1H, d, J = 7.4 Hz), 6.94-7.02 (2H, m), 7.21 (1H, t, J = 7.8 Hz), 7.65 (1H, d, J = 8.6 Hz).

### (32d) Methyl 3-{[1-methyl-6-(3-methylphenoxy)-1H-benzimidazol-2-yl]methoxy}benzoate

The reaction and post-treatment were carried out according to Example (28d) using [1-methyl-6-(3-methylphenoxy)-1H-benzimidazol-2-yl]methano produced in Example (32c) (0.24 g, 0.89 mmol), methyl 3-hydroxybenzoate (0.20 g, 1.3 mmol), tri-n-butylphosphine (0.45 mL, 1.8 mmol), 1,1'-(azodicarbonyl)dipiperidine (0.45 g, 1.8 mmol) and dichloromethane (6.0 mL) to obtain the desired compound (0.31 g, yield: 85%) as a white solid.

¹H-NMR (CDCl₃, 400 MHz) δ: 3.81 (3H, br s), 3.92 (3H, s), 5.39 (2H, s), 6.78-6.83 (2H, m), 6.91 (1H, d, J = 7.8 Hz), 6.97-7.04 (2H, m), 7.21 (1H, t, J = 8.0 Hz), 7.27-7.32 (1H, m), 7.38 (1H, t, J = 7.8 Hz), 7.69 (1H, td, J = 1.2, 1.4, 7.6 Hz), 7.71-7.75 (2H, m).
MS (FAB) m/z: 403 (M+H)⁺.

### (32e) 3-{[1-Methyl-6-(3-methylphenoxy)-1H-benzimidazol-2-yl]methoxy}benzoic acid

The reaction and post-treatment were carried out according to Example (28e) using methyl 3-{[1-methyl-6-(3-methylphenoxy)-1H-benzimidazol-2-yl]methoxy}benzoate produced in Example (32d) (0.29 g, 0.72 mmol), a 1 N sodium hydroxide aqueous solution (1.1 mL, 1.1 mmol) and 1,4-dioxane (1.0 mL) to obtain the desired compound (0.17 g, yield: 60%) as a white solid.

¹H-NMR (DMSO-d₆, 400 MHz) δ: 2.27 (3H, s), 3.81 (3H, s), 5.47 (2H, s), 6.74-6.83 (2H, m), 6.87-6.97 (2H, m), 7.32 (1H, d, J = 2.4 Hz), 7.36-7.41 (1H, m), 7.45 (1H, t, J = 8.0 Hz), 7.58 (1H, dt, J = 1.2, 1.4, 7.6 Hz), 7.62-7.68 (2H, m).

MS (FAB) m/z: 389 (M+H)⁺.

Anal. calcd for C₂₃H₂₀N₂O₄+0.33H₂O: C, 70.04; H, 5.28; N, 7.10. Found C, 69.99; H, 5.16; N, 7.15.

### (Example 33) 3-({1-Methyl-6-[3-(trifluoromethoxy)phenoxy]-1H-benzimidazol-2-yl}methoxy]benzoic acid (Compound No. 1-176)

### (33a) tert-Butyl methyl{2-nitro-5-[3-(trifluoromethoxy)phenoxy]phenyl}carbamate

The desired title compound (4.28 g, yield: 99%) was obtained as a yellow oil according to the method described in Example (31a) using 3-trifluoromethoxyphenol (1.78 g, 10 mmol), tert-butyl (5-chloro-2-nitrophenyl)methylcarbamate (2.87 g, 10 mmol) and sodium hydride (56%, 0.38 g, 10 mmol).

¹H-NMR (CDCl₃, 400 MHz) δ: 1.33 (6H, s), 1.50 (3H, s), 3.27 (3H, s), 6.89-6.91 (2H, m), 6.99 (1H, s.), 7.05 (1H, d, J = 8.6 Hz), 7.13 (1H, d, J = 7.8 Hz), 7.47 (1H, t, J - 8.2 Hz), 7.97 (1H, d, J = 8.6 Hz).

### (33b) tert-Butyl {2-amino-5-[3-(trifluoromethoxy)phenoxy]phenyl}methylcarbamate

The desired title compound (3.98 g, yield: 99%) was obtained as a yellow oil according to the method described in Example (31b) using tert-butyl methyl {2-nitro-5-[3-(trifluoromethoxy)phenoxy]phenyl}carbamate obtained in Example (33a) (2.87 g, 10 mmol) and iron powder (2.79 g, 50 mmol).

¹H-NMR (CDCl₃, 400 MHz) δ: 1.41 (9H, brs), 3.15 (3H, s), 3.72 (2H, br s), 6.76-6.89 (5H, m), 7.26-7.30 (2H, m).

### (33c) {1-Methyl-6-[3-(trifluoromethoxy)phenoxy]-1H-benzimidazol-2-yl}methanol

The desired title compound (3.08 g, yield: 91%) was obtained as a pale brown powder according to the method described in Example (31c) using tert-butyl {2-amino-5-[3-(trifluoromethoxy)phenoxy]phenyl}methylcarbamate obtained in Example (33b) (3.98 g, 10 mmol) and glycolic acid (1.52 g, 20 mmol).

¹H-NMR (CDCl₃, 400 MHz) δ: 3.79 (3H, s), 4.91 (2H, s), 6.85 (1H, s), 6.89 (1H, dd, J = 2.4, 8.6 Hz), 6.92-6.95 (1H, m), 6.98-7.01 (2H, m), 7.32 (1H, t, J = 8.2 Hz), 7.67 (1H, d, J = 9.0 Hz).

### (33d) Methyl 3-({1-methyl-6-[3-(trifluoromethoxy)phenoxy]-1H-benzimidazol-2-yl}methoxy)benzoate

The desired title compound (3.52 g, yield: 91%) was obtained as a white powder according to the method described in Example (31d) using {1-methyl-6-[3-(trifluoromethoxy)phenoxyl-1H-benzimidazol-2-yl}methanol obtained in Example (33c) (3.08 g, 9.1 mmol), methyl 3-hydroxybenzoate (2.08 g, 13.7 mmol), tri-n-butylphosphine (3.68 g, 18.2 mmol) and 1,1'-(azodicarbonyl)dipiperidine (4.59 g, 18.2 mmol).

¹H-NMR (CDCl₃, 400 MHz) δ: 3.85 (3H, s), 3.93 (3H, s), 5.42 (2H, s), 6.86 (1H, s), 6.91 (1H, dd, J = 2.4, 7.4 Hz), 6.93-6.96 (1H, m), 7.02-7.06 (2H, m), 7.29-7.34 (2H, m), 7.39 (1H, t, J = 7.4 Hz), 7.69-7.74 (2H, m), 7.77 (1H, d, J = 8.2 Hz).

### (33e) 3-({1-Methyl-6-[3-(trifluoromethoxy)phenoxy]-1H-benzimidazol-2-yl}methoxy]benzoic acid

A solution of methyl 3-({1-methyl-6-[3-(trifluoromethoxy)phenoxy]-1H-benzimidazol-2-yl}methoxy)benzoate obtained in Example (33d) (3.52 g, 7.45 mmol) in a 2 M sodium hydroxide aqueous solution (20 mL) and dioxane (40 mL) was stirred with heating under reflux for two hours. After leaving to cool, 1 M hydrochloric acid (50 mL) was added to the reaction mixture, and the precipitated solid was collected by filtration. The solid was dissolved in a 1 M sodium hydroxide aqueous solution (50 mL), and 1 M hydrochloric acid (50 mL) was added. The precipitated solid was collected by filtration to obtain the title compound (2.75 g, yield: 81%) as a white powder.

¹H-NMR (DMSO-d₆, 400 MHz) δ: 3.83 (3H, s), 5.48 (2H, s), 6.95-6.97 (2H, m), 7.00 (1H, dd, J = 2.4, 8.6 Hz), 7.07-7.09 (1H, m), 7.36-7.39 (1H, m), 7.43-7.49 (3H, m), 7.58 (1H, dt, J = 1.2, 7.8 Hz), 7.64 (1H, dd, J = 1.2, 2.4 Hz), 7.71 (1H, d, J = 8.2 Hz), 13.08 (1H, br s).

MS (FAB+) m/z: 459 (M+H)⁺.

Mp: 221-227°C.

### (Example 34) 3-{[6-(4-Fluoro-3-methylphenoxy)-1-methyl-1H-benzimidazol-2-yl]methoxy}benzoic acid (Compound No. 1-187)

### (34a) tert-Butyl [5-(4-fluoro-3-methylphenoxy)-2-nitrophenyl]methylcarbamate

The synthesis was carried out in the same manner as in Example (28a) using 4-fluoro-3-methylphenol (0.78 mL, 7.0 mmol), tert-butyl (5-chloro-2-nitrophenyl)methylcarbamate (2.0 g, 7.0 mmol), sodium hydride (> 56% in oil, 0.28 g, 7.0 mmol) and N,N-dimethylformamide (20 mL). The resulting yellow oil was directly used for the next reaction.

### (34b) tert-Butyl [2-amino-5-(4-fluoro-3-methylphenoxy)phenyl]methylcarbamate

The synthesis was carried out in the same manner as in Example (28b) using tert-butyl [5-(4-fluoro-3-methylphenoxy)-2-nitrophenyl]methylcarbamate produced in Example (34a) (2.6 g, 7.0 mmol), iron powder (1.9 g, 35 mmol), ammonium chloride (0.19 g, 3.5 mmol), ethanol (20 mL) and water (10 mL). The resulting oil was directly used for the next reaction.

### (34c) [6-(4-Fluoro-3-methylphenoxy)-1-methyl-1H-benzimidazol-2-yl]methanol

tert-Butyl [2-amino-5-(4-fluoro-3-methylphenoxy)phenyl]methylcarbamate produced in Example (34b) (2.4 g, 7.0 mmol) was dissolved in 5 N hydrochloric acid (20 mL) and 1,4-dioxane (20 mL). Glycolic acid (0.80 g, 10 mmol) was added and the mixture was heated under reflux overnight. The reaction solution was cooled to room temperature and a sodium bicarbonate aqueous solution (100 mL) was added, followed by extraction with ethyl acetate (100 mL × 2). The resulting organic layer was washed with brine (80 mL) and then dried over anhydrous magnesium sulfate. The solvent was evaporated under reduced pressure. The resulting solid was washed with diisopropyl ether to obtain the desired compound (1.5 g, yield: 77%) as a pale red brown solid.

¹H-NMR (CDCl₃, 400 MHz) δ: 2.25 (3H, d, J = 2.0 Hz), 3.78 (3H, s), 4.05 (1H, br s), 4.91 (2H, s), 6.75-6.81 (1H, m), 6.83 (2H, dd, J = 2.9, 6.1 Hz), 6.87 (1H, d, J = 2.0 Hz), 6.96 (2H, d, J = 8.6 Hz), 7.60 (1H, d, J = 8.6 Hz).

### (34d) Methyl 3-{[6-(4-fluoro-3-methylphenoxy)-1-methyl-1H-benzimidazol-2-yl]methoxy}benzoate

The reaction and post-treatment were carried out according to Example (28d) using [6-(4-fluoro-3-methylphenoxy)-1-methyl-1H-benzimidazol-2-yl]methanol produced in Example (34c) (0.25 g, 0.88 mmol), methyl 3-hydroxybenzoate (0.20 g, 1.3 mmol), tri-n-butylphosphine (0.44 mL, 1.8 mmol), 1,1'-(azodicarbonyl)dipiperidine (0.44 g, 1.8 mmol) and dichloromethane (4 mL) to obtain the desired compound (0.30 g, yield: 80%) as a white solid.

¹H-NMR (CDCl₃, 400 MHz) δ: 2.25 (3H, d, J = 2.0 Hz), 3.81 (3H, s), 3.92 (3H, s), 5.39 (2H, s), 6.76-6.86 (2H, m), 6.92-7.00 (3H, m), 7.27-7.31 (1H, m), 7.38 (1H, t, J = 8.0 Hz), 7.71 (3H, d, J = 8.6 Hz).

### (34e) 3-{[6-(4-Fluoro-3-methylphenoxy)-1-methyl-1H-benzimidazol-2-yl]methoxy}benzoic acid

The reaction and post-treatment were carried out according to Example (28e) using methyl 3-{[6-(4-fluoro-3-methylphenoxy)-1-methyl-1H-benzimidazol-2-yl]methoxy}benzoate produced in Example (34d) (0.31 g, 0.72 mmol), a 1 N sodium hydroxide aqueous solution (1.2 mL, 1.2 mmol) and 1,4-dioxane (1.0 mL) to obtain the desired compound (0.24 g, yield: 81%) as a white solid.

¹H-NMR (DMSO-d₆, 500 MHz) δ: 2.20 (3H, s), 3.81 (3H, s), 5.46 (2H, s), 6.80-6.87 (1 H, m), 6.89-6.95 (2H, m), 7.12 (1H, t, J = 9.0 Hz), 7.28 (1H, d, J = 2.0 Hz), 7.36-7.41 (1H, m), 7.45 (1H, t, J = 7.8 Hz), 7.57 (1H, d, J = 7.8 Hz), 7.62-7.67 (2H, m), 13.03 (1H, br s).

MS (FAB) m/z: 407 (M+H)⁺.

Anal. calcd for C₂₃H₁₉FN₂O₄+0.10HCl: C, 67.37; H, 4.69; F, 4.63; N, 6.83; Cl, 0.86. Found C, 67.24; H, 4.70; F, 4.56; N, 7.00; Cl, 0.64.

### (Example 35) 3-{[6-(3,4-Difluorophenoxy)-1-methyl-1H-benzimidazol-2-yl]methoxy}benzoic acid (Compound No. 1-180)

### (35a) tert-Butyl [5-(3,4-difluorophenoxy)-2-nitrophenyl]methylcarbamate

The synthesis was carried out in the same manner as in Example (28a) using 3,4-difluorophenol (0.95 g, 7.0 mmol), tert-butyl (5-chloro-2-nitrophenyl)methylcarbamate (2.0 g, 7.0 mmol), sodium hydride (> 56% in oil, 0.28 g, 7.0 mmol) and N,N-dimethylformamide (20 mL). The resulting yellow solid was directly used for the next reaction.

### (35b) tert-Butyl [2-amino-5-(3,4-difluorophenoxy)phenyl]methylcarbamate

The synthesis was carried out in the same manner as in Example (28b) using tert-butyl [5-(3,4-difluorophenoxy)-2-nitrophenyl]methylcarbamate produced in Example (35a) (2.7 g, 7.0 mmol), iron powder (1.9 g, 35 mmol), ammonium chloride (0.19 g, 3.5 mmol), ethanol (20 mL) and water (10 mL). The resulting oil was directly used for the next reaction.

### (35c) [6-(3,4-Difluorophenoxy)-1-methyl-1H-benzimidazol-2-yl]methanol

The reaction and post-treatment were carried out according to Example (34c) using tert-butyl [2-amino-5-(3,4-difluorophenoxy)phenyl]methylcarbamate produced in Example (35b) (2.4 g, 7.0 mmol), glycolic acid (0.80 g, 10 mmol), a 5 N hydrochloric acid solution (20 mL) and 1,4-dioxane (20 mL) to obtain the desired compound (1.5 g, yield: 76%) as a pale brown solid.

¹H-NMR (CDCl₃, 400 MHz) δ: 3.78 (3H, s), 4.90 (2H, s), 6.67-6.74 (1H, m), 6.77-6.85 (1H, m), 6.97 (2H, s), 7.11 (1H, q, J = 9.3 Hz), 7.67 (1H, d, J = 8.2 Hz).

### (35d) Methyl 3-{[6-(3,4-difluorophenoxy)-1-methyl-1H-benzimidazol-2-yl]methoxy}benzoate

The reaction and post-treatment were carried out according to Example (28d) using [6-(3,4-difluorophenoxy)-1-methyl-1H-benzimidazol-2-yl]methanol produced in Example (35c) (0.25 g, 0.88 mmol), methyl 3-hydroxybenzoate (0.20 g, 1.3 mmol), tri-n-butylphosphine (0.44 mL, 1.8 mmol), 1,1'-(azodicarbonyl)dipiperidine (0.44 g, 1.8 mmol) and dichloromethane (4.0 mL) to obtain the desired compound (0.30 g, yield: 80%) as a white solid.

¹H-NMR (CDCl₃, 400 MHz) δ: 3.84 (3H, s), 3.94 (3H, s), 5.40 (2H, s), 5.41 (10H, br s), 6.69-6.75 (1H, m), 6.79-6.85 (1H, m), 6.97-7.02 (2H, m), 7.07-7.15 (1H, m), 7.28-7.32 (1H, m), 7.38 (1H, t, J = 8.1 Hz), 7.67-7.71 (1H, m), 7.74 (2H, s).

MS (FAB) m/z: 425 (M+H)⁺.

### (35e) 3-{[6-(3,4-Difluorophenoxy)-1-methyl-1H-benzimidazol-2-yl]methoxy}benzoic acid

The reaction and post-treatment were carried out according to Example (28e) using methyl 3-{[6-(3,4-difluorophenoxy)-1-methyl-1H-benzimidazol-2-yl]methoxy}benzoate produced in Example (35d) (0.31 g, 0.72 mmol), a 1 N sodium hydroxide aqueous solution (1.2 mL, 1.2 mmol) and 1,4-dioxane (1.0 mL) to obtain the desired compound (0.25 g, yield: 82%) as a white solid.

¹H-NMR (DMSO-d₆, 400 MHz) δ: 3.82 (3H, s), 5.47 (2H, s), 6.77-6.84 (1H, m), 6.97 (1H, dd, J = 2.4, 8.6 Hz), 7.10-7.18 (1H, m), 7.35-7.48 (4H, m), 7.55-7.70 (3H, m).

MS (FAB) m/z: 411 (M+H)⁺.

Anal. calcd for C₂₂H₁₆F₂N₂O₄+0.10HCl: C, 67.37; H, 4.69; F, 4.63; N, 6.83; Cl, 0.86. Found C, 67.24; H, 4.70; F, 4.56; N, 7.00; Cl, 0.64.

### (Example 36) 3-({1-Methyl-6-[3-(trifluoromethyl)phenoxy]-1H-benzimidazol-2-yl}methoxy)benzoic acid (Compound No. 1-175)

### (36a) tert-Butyl methyl{2-nitro-5-[3-(trifluoromethyl)phenoxy]phenyl}carbamate

The synthesis was carried out in the same manner as in Example (28a) using 3-(trifluoromethyl)phenol (0.87 mL, 7.0 mmol), tert-butyl (5-chloro-2-nitrophenyl)methylcarbamate (2.00 g, 7.0 mmol), sodium hydride (> 56% in oil, 0.28 g, 7.0 mmol) and N,N-dimethylformamide (20 mL). The resulting yellow solid was directly used for the next reaction.

### (36b) tert-Butyl {2-amino-5-[3-(trifluoromethyl)phenoxy]phenyl}methylcarbamate

The synthesis was carried out in the same manner as in Example (28b) using tert-butyl methyl{2-nitro-5-[3-(trifluoromethyl)phenoxy]phenyl}carbamate produced in Example (36a) (2.9 g, 7.0 mmol), iron powder (1.9 g, 35 mmol), ammonium chloride (0.19 g, 3.5 mmol), ethanol (20 mL) and water (10 mL). The resulting oil was directly used for the next reaction.

### (36c) {1-Methyl-6-[3-(trifluoromethyl)phenoxy]-1H-benzimidazol-2-yl}methanol

The reaction and post-treatment were carried out according to Example (34c) using tert-butyl {2-amino-5-[3-(trifluoromethyl)phenoxy]phenyl}methylcarbamate produced in Example (36b) (2.4 g, 7.0 mmol), glycolic acid (0.80 g, 10 mmol), a 5 N hydrochloric acid solution (20 mL) and 1,4-dioxane (20 mL) to obtain the desired compound (1.3 g, yield: 57%) as a pale brown solid.

¹H-NMR (CDCl₃, 400 MHz) δ: 3.78 (3H, s), 4.93 (2H, s), 6.97-7.04 (2H, m), 7.09-7.18 (1H, m), 7.21 (1H, s), 7.33 (1H, d, J = 8.6 Hz), 7.43 (1H, t, J = 7.8 Hz), 7.71 (1H, d, J = 8.2 Hz).

### (36d) Methyl 3-({1-methyl-6-[3-(trifluoromethyl)phenoxy]-1H-benzimidazol-2-yl}methoxy)benzoate

The reaction and post-treatment were carried out according to Example (28d) using {1-methyl-6-[3-(trifluoromethyl)phenoxy]-1H-benzimidazol-2-yl}methanol produced in Example (36c) (0.25 g, 0.88 mmol), methyl 3-hydroxybenzoate (0.20 g, 1.3 mmol), tri-n-butylphosphine (0.44 mL, 1.8 mmol), 1,1'-(azodicarbonyl)dipiperidine (0.44 g, 1.8 mmol) and dichloromethane (4.0 mL) to obtain the desired compound (0.33 g, yield: 81%) as a white solid.

¹H-NMR (CDCl₃, 400 MHz) δ: 3.85 (3H, s), 3.93 (3H, s), 5.41 (8H, s), 5.41 (2H, s), 7.00-7.06 (2H, m), 7.15 (1H, dd, J = 2.4, 8.2 Hz), 7.23 (1H, s), 7.28-7.46 (4H, m), 7.68-7.79 (3H, m).

MS (FAB) m/z: 457 (M+H)⁺.

### (36e) 3-({1-Methyl-6-[3-(trifluoromethyl)phenoxy]-1H-benzimidazol-2-yl}methoxy]benzoic acid

The reaction and post-treatment were carried out according to Example (28e) using methyl 3-({1-methyl-6-[3-(trifluoromethyl)phenoxy]-1H-benzimidazol-2-yl}methoxy)benzoate produced in Example (36d) (0.33 g, 0.72 mmol), a 1 N sodium hydroxide aqueous solution (1.1 mL, 1.1 mmol) and 1,4-dioxane (1.0 mL) to obtain the desired compound (0.28 g, yield: 89%) as a white solid.

¹H-NMR (DMSO-d₆, 400 MHz) δ: 3.84 (3H, s), 5.48 (2H, s), 7.01 (1H, dd, J = 2.2, 8.8 Hz), 7.22-7.28 (2H, m), 7.32-7.50 (4H, m), 7.60 (3H, s), 7.72 (1H, d, J = 8.6 Hz).

MS (FAB) m/z: 443 (M+H)⁺.

Anal. calcd for C₂₃H₁₇F₃N₂O₄+0.10HCl: C, 61.93; H, 3.86; F, 12.78; N, 6.28; Cl, 0.79. Found C, 61.78; H, 3.85; F, 12.55; N, 6.37; Cl, 0.83.

### (Example 37) 3-{[6-(3-Fluoro-4-methylphenoxy)-1-methyl-1H-benzimidazol-2-yl]methoxy}benzoic acid (Compound No. 1-186)

### (37a) tert-Butyl [5-(3-fluoro-4-methylphenoxy)-2-nitrophenyl]methylcarbamate

The synthesis was carried out in the same manner as in Example (28a) using 3-fluoro-4-methylphenol (0.93 g, 7.4 mmol), tert-butyl (5-chloro-2-nitrophenyl)methylcarbamate (2.0 g, 7.0 mmol), sodium hydride (> 56% in oil, 0.29 g, 7.4 mmol) and N,N-dimethylformamide (20 mL). The resulting yellow oil was directly used for the next reaction.

### (37b) tert-Butyl [2-amino-5-(3-fluoro-4-methylphenoxy)phenyl]methylcarbamate

The synthesis was carried out in the same manner as in Example (28b) using tert-butyl [5-(3-fluoro-4-methylphenoxy)-2-nitrophenyl]methylcarbamate produced in Example (37a) (2.6 g, 7.0 mmol), iron powder (1.9 g, 35 mmol), ammonium chloride (0.19 g, 3.5 mmol), ethanol (20 mL) and water (10 mL). The resulting oil was directly used for the next reaction.

### (37c) [6-(3-Fluoro-4-methylphenoxy)-1-methyl-1H-benzimidazol-2-yl]methanol

The reaction and post-treatment were carried out according to Example (34c) using tert-butyl [2-amino-5-(3-fluoro-4-methylphenoxy)phenyl]methylcarbamate produced in Example (37b) (2.4 g, 7.0 mmol), glycolic acid (0.80 g, 10 mmol), a 5 N hydrochloric acid solution (20 mL) and 1,4-dioxane (20 mL) to obtain the desired compound (1.6 g, yield: 81%) as a pale brown solid.

¹H-NMR (CDCl₃, 400 MHz) δ: 2.24 (3H, d, J = 2.0 Hz), 3.76 (3H, s), 4.90 (2H, s), 6.63-6.72 (2H, m), 6.92-7.01 (2H, m), 7.11 (1H, t, J = 9.0 Hz), 7.65 (1H, d, J = 8.6 Hz).

### (37d) Methyl 3-{[6-(3-fluoro-4-methylphenoxy)-1-methyl-1H-benzimidazol-2-yl]methoxy}benzoate

The reaction and post-treatment were carried out according to Example (28d) using [6-(3-fluoro-4-methylphenoxy)-1-methyl-1H-benzimidazol-2-yl]methanol produced in Example (37c) (0.25 g, 0.88 mmol), methyl 3-hydroxybenzoate (0.20 g, 1.3 mmol), tri-n-butylphosphine (0.44 mL, 1.8 mmol), 1,1'-(azodicarbonyl)dipiperidine (0.44 g, 1.8 mmol) and dichloromethane (4 mL) to obtain the desired compound (0.30 g, yield: 82%) as a white solid.

¹H-NMR (CDCl₃, 400 MHz) δ: 2.24 (3H, d, J = 1.6 Hz), 3.82 (3H, s), 3.92 (3H, s), 5.40 (2H, s), 6.66-6.71 (2H, m), 6.98-7.04 (2H, m), 7.11 (1H, t, J = 9.0 Hz), 7.27-7.32 (1H, m), 7.38 (1H, t, J = 8.0 Hz), 7.67-7.70 (1H, m), 7.71-7.75 (2H, m).

MS (FAB) m/z: 421 (M+H)⁺.

### (37e) 3-{[6-(3-Fluoro-4-methylphenoxy)-1-methyl-1H-benzimidazol-2-yl]methoxy}benzoic acid

The reaction and post-treatment were carried out according to Example (28e) using methyl 3-{[6-(3-fluoro-4-methylphenoxy)-1-methyl-1H-benzimidazol-2-yl]methoxy}benzoate produced in Example (37d) (0.30 g, 0.72 mmol), a 1 N sodium hydroxide aqueous solution (1.1 mL, 1.1 mmol) and 1,4-dioxane (1.0 mL) to obtain the desired compound (0.27 g, yield: 94%) as a white solid.

¹H-NMR (DMSO-d₆, 400 MHz) δ: 2.18 (3H, s), 3.82 (3H, s), 5.47 (2H, s), 6.72 (6H, dd, J = 2.4, 8.6 Hz), 6.80 (6H, dd, J = 2.5, 11.1 Hz), 6.95 (6H, dd, J = 2.4, 8.6 Hz), 7.25 (6H, t, J = 8.6 Hz), 7.35-7.40 (2H, m), 7.36 (1H, d, J = 2.4 Hz), 7.45 (1H, t, J = 7.8 Hz), 7.58 (1H, d, J = 7.8 Hz), 7.67 (1H, d, J = 8.6 Hz), 7.63-7.69 (1H, m), 13.08 (1H, br s).

MS (FAB) m/z: 407 (M+H)⁺.

Anal. calcd for C₂₃H₁₉FN₂O₄: C, 67.97; H, 4.71; F, 4.67; N, 6.89. Found C, 67.58; H, 4.63; F, 4.67; N, 6.91.

### (Example 38) 3-{[6-(3-Chloro-5-fluorophenoxy)-1-methyl-1H-benzimidazol-2-yl]methoxy}benzoic acid (Compound No. 1-207)

### (38a) tert-Butyl [5-(3-chloro-5-fluorophenoxy)-2-nitrophenyl]methylcarbamate

The desired title compound (7.94 g, yield: 99%) was obtained as a yellow powder according to the method described in Example (31a) using 3-chloro-5-fluorophenol (2.93 g, 20 mmol), tert-butyl (5-chloro-2-nitrophenyl)methylcarbamate (5.73 g, 20 mmol) and sodium hydride (56%, 0.76 g, 20 mmol).

¹H-NMR (CDCl₃, 400 MHz) δ: 1.33 (6H, s), 1.51 (3H, s), 3.28 (3H, s), 6.74 (1H, dt, J = 2.4, 9.0 Hz), 6.90-6.93 (3H, m), 7.00 (1H, d, J = 7.8 Hz), 7.98 (1H, d, J = 8.6 Hz).

### (38b) tert-Butyl [2-amino-5-(3-chloro-5-fluorophenoxy)phenyl]methylcarbamate

The desired title compound (7.34 g, yield: 99%) was obtained as a brown oil according to the method described in Example (31b) using tert-butyl [5-(3-chloro-5-fluorophenoxy)-2-nitrophenyl]methylcarbamate obtained in Example (38a) (7.94 g, 20 mmol) and iron powder (5.59 g, 100 mmol).

¹H-NMR (CDCl₃, 400 MHz) δ: 1.42 (9H, s), 3.15 (3H, s), 3.74 (2H, br s), 6.53 (1H, d, J = 9.8 Hz), 6.68-6.82 (4H, m), 7.04 (1H, dd, J = 2.0, 8.6 Hz).

### (38c) [6-(3-Chloro-5-fluorophenoxy)-1-methyl-1H-benzimidazol-2-yl]methanol

The desired title compound (2.99 g, yield: 49%) was obtained as a white powder according to the method described in Example (31c) using tert-butyl [2-amino-5-(3-chloro-5-fluorophenoxy)phenyl]methylcarbamate obtained in Example (38b) (7.34 g, 20 mmol) and glycolic acid (3.04 g, 40 mmol).

¹H-NMR (CDCl₃, 400 MHz) δ: 3.80 (3H, s), 4.91 (2H, s), 6.58 (1H, dt, J = 2.4, 10.2 Hz), 6.98-7.02 (2H, m), 7.23 (1H, dd, J = 2.0, 9.4 Hz), 7.32 (1H, d, J = 2.0 Hz), 7.70 (1H, d, J = 8.6 Hz).

### (38d) Methyl 3-{[6-(3-chloro-5-fluorophenoxy)-1-methyl-1H-benzimidazol-2-yl]methoxy}benzoate

The desired title compound (1.30 g, yield: 32%) was obtained as a white powder according to the method described in Example (31d) using [6-(3-chloro-5-fluorophenoxy)-1-methyl-1H-benzimidazol-2-yl]methanol obtained in Example (38c) (2.99 g, 9.75 mmol), methyl 3-hydroxybenzoate (2.22 g, 14.6 mmol), tri-n-butylphosphine (3.94 g, 19.5 mmol) and 1,1'-(azodicarbonyl)dipiperidine (4.92 g, 19.5 mmol).

¹H-NMR (CDCl₃, 400 MHz) δ: 3.87 (3H, s), 3.93 (3H, s), 5.42 (2H, s), 6.59 (1H, dt, J = 2.4, 9.8 Hz), 6.75 (1H, s) 6.80 (1H, dt, J = 2.4, 7.8 Hz), 7.03 (1H, dd, J = 2.0, 8.6 Hz), 7.07 (1H, d, J = 2.4 Hz), 7.31 (1H, ddd, J = 1.2, 2.7, 8.2 Hz), 7.39 (1H, t, J = 7.8 Hz), 7.70 (1H, dd, J = 1.2, 7.8 Hz), 7.74 (1H, s), 7.79 (1H, d, J = 8.6 Hz).

### (38e) 3-[6-(3-Chloro-5-fluorophenoxy)-1-methyl-1H-benzimidazol-2-ylmethoxy]benzoic acid

The desired title compound (1.26 g, yield: 86%) was obtained as a white powder according to the method described in Example (33e) using methyl 3-{[6-(3-chloro-5-fluorophenoxy)-1-methyl-1H-benzimidazol-2-yl]methoxy}benzoate obtained in Example (38d) (1.30 g, 2.95 mmol).

¹H-NMR (DMSO-d₆, 400 MHz) δ: 3.84 (3H, s), 5.48 (2H, s), 6.83-6.86 (2H, m), 7.02 (1H, dd, J = 2.4, 9.0 Hz), 7.15 (1H, dt, J = 2.0, 8.6 Hz), 7.39 (1H, ddd, J = 1.2, 2.4, 8.2 Hz), 7.45 (1H, t, J = 7.4 Hz), 7.49 (1H, d, J = 2.4 Hz), 7.58 (1H, dt, J = 1.2, 7.4 Hz), 7.65 (1H, dd, J - 1.2, 2.4 Hz), 7.72 (1H, d, J = 8.6 Hz), 13.04 (1H, s).

MS (FAB+) m/z: 427 (M+H)⁺.

Mp: 209-213°C.

### (Example 39) 3-{[6-(3,5-Difluorophenoxy)-1-methyl-1H-benzimidazol-2-yl]methoxy}benzoic acid (Compound No. 1-181)

### (39a) tert-Butyl [5-(3,5-difluorophenoxy)-2-nitrophenyl]methylcarbamate

The synthesis was carried out in the same manner as in Example (28a) using 3,5-difluorophenol (2.9 g, 22 mmol), tert-butyl (5-chloro-2-nitrophenyl)methylcarbamate (6.0 g, 21 mmol), sodium hydride (> 56% in oil, 0.88 g, 22 mmol) and N,N-dimethylformamide (30 mL). The resulting yellow solid was directly used for the next reaction.

### (39b) tert-Butyl [2-amino-5-(3,5-difluorophenoxy)phenyl]methylcarbamate

The synthesis was carried out in the same manner as in Example (28b) using tert-butyl [5-(3,5-difluorophenoxy)-2-nitrophenyl]methylcarbamate produced in Example (39a) (8.4 g, 22 mmol), iron powder (5.9 g, 110 mmol), ammonium chloride (0.59 g, 11 mmol), ethanol (30 mL) and water (15 mL). The resulting red brown solid was directly used for the next reaction.

### (39c) [6-(3,5-Difluorophenoxy)-1-methyl-1H-benzimidazol-2-yl]methanol

The reaction and post-treatment were carried out according to Example (34c) using tert-butyl [2-amino-5-(3,5-difluorophenoxy)phenyl]methylcarbamate produced in Example (39b) (7.7 g, 22 mmol), glycolic acid (2.5 g, 33 mmol), a 5 N hydrochloric acid solution (40 mL) and 1,4-dioxane (40 mL) to obtain the desired compound (4.5 g, yield: 69%) as a pale gray solid.

¹H-NMR (CDCl₃, 400 MHz) δ: 3.80 (3H, s), 4.92 (2H, s), 6.42-6.55 (3H, m), 7.00 (1H, dd, J = 2.4, 8.6 Hz), 7.04 (1H, d, J = 2.4 Hz), 7.71 (1H, d, J = 8.6 Hz).

### (39d) Methyl 3-{[6-(3,5-difluorophenoxy)-1-methyl-1H-benzimidazol-2-yl]methoxy}benzoate

The reaction and post-treatment were carried out according to Example (28d) using [6-(3,5-difluorophenoxy)-1-methyl-1H-benzimidazol-2-yl]methanol produced in Example (39c) (0.25 g, 0.88 mmol), methyl 3-hydroxybenzoate (0.20 g, 1.3 mmol), tri-n-butylphosphine (0.44 mL, 1.8 mmol), 1,1'-(azodicarbonyl)dipiperidine (0.44 g, 1.8 mmol) and dichloromethane (4.0 mL) to obtain the desired compound (0.32 g, yield: 86%) as a white solid.

¹H-NMR (CDCl₃, 400 MHz) δ: 3.86 (3H, s), 3.92 (3H, s), 3.93 (9H, s), 5.41 (2H, s), 6.44-6.55 (2H, m), 7.03 (1H, dd, J = 2.2, 8.8 Hz), 7.07 (1H, d, J = 2.4 Hz), 7.27-7.33 (2H, m), 7.39 (1H, t, J = 7.8 Hz), 7.67-7.71 (1H, m), 7.73 (1H, dd, J = 1.4, 2.5 Hz), 7.78 (1H, d, J = 8.6 Hz).

### (39e) 3-{[6-(3,5-Difluorophenoxy)-1-methyl-1H-benzimidazol-2-yl]methoxy}benzoic acid

The reaction and post-treatment were carried out according to Example (28e) using methyl 3-{[6-(3,5-difluorophenoxy)-1-methyl-1H-benzimidazol-2-yl]methoxy}benzoate produced in Example (39d) (0.32 g, 0.75 mmol), a 1 N sodium hydroxide aqueous solution (1.9 mL, 1.9 mmol) and 1,4-dioxane (1.5 mL) to obtain the desired compound (0.23 g, yield: 76%) as a white solid.

¹H-NMR (DMSO-d₆, 400 MHz) δ: 3.85 (3H, s), 5.47 (2H, s), 6.69 (2H, dd, J = 2.2, 8.8 Hz), 6.95 (1H, tt, J = 2.2, 9.3 Hz), 7.02 (1H, dd, J = 2.4, 8.6 Hz), 7.30-7.36 (1H, m), 7.42 (1H, t, J = 7.8 Hz), 7.48 (1H, d, J = 2.4 Hz), 7.57 (1H, d, J = 7.4 Hz), 7.63 (1H, dd, J = 1.6, 2.4 Hz), 7.72 (1H, d, J = 8.6 Hz).

MS (FAB) m/z: 411 (M+H)⁺.

Anal. calcd for C₂₂H₁₆F₂N₂O₄+1.00H₂O: C, 61.68; H, 4.24; F, 8.87; N, 6.54. Found C, 61.58; H, 3.95; F, 9.06; N, 6.51.

### (Example 40) 3-{[6-(3-Fluoro-5-methylphenoxy)-1-methyl-1H-benzimidazol-2-yl]methoxy}benzoic acid (Compound No. 1-188)

### (40a) 1-Fluoro-3-methoxy-5-methylbenzene

A mixed solution of 3-bromo-5-fluoroanisole (2.05 g, 10 mmol), trimethylboroxine (50% solution in THF, 2.51 g, 20 mmol), PdCl₂(dppf) (0.82 g, 1.0 mmol) and cesium carbonate (6.52 g, 20 mmol) in dioxane (100 mL) and water (50 mL) was stirred with heating under reflux for 10 hours. After leaving to cool, water (100 mL) was added to the reaction solution, followed by extraction with ethyl acetate (200 mL) twice. The organic layers were washed with water (100 mL) twice and dried over anhydrous sodium sulfate. After concentration under reduced pressure, the residue was purified by silica gel chromatography (hexane:ethyl acetate, 6:1) to obtain the title compound (1.40 g, yield: 66%) as a yellow oil.

¹H-NMR (CDCl₃, 400 MHz) δ: 1.78 (3H, s), 6.44 (1H, dd, J = 2.0, 11.0 Hz), 6.50 (1H, d, J = 11.0 Hz), 6.51 (1H, s).

### (40b) 3-Fluoro-5-methylphenol

A solution of 1-fluoro-3-methoxy-5-methylbenzene obtained in Example (40a) (0.92 g, 6.56 mmol) and boron tribromide (1.0 M solution in methylene chloride, 8.53 mL, 8.53 mmol) in methylene chloride (20 mL) was stirred at 0°C for 10 hours. Water (100 mL) was added to the reaction solution, followed by extraction with methylene chloride (100 mL). Then, the organic layer was dried over anhydrous sodium sulfate. After concentration under reduced pressure, the residue was purified by silica gel chromatography (hexane:ethyl acetate, 1:1) to obtain the title compound (0.83 g, yield: 99%) as a yellow oil.

¹H-NMR (CDCl₃, 400 MHz) δ: 2.06 (3H, s), 4.97 (1H, s), 6.37 (1H, dt, J = 2.4, 10.2 Hz), 6.44 (1H, s), 6.48 (1H, d, J = 8.6 Hz).

### (40c) tert-Butyl [5-(3-fluoro-5-methylphenoxy)-2-nitrophenyl]methylcarbamate

The desired title compound (1.71 g, yield: 69%) was obtained as a yellow oil according to the method described in Example (31a) using 3-fluoro-5-methylphenol obtained in Example (40b) (0.83 g, 6.56 mmol), tert-butyl (5-chloro-2-nitrophenyl)methylcarbamate (1.88 g, 6.56 mmol) and sodium hydride (56%, 0.25 g, 6.56 mmol).

¹H-NMR (CDCl₃, 400 MHz): δ 1.33 (6H, s), 1.51 (3H, s), 2.38 (3H, s), 3.27 (3H, s), 6.63 (1H, dt, J = 2.4, 9.4 Hz), 6.71 (1H, s), 6.80 (1H, d, J = 8.6 Hz), 6.86-6.88 (2H, m), 7.95 (1H, d, J = 8.2 Hz).

### (40d) tert-Butyl [2-amino-5-(3-fluoro-5-methylphenoxy)phenyl]methylcarbamate

The desired title compound (1.38 g, yield: 88%) was obtained as a yellow oil according to the method described in Example (31b) using tert-butyl [5-(3-fluoro-5-methylphenoxy)-2-nitrophenyl]methylcarbamate obtained in Example (40c) (1.71 g, 4.54 mmol) and iron powder (1.27 g, 22.7 mmol).

¹H-NMR (CDCl₃, 400 MHz) δ: 1.41 (9H, s), 2.29 (3H, s), 3.15 (3H, s), 3.70 (2H, br s), 6.42-6.56 (3H, m), 6.75-6.83 (3H, m).

### (40e) [6-(3-Fluoro-5-methylphenoxy)-1-methyl-1H-benzimidazol-2-yl]methanol

The desired title compound (0.76 g, yield: 67%) was obtained as a pale brown oil according to the method described in Example (31c) using tert-butyl [2-amino-5-(3-fluoro-5-methylphenoxy)phenyl]methylcarbamate obtained in Example (40d) (1.38 g, 3.98 mmol) and glycolic acid (0.61 g, 7.97 mmol).

¹H-NMR (CDCl₃, 400 MHz) δ: 2.31 (3H, s), 3.78 (3H, s), 4.92 (2H, s), 6.49 (1H, dt, J = 2.0, 10.2 Hz), 6.58 (1H, s), 6.61 (1H, d, J = 11.0 Hz), 6.99-7.01 (2H, m), 7.69 (1H, d, J = 9.4 Hz).

### (40f) Methyl 3-{[6-(3-fluoro-5-methylphenoxy)-1-methyl-1H-benzimidazol-2-yl]methoxy}benzoate

The desired title compound (0.82 g, yield: 74%) was obtained as a white powder according to the method described in Example (31d) using [6-(3-fluoro-5-methylphenoxy)-1-methyl-1H-benzimidazol-2-yl]methanol obtained in Example (40e) (0.76 g, 2.65 mmol), methyl 3-hydroxybenzoate (0.61 g, 3.98 mmol), tri-n-butylphosphine (1.07 g, 5.31 mmol) and 1,1'-(azodicarbonyl)dipiperidine (1.34 g, 5.31 mmol).

¹H-NMR (CDCl₃, 400 MHz) δ: 2.30 (3H, s), 3.84 (3H, s), 3.93 (3H, s), 5.40 (2H, s), 6.49 (1H, dt, J = 2.4, 10.2 Hz), 6.58 (1H, s), 6.60 (1H, d, J = 10.6 Hz), 7.00-7.03 (2H, m), 7.28-7.31 (1H, m), 7.38 (1H, t, J = 7.8 Hz), 7.68 (1H, dt, J = 1.6, 8.6 Hz), 7.72-7.76 (2H, m).

### (40g) 3-{[6-(3-Fluoro-5-methylphenoxy)-1-methyl-1H-benzimidazol-2-yl]methoxy}benzoic acid

The desired title compound (0.55 g, yield: 73%) was obtained as a white powder according to the method described in Example (33e) using methyl 3-{[6-(3-fluoro-5-methylphenoxy)-1-methyl-1H-benzimidazol-2-yl]methoxy}benzoate obtained in Example (40f) (0.82 g, 1.86 mmol).

¹H-NMR (DMSO-d₆, 400 MHz) δ: 2.25 (3H, s), 3.83 (3H, s), 5.47 (2H, s), 6.58-6.60 (2H, m), 6.75 (1H, d, J = 10.6 Hz), 6.96 (1H, dd, J = 2.4, 8.6 Hz), 7.37-7.39 (2H, m), 7.45 (1H, t, J = 7.4 Hz), 7.57 (1H, dt, J = 1.2, 7.8 Hz), 7.64 (1H, dd, J = 1.2, 2.4 Hz), 7.68 (1H, d, J = 8.6 Hz), 13.03 (1H, s).

MS (FAB+) m/z: 407 (M+H)⁺.

Mp: 224-226°C.

### (Example 41) 3-{[6-(2,5-Difluorophenoxy)-1-methyl-1H-benzimidazol-2-yl]methoxy}benzoic acid (Compound No. 1-179)

### (41a) tert-Butyl [5-(2,5-difluorophenoxy)-2-nitrophenyl]methylcarbamate

The reaction and post-treatment were carried out according to Example (28a) using 2,5-difluorophenol (5.1 g, 38 mmol), tert-butyl (5-chloro-2-nitrophenyl)methylcarbamate (9.8 g, 34 mmol), sodium hydride (> 56% in oil, 1.5 g, 38 mmol) and N,N-dimethylformamide (90 mL) to obtain the desired compound (12 g, yield: 92%) as a yellow solid.

¹H-NMR (CDCl₃, 400 MHz) δ: 1.32 (6H, s), 1.50 (3H, br. s.), 3.28 (3H, s), 6.81-7.07 (4H, m), 7.17-7.26 (1H, m), 7.96 (1 H, d, J = 9.0 Hz).

### (41b) tert-Butyl [2-amino-5-(2,5-difluorophenoxy)phenyl]methylcarbamate

The synthesis was carried out in the same manner as in Example (28b) using tert-butyl [5-(2,5-difluorophenoxy)-2-nitrophenyl]methylcarbamate produced in Example (41a) (12 g, 31 mmol), iron powder (8.4 g, 160 mmol), ammonium chloride (0.84 g, 16 mmol), ethanol (30 mL) and water (15 mL). The resulting red brown solid was directly used for the next reaction.

### (41c) [6-(2,5-Difluorophenoxy)-1-methyl-1H-benzimidazol-2-yl]methanol

The reaction and post-treatment were carried out according to Example (34c) using tert-butyl [2-amino-5-(2,5-difluorophenoxy)phenyl]methylcarbamate produced in Example (41b) (11 g, 31 mmol), glycolic acid (3.6 g, 47 mmol), a 5 N hydrochloric acid solution (40 mL) and 1,4-dioxane (40 mL) to obtain the desired compound (7.6 g, yield: 83%) as a pale brown solid.

¹H-NMR (CDCl₃, 400 MHz) δ: 3.77 (3H, s), 4.91 (2H, s), 6.60-6.71 (1H, m), 6.71-6.82 (1H, m), 6.97-7.06 (2 H, m), 7.09-7.21 (1H, m), 7.68 (1H, d, J = 9.0 Hz).

### (41d) Methyl 3-{[6-(2,5-difluorophenoxy)-1-methyl-1H-benzimidazol-2-yl]methoxy}benzoate

The reaction and post-treatment were carried out according to Example (28d) using [6-(2,5-difluorophenoxy)-1-methyl-1H-benzimidazol-2-yl]methanol produced in Example (41c) (0.25 g, 0.86 mmol), methyl 3-hydroxybenzoate (0.20 g, 1.3 mmol), tri-n-butylphosphine (0.43 mL, 1.7 mmol), 1,1'-(azodicarbonyl)dipiperidine (0.43 g, 1.7 mmol) and dichloromethane (4.0 mL) to obtain the desired compound (0.28 g, yield: 78%) as a white solid.

¹H-NMR (CDCl₃, 400 MHz): δ ppm: 3.84 (3 H, s), 3.92 (3 H, s), 5.40 (2 H, s), 6.65-6.71 (1 H, m), 6.73-6.80 (1 H, m), 7.01-7.05 (2 H, m), 7.11-7.18 (1 H, m), 7.29 (1 H, dd, J = 2.7, 8.2 Hz), 7.38 (1H, t, J = 8.0 Hz), 7.67-7.77 (3 H, m).

### (41e) 3-{[6-(2,5-Difluorophenoxy)-1-methyl-1H-benzimidazol-2-yl]methoxy}benzoic acid

The reaction and post-treatment were carried out according to Example (28e) using methyl 3-{[6-(2,5-difluorophenoxy)-1-methyl-1H-benzimidazol-2-yl]methoxy}benzoate produced in Example (41d) (0.28 g, 0.67 mmol), a 1 N sodium hydroxide aqueous solution (1.0 mL, 1.0 mmol) and 1,4-dioxane (1.0 mL) to obtain the desired compound (0.25 g, yield: 91%) as a white solid.

¹H-NMR (DMSO-d₆, 500 MHz) δ: 3.83 (3H, s), 5.47 (2H, s), 6.88-6.95 (1H, m), 6.98-7.05 (2H, m), 7.38 (2H, s), 7.41-7.48 (2H, m), 7.58 (1H, d, J = 7.3 Hz), 7.63 (1H, s), 7.68 (1H, d, J = 8.8 Hz), 13.03 (1H, br s).

MS (FAB) m/z: 411 (M+H)⁺.

Anal. calcd for C₂₂H₁₆F₂N₂O₄+0.25H₂O: C, 63.69; H, 4.01; F, 9.16; N, 6.75. Found C, 63.84; H, 4.05; F, 9.22; N, 6.83.

### (Example 42) 3-{[6-(3-Ethylphenoxy)-1-methyl-1H-benzimidazol-2-yl]methoxy}benzoic acid (Compound No. 1-170)

### (42a) tert-Butyl [5-(3-ethylphenoxy)-2-nitrophenyl]methylcarbamate

The desired title compound (20.5 g, yield: 97%) was obtained as a yellow oil according to the method described in Example (31a) using 3-ethylphenol (6.72 g, 55 mmol), tert-butyl (5-chloro-2-nitrophenyl)methylcarbamate (14.19 g, 49.5 mmol) and sodium hydride (56%, 2.10 g, 55.0 mmol).

¹H-NMR (CD Cl₃, 400 MHz) δ: 1.27 (3H, t, J = 7.4 Hz), 1.33 (6H, s), 1.50 (3H, s), 2.69 (2H, q, J = 7.4 Hz), 3.26 (3H, s), 6.82-6.94 (4H, m), 7.11 (1H, d, J = 7.0 Hz), 7.35 (1H, t, J = 7.4 Hz), 7.94 (1H, d, J = 8.6 Hz).

### (42b) tert-Butyl [2-amino-5-(3-ethylphenoxy)phenyl]methylcarbamate

The desired title compound (18.2 g, yield: 99%) was obtained as a pale brown oil according to the method described in Example (31b) using tert-butyl [5-(3-ethylphenoxy)-2-nitrophenyl]methylcarbamate obtained in Example (42a) (19.8 g, 53.2 mmol) and iron powder (14.9 g, 266 mmol).

¹H-NMR (CDCl₃, 400 MHz) δ: 1.22 (3H, t, J = 7.4 Hz), 1.56 (9H, s), 2.61 (2H, q, J = 7.4 Hz), 3.15 (3H, s), 3.66 (2H, br s), 6.73-6.84 (5H, m), 6.88 (1H, d, J = 7.4 Hz), 7.19 (1H, t, J = 7.8 Hz).

### (42c) [6-(3-Ethylphenoxy)-1-methyl-1H-benzimidazol-2-yl]methanol

The desired title compound (15.0 g, yield: 83%) was obtained as a brown powder according to the method described in Example (31c) using tert-butyl [2-amino-5-(3-ethylphenoxy)phenyl]methylcarbamate obtained in Example (42b) (18.2 g, 53.2 mmol) and glycolic acid (8.09 g, 106 mmol).

¹H-NMR (CDCl₃, 400 MHz) δ: 1.23 (3H, t, J = 7.8 Hz), 2.63 (2H, q, J = 7.4 Hz), 3.77 (3H, s), 4.08 (2H, s), 6.80 (1H, dd, J = 2.0, 8.2 Hz), 6.86 (1H, s), 6.94-6.95 (2H, m), 6.98 (1H, dd, J = 2. 4, 9.0 Hz), 7.24 (1H, t, J = 7.8 Hz), 7.62 (1H, d, J = 8.6 Hz).

### (42d) Methyl 3-{[6-[3-ethylphenoxy]-1-methyl-1H-benzimidazol-2-yl]methoxy}benzoate

The desired title compound (18.5 g, yield: 80%) was obtained as a pale yellow oil according to the method described in Example (31d) using [6-(3-ethylphenoxy)-1-methyl-1H-benzimidazol-2-yl]methanol obtained in Example (42c) (12.5 g, 44.3 mmol), methyl 3-hydroxybenzoate (10.1 g, 66.5 mmol), tri-n-butylphosphine (17.9 g, 88.7 mmol) and 1,1'-(azodicarbonyl)dipiperidine (22.4 g, 88.7 mmol).

¹H-NMR (CDCl₃, 400 MHz,) δ: 1.23 (3H, t, J = 7.8 Hz), 2.63 (2H, q, J = 7.4 Hz), 3.82 (3H, s), 3.93 (3H, s), 5.40 (2H, s), 6.82 (1H, dd, J = 2.4, 8.2 Hz), 6.87 (1H, s), 6.95 (1H, d, J = 7.0 Hz), 7.01 (1H, d, J = 2.4 Hz), 7.03 (1H, dd, J = 2.4, 8.6 Hz), 7.23 (1H, d, J = 7.8 Hz), 7.29-7.32 (1H, m), 7.38 (1H, t, J = 7.4 Hz), 7.70 (1H, d, J = 7.4 Hz), 7.72-7.74 (2H, m).

### (42e) 3-{[6-(3-Ethylphenoxy)-1-methyl-1H-benzimidazol-2-yl]methoxy}benzoic acid

The desired title compound (14.2 g, yield: 83%) was obtained as a white powder according to the method described in Example (33e) using methyl 3-{[6-[3-ethylphenoxy]-1-methyl-1H-benzimidazol-2-yl]methoxy}benzoate obtained in Example (42d) (14.7 g, 35.3 mmol).

¹H-NMR (DMSO-d₆, 400 MHz) δ: 1.15 (3H, t, J = 7.4 Hz), 2.57 (2H, q, J = 7.4 Hz), 3.81 (3H, s), 5.47 (2H, s), 6.76 (1H, dd, J = 2.4, 8.2 Hz), 6.84 (1H, s), 6.92-9.95 (2H, m), 7.25 (1H, t, J = 7.8 Hz), 7.32 (1H, d, J = 2.4 Hz), 7.37-7.40 (1H, m), 7.45 (1H, t, J = 7.8 Hz), 7.58 (1H, d, J = 7.4 Hz), 7.63-7.65 (1H, m), 7.66 (1H, d, J = 8.6 Hz), 13.03 (1H, br s).

MS (FAB+) m/z: 403 (M+H)⁺.

Mp: 204-208°C.

### (Example 43) 3-{[6-(2,4-Difluorophenoxy)-1-methyl-1H-benzimidazol-2-yl]methoxy}benzoic acid (Compound No. 1-178)

### (43a) 5-(2,4-Difluorophenoxy)-N-methyl-2-nitroaniline

The synthesis was carried out in the same manner as in Example (28a) using 2,4-difluorophenol (5.2 g, 38 mmol), 5-chloro-N-methylnitroaniline (6.5 g, 35 mmol), sodium hydride (> 56% in oil, 1.8 g, 46 mmol) and N,N-dimethylformamide (80 mL). The resulting yellow solid was directly used for the next reaction.

### (43b) 4-(2,4-Difluorophenoxy)-2-N-methylaminoaniline

The synthesis was carried out in the same manner as in Example (28b) using 5-(2,4-difluorophenoxy)-N-methyl-2-nitroaniline (9.8 g, 35 mmol) produced in Example (43a), iron powder (9.3 g, 170 mmol), ammonium chloride (0.93 g, 17 mmol), ethanol (30 mL) and water (15 mL). The resulting red brown oil was directly used for the next reaction.

### (43c) [6-(2,4-Difluorophenoxy)-1-methyl-1H-benzimidazol-2-yl]methanol

The reaction and post-treatment were carried out according to Example (34c) using 4-(2,4-difluorophenoxy)-2-N-methylaminoaniline produced in Example (43b) (2.4 g, 7.0 mmol), glycolic acid (3.1 g, 41 mmol), a 5 N hydrochloric acid solution (40 mL) and 1,4-dioxane (40 mL) to obtain the desired compound (7.7 g, yield: 76%) as a reddish gray solid.

¹H-NMR (CDCl₃, 400 MHz) δ: 3.74 (3H, s), 4.58 (1H, br s), 4.87 (2H, s), 6.79-6.90 (2H, m), 6.90-7.07 (3H, m), 7.61 (1H, d, J = 8.6 Hz).

### (43d) Methyl 3-{[6-(2,4-difluorophenoxy)-1-methyl-1H-benzimidazol-2-yl]methoxy}benzoate

The reaction and post-treatment were carried out according to Example (28d) using [6-(2,4-difluorophenoxy)-1-methyl-1H-benzimidazol-2-yl]methanol produced in Example (43c) (0.25 g, 0.86 mmol), methyl 3-hydroxybenzoate (0.20 g, 1.3 mmol), tri-n-butylphosphine (0.43 mL, 1.7 mmol), 1,1'-(azodicarbonyl)dipiperidine (0.43 g, 1.7 mmol) and dichloromethane (4.0 mL) to obtain the desired compound (0.31 g, yield: 84%) as a white solid.

¹H-NMR (CDCl₃, 400 MHz) δ: 3.81 (3H, s), 3.92 (3H, s), 5.39 (2H, s), 6.81-6.88 (1H, m), 6.90 (1H, d, J = 2.4 Hz), 6.94-7.07 (3H, m), 7.29 (1H, d, J = 1.6 Hz), 7.37 (1H, t, J = 8.2 Hz), 7.66-7.76 (3H, m).

MS (FAB) m/z: 425 (M+H)⁺.

### (43e) 3-{[6-(2,4-Difluorophenoxy)-1-methyl-1H-benzimidazol-2-yl]methoxy}benzoic acid

The reaction and post-treatment were carried out according to Example (28e) using methyl 3-{[6-(2,4-difluorophenoxy)-1-methyl-1H-benzimidazol-2-yl]methoxy}benzoate produced in Example (43d) (0.31 g, 0.73 mmol), a 1 N sodium hydroxide aqueous solution (1.2 mL, 1.2 mmol) and 1,4-dioxane (1.0 mL) to obtain the desired compound (0.14 g, yield: 48%) as a white solid.

¹H-NMR (DMSO-d₆, 400 MHz) δ: 3.80 (3H, s), 5.45 (2H, s), 6.93 (1H, dd, J = 2.5, 8.8 Hz), 7.05-7.12 (1H, m), 7.19 (1H, dt, J = 5.9, 9.2 Hz), 7.25 (1H, d, J = 2.4 Hz), 7.32-7.37 (1H, m), 7.43 (1H, t, J = 7.82 Hz), 7.44-7.51 (1H, m), 7.54-7.59 (1H, m), 7.62 (1H, dd, J = 1.4, 2.5 Hz), 7. 64 (1H, d, J = 8. 6 Hz).

MS (FAB) m/z: 411 (M+H)⁺.

Anal. calcd for C₂₂H₁₆F₂N₂O₄+0.50H₂O: C, 63.01; H, 4.09; F, 9.06; N, 6.68. Found C, 63.13; H, 3.86; F, 9.20; N, 6.70.

### (Example 44) 3-{[1-Methyl-6-(2-methylphenoxy)-1H-benzimidazol-2-yl]methoxy}benzoic acid (Compound No. 1-166)

### (44a) tert-Butyl methyl[5-(2-methylphenoxy)-2-nitrophenyl]carbamate

A crude product of the desired title compound was obtained as a brown oil according to the method described in Example (28a) using 2-methylphenol (4.53 g, 41.9 mmol), tert-butyl (5-chloro-2-nitrophenyl)methylcarbamate (10.0 g, 34.9 mmol) and sodium hydride (63%, 1.59 g, 41.9 mmol). The crude product was directly used for the next reaction.

### (44b) tert-Butyl [2-amino-5-(2-methylphenoxy)phenyl]methylcarbamate

A crude product of the desired title compound was obtained as a brown oil according to the method described in Example (28b) using tert-butyl methyl[5-(2-methylphenoxy)-2-nitrophenyl]carbamate obtained in Example (44a) (12.5 g, 34.9 mmol), iron powder (9.74 g, 174 mmol) and ammonium chloride (0.933 g, 17.4 mmol). The crude product was directly used for the next reaction.

### (44c) [1-Methyl-6-(2-methylphenoxy)-1H-benzimidazol-2-yl]methanol

The desired title compound (6.71 g, yield: 72%) was obtained as a brown powder according to the method described in Example (28c) using tert-butyl [2-amino-5-(2-methylphenoxy)phenyl]methylcarbamate obtained in Example (44b) (11.5 g, 34.9 mmol) and glycolic acid (3.98 g, 52.3 mmol).

¹H NMR (CDCl₃, 400 MHz) δ: 2.28 (3H, s), 3.73 (3H, s), 4.86 (2H, s), 6.80 (1H, d, J = 2.0 Hz), 6.83-6.87 (1H, m), 6.91 (1H, dd, J = 2.0, 8.6 Hz), 7.03-7.08 (1H, m), 7.12-7.18 (1H, m), 7.24-7.29 (1H, m), 7.59 (1H, d, J = 8.6 Hz).

### (44d) Methyl 3-{[1-methyl-6-(2-methylphenoxy)-1H-benzimidazol-2-yl]methoxy}benzoate

The desired title compound (7.33 g, yield: 81%) was obtained as a white powder according to the method described in Example (28d) using [1-methyl-6-(2-methylphenoxy)-1H-benzimidazol-2-yl]methanol obtained in Example (44c) (6.00 g, 22.4 mmol), methyl 3-hydroxybenzoate (4.08 g, 26.8 mmol), tri-n-butylphosphine (8.38 mL, 33.5 mmol) and 1,1'-(azodicarbonyl)dipiperidine (8.46 g, 33.5 mmol).

¹H NMR (CDCl₃, 400 MHz) δ: 2.29 (3H, s), 3.79 (3H, s), 3.92 (3H, s), 5.38 (2H, s), 6.84 (1H, d, J = 2.3 Hz), 6.88 (1H, d, J = 8.2 Hz), 6.97 (1H, dd, J = 2.3, 9.0 Hz), 7.04-7.10 (1H, m), 7.13-7.19 (1H, m), 7.25-7.32 (2H, m), 7.37 (1H, t, J = 7.8 Hz), 7.66-7.73 (3H, m).

### (44e) 3-{[1-Methyl-6-(2-methylphenoxy)-1H-benzimidazol-2-yl]methoxy}benzoic acid

The desired title compound (6.13 g, yield: 98%) was obtained as a white powder according to the method described in Example (28e) using methyl 3-{[1-methyl-6-(2-methylphenoxy)-1H-benzimidazol-2-yl]methoxy}benzoate (6.50 g, 16.2 mmol) obtained in Example (44d) and a 1 N sodium hydroxide aqueous solution (24.2 mL, 24.2 mmol).

¹H NMR (DMSO-d₆, 500 MHz) δ: 2.25 (3H, s), 3.79 (3H, s), 5.45 (2H, s), 6.81 (1H, d, J = 7.8Hz), 6.86 (1H, dd, J = 2.4, 8.8 Hz), 7.06 (1H, t, J = 7.3 Hz), 7.14-7.21 (2H, m), 7.32 (1H, d, J = 7.3 Hz), 7.36-7.41 (1H, m), 7.45 (1H, t, J = 8.1 Hz), 7.57 (1H, d, J = 7.3 Hz), 7.63 (2H, d, J - 8.3 Hz), 13.03 (1H, s).

MS (FAB) m/z: 389 (M+H)⁺.

### (Example 45) 3-{[6-(2-Fluoro-5-methylphenoxy)-1-methyl-1H-benzimidazol-2-yl]methoxy}benzoic acid (Compound No. 1-184)

### (45a) tert-Butyl [5-(2-fluoro-5-methylphenoxy)-2-nitrophenyl]methylcarbamate

The synthesis was carried out in the same manner as in Example (28a) using 2-fluoro-5-methylphenol (5.1 g, 40 mmol), tert-butyl (5-chloro-2-nitrophenyl)methylcarbamate (10 g, 36 mmol), sodium hydride (> 56% in oil, 1.6 g, 40 mmol) and N,N-dimethylformamide (70 mL). The resulting yellow oil was directly used for the next reaction.

### (45b) tert-Butyl [2-amino-5-(2-fluoro-5-methylphenoxy)phenyl]methylcarbamate

The synthesis was carried out in the same manner as in Example (28b) using tert-butyl [5-(2-fluoro-5-methylphenoxy)-2-nitrophenyl]methylcarbamate produced in Example (45a) (15 g, 40 mmol), iron powder (11 g, 200 mmol), ammonium chloride (1.1 g, 20 mmol), ethanol (30 mL) and water (15 mL). The resulting oil was directly used for the next reaction.

### (45c) [6-(2-Fluoro-5-methylphenoxy)-1-methyl-1H-benzimidazol-2-yl]methanol

The reaction and post-treatment were carried out according to Example (34c) using tert-butyl [2-amino-5-(2-fluoro-5-methylphenoxy)phenyl]methylcarbamate produced in Example (45b) (15 g, 40 mmol), glycolic acid (4.0 g, 52 mmol), a 5 N hydrochloric acid solution (30 mL) and 1,4-dioxane (30 mL) to obtain the desired compound (8.0 g, yield: 70%) as a pale brown solid.

¹H-NMR (CDCl₃, 400 MHz) δ: 2.27 (3H, s), 3.75 (3H, s), 4.89 (2H, s), 6.78-6.84 (1H, m), 6.85-6.91 (1H, m), 6.92 (1H, d, J = 2.4 Hz), 6.98 (1H, dd, J = 2.2, 8.8 Hz), 7.07 (1H, dd, J = 8.6, 10. 6 Hz), 7.64 (1H, d, J = 9.0 Hz).

### (45d) Methyl 3-{[6-(2-fluoro-5-methylphenoxy)-1-methyl-1H-benzimidazol-2-yl]methoxy}benzoate

The reaction and post-treatment were carried out according to Example (28d) using [6-(2-fluoro-5-methylphenoxy)-1-methyl-1H-benzimidazol-2-yl]methanol produced in Example (45c) (0.25 g, 0.94 mmol), methyl 3-hydroxybenzoate (0.16 g, 1.0 mmol), tri-n-butylphosphine (0.47 mL, 1.9 mmol), 1,1'-(azodicarbonyl)dipiperidine (0.47 g, 1.9 mmol) and dichloromethane (4 mL) to obtain the desired compound (0.20 g, yield: 49%) as a white solid.

¹H-NMR (CDCl₃, 400 MHz) δ: 2.27 (3H, s), 3.82 (3H, s), 3.92 (3H, s), 5.39 (2H, s), 6.82 (1H, s), 6.86-6.92 (1H, m), 6.95 (1H, d, J = 2.4 Hz), 7.01 (1H, dd, J = 2.4, 9.0 Hz), 7.07 (1H, dd, J = 8.2, 10.6 Hz), 7.28-7.31 (1H, m), 7.38 (1H, t, J = 7.8 Hz), 7.67-7.73 (3H, m).

MS (FAB) m/z: 421 (M+H)⁺.

### (45e) 3-{[6-(2-Fluoro-5-methylphenoxy)-1-methyl-1H-benzimidazol-2-yl]methoxy}benzoic acid

The reaction and post-treatment were carried out according to Example (28e) using methyl 3-{[6-(2-fluoro-5-methylphenoxy)-1-methyl-1H-benzimidazol-2-yl]methoxy}benzoate produced in Example (45d) (0.20 g, 0.46 mmol), a 1 N sodium hydroxide aqueous solution (0.70 mL, 0.70 mmol) and 1,4-dioxane (1.0 mL) to obtain the desired compound (0.18 g, yield: 95%) as a white solid.

¹H-NMR (DMSO-d₆, 400 MHz) δ: 2.23 (3H, s), 3.81 (3H, s), 5.46 (2H, s), 6.87 (1H, s), 6.93 (1H, dd, J = 2. 4, 9.0 Hz), 6.94-6.99 (1H, m), 7.22-7.31 (2H, m), 7.39 (1H, s), 7.45 (1H, t, J = 7.8 Hz), 7.58 (1H, d, J = 7.4 Hz), 7.62-7.68 (2H, m).

MS (FAB) m/z: 407 (M+H)⁺.

Anal. calcd for C₂₃H₁₉FN₂O₄+0.20H₂O: C, 67.38; H, 4.77; F, 4.63; N, 6.83. Found C, 67.43; H, 4.71; F, 4.80; N, 6.87.

### (Example 46) 3-{[6-(4-Fluoro-2-methylphenoxy)-1-methyl-1H-benzimidazol-2-yl]methoxy}benzoic acid (Compound No. 1-183)

### (46a) tert-Butyl [5-(4-fluoro-2-methylphenoxy)-2-nitrophenyl]methylcarbamate

A crude product of the desired title compound was obtained as a brown oil according to the method described in Example (28a) using 4-fluoro-2-methylphenol (5.28 g, 41.9 mmol), tert-butyl (5-chloro-2-nitrophenyl)methylcarbamate (10.0 g, 34.9 mmol) and sodium hydride (63%, 1.59 g, 41.9 mmol). The crude product was directly used for the next reaction.

### (46b) tert-Butyl [2-amino-5-(4-fluoro-2-methylphenoxy)phenyl]methylcarbamate

A crude product of the desired title compound was obtained as a brown powder according to the method described in Example (28b) using tert-butyl [5-(4-fluoro-2-methylphenoxy)-2-nitrophenyl]methylcarbamate obtained in Example (46a) (13.1 g, 34.9 mmol), iron powder (9.74 g, 174 mmol) and ammonium chloride (0.933 g, 17.4 mmol). The crude product was directly used for the next reaction.

### (46c) [6-(4-Fluoro-2-methylphenoxy)-1-methyl-1H-benzimidazol-2-yl]methanol

The desired title compound (8.62 g, yield: 86%) was obtained as a brown powder according to the method described in Example (28c) using tert-butyl [2-amino-5-(4-fluoro-2-methylphenoxy)phenyl]methylcarbamate obtained in Example (46b) (12.1 g, 34.9 mmol) and glycolic acid (3.98 g, 52.3 mmol).

¹H NMR (CDCl₃, 400 MHz) δ: 2.25 (3H, s), 3.73 (3H, s), 4.86 (2H, s), 6.73 (1H, d, J = 2.3 Hz), 6.84-6.90 (3H, m), 6.96-7.00 (1H, m), 7.59 (1H, d, J = 8.6 Hz).

### (46d) Methyl 3-{[6-(4-fluoro-2-methylphenoxy)-1-methyl-1H-benzimidazol-2-yl]methoxy}benzoate

The desired title compound (8.49 g, yield: 77%) was obtained as a white powder according to the method described in Example (28d) using [6-(4-fluoro-2-methylphenoxy)-1-methyl-1H-benzimidazol-2-yl]methanol obtained in Example (46c) (7.50 g, 26.2 mmol), methyl 3-hydroxybenzoate (4.78 g, 31.4 mmol), tri-n-butylphosphine (7.85 mL, 31.4 mmol) and 1,1'-(azodicarbonyl)dipiperidine (7.93 g, 31.4 mmol).

¹H NMR (CDCl₃, 400 MHz) δ: 2.25 (3H, s), 3.79 (3H, s), 3.92 (3H, s), 5.38 (2H, s), 6.77 (1H, d, J = 2.3 Hz), 6.84-7.02 (4H, m), 7.25-7.33 (1H, m), 7.37 (1H, t, J = 8.0 Hz), 7.66-7.74 (3H, m).

### (46e) 3-{[6-(4-Fluoro-2-methylphenoxy)-1-methyl-1H-benzimidazol-2-yl]methoxy}benzoic acid

The desired title compound (6.48 g, yield: 89%) was obtained as a white powder according to the method described in Example (28e) using methyl 3-{[6-(4-fluoro-2-methylphenoxy)-1-methyl-1H-benzimidazol-2-yl]methoxy}benzoate obtained in Example (46d) (7.50 g, 17.8 mmol) and a 1 N sodium hydroxide aqueous solution (26.8 mL, 26.8 mmol).

¹H NMR (DMSO-d₆, 400 MHz) δ: 2.22 (3H, s), 3.78 (3H, s), 5.45 (2H, s), 6.82-6.91 (2H, m), 6.98-7.05 (1H, m), 7.12 (1H, d, J = 2.3 Hz), 7.20 (1H, dd, J = 3.1, 9.4 Hz), 7.35-7.40 (1H, m), 7.45 (1H, t, J = 7.8 Hz), 7.55-7.59 (1H, m), 7.60-7.65 (2H, m), 13.03 (1H, s).

MS (FAB) m/z: 407 (M+H)⁺.

### (Example 47) 3-{[6-(3-Fluoro-5-methoxyphenoxy)-1-methyl-1H-benzimidazol-2-yl]methoxy}benzoic acid (Compound No. 1-195)

### (47a) tert-Butyl [5-(3-fluoro-5-methoxyphenoxy)-2-nitrophenyl]methylcarbamate

The reaction and post-treatment were carried out according to Example (27a) using known [WO2005037763] 3-fluoro-5-methoxyphenol (4.5 g, 32 mmol), tert-butyl (5-chloro-2-nitrophenyl)methylcarbamate (7.7 g, 27 mmol), sodium hydride (> 56% in oil, 1.3 g, 32 mmol) and N,N-dimethylformamide (60 mL) to obtain the desired compound (3.7 g, yield: 57%).

¹H-NMR (CDCl₃, 400 MHz) δ: 1.32 (6H, s), 1.50 (3H, s), 3.27 (3H, s), 3.81 (3H, s), 6.37-6.46 (2H, m), 6.52 (1H, d, J = 11.0 Hz), 6.86-6.96 (2H, m), 7.91-8.03 (1H, m).

### (47b) tert-Butyl [2-amino-5-(3-fluoro-5-methoxyphenoxy)phenyl]methylcarbamate

The synthesis was carried out in the same manner as in Example (28b) using tert-butyl [5-(3-fluoro-5-methoxyphenoxy)-2-nitrophenyl]methylcarbamate produced in Example (47a) (3.7 g, 9.5 mmol), iron powder (2.5 g, 47 mmol), ammonium chloride (0.25 g, 4.7 mmol), ethanol (30 mL) and water (15 mL). The resulting pale brown oil was directly used for the next reaction.

### (47c) [6-(3-Fluoro-5-methoxyphenoxy)-1-methyl-1H-benzimidazol-2-yl]methanol

The reaction and post-treatment were carried out according to Example (34c) using tert-butyl [2-amino-5-(3-fluoro-5-methoxyphenoxy)phenyl]methylcarbamate produced in Example (47b) (3.1 g, 8.7 mmol), glycolic acid (0.86 g, 11 mmol), a 5 N hydrochloric acid solution (10 mL) and 1,4-dioxane (10 mL) to obtain the desired compound (2.0 g, yield: 75%) as a pale brown solid.

¹H-NMR (CDCl₃, 400 MHz) δ: 3.72 (3H, s), 3.75 (3H, s), 4.90 (2H, d, J = 5.5 Hz), 6.54 (1H, dd, J = 2.9, 6.8 Hz), 6.57-6.63 (1H, m), 6.95 (1H, d, J = 2.4 Hz), 7.00 (1H, dd, J = 2.2, 8.8 Hz), 7.10 (1H, dd, J = 9.0, 10.6 Hz), 7.66 (1H, d, J = 8.6 Hz).

### (47d) Methyl 3-{[6-(3-fluoro-5-methoxyphenoxy)-1-methyl-1H-benzimidazol-2-yl]methoxylbenzoate

The reaction and post-treatment were carried out according to Example (28d) using [6-(3-fluoro-5-methoxyphenoxy)-1-methyl-1H-benzimidazol-2-yl]methanol produced in Example (47c) (0.35 g, 1.2 mmol), methyl 3-hydroxybenzoate (0.19 g, 1.3 mmol), tri-n-butylphosphine (0.58 mL, 2.3 mmol), 1,1'-(azodicarbonyl)dipiperidine (0.58 g, 2.3 mmol) and dichloromethane (4 mL) to obtain the desired compound (0.31 g, yield: 60%) as a white solid.

¹H-NMR (CDCl₃, 400 MHz) δ: 3.75 (3H, s), 3.84 (3H, s), 3.93 (3H, s), 5.41 (2H, s), 6.27 (1H, ddd, J = 2.0, 2.2, 10.0 Hz), 6.32-6.37 (2H, m), 6.98-7.09 (2H, m), 7.30 (1H, dd, J = 2.7, 8.2 Hz), 7.38 (1H, t, J = 7.8 Hz), 7.69 (1H, dt, J = 1.3, 7.5 Hz), 7.73 (1H, dd, J = 1.4, 2.5 Hz), 7.75 (1H, dd, J = 1.2, 8.2 Hz).

MS (FAB) m/z: 437 (M+H)⁺.

### (47e) 3-{[6-(3-Fluoro-5-methoxyphenoxy)-1-methyl-1H-benzimidazol-2-yl]methoxy}benzoic acid

The reaction and post-treatment were carried out according to Example (28e) using methyl 3-{[6-(3-fluoro-5-methoxyphenoxy)-1-methyl-1H-benzimidazol-2-yl]methoxy}benzoate produced in Example (47d) (0.31 g, 0.70 mmol), a 1 N sodium hydroxide aqueous solution (1.1 mL, 1.1 mmol) and 1,4-dioxane (1.0 mL) to obtain the desired compound (0.20 g, yield: 67%) as a white solid.

¹H-NMR (DMSO-d₆, 400 MHz) δ: 3.73 (3H, s), 3.84 (3H, s), 5.47 (2H, s), 6.38 (1H, s), 6.33 (1H, dt, J = 2.3, 10.3 Hz), 6.56 (1H, dt, J = 2.4, 11.0 Hz), 6.98 (1H, dd, J = 2.4, 8.6 Hz), 7.37-7.48 (3H, m), 7.58 (1H, d, J = 7.4 Hz), 7.64 (1H, s), 7.69 (1H, d, J = 8.6 Hz), 13.03 (1H, br s).

MS (FAB) m/z: 423 (M+H)⁺.

Anal. calcd for C₂₃H₁₉FN₂O₅+0.20H₂O: C, 64.85; H, 4.59; F, 4.46; N, 6.58. Found C, 64.77; H, 4.50; F, 4.58; N, 6.65.

### (Example 48) 3-{[6-(2-Fluoro-4-methylphenoxy)-1-methyl-1H-benzimidazol-2-yl]methoxy}benzoic acid (Compound No. 1-182)

### (48a) tert-Butyl [5-(2-fluoro-4-methylphenoxy)-2-nitrophenyl]methylcarbamate

The synthesis was carried out in the same manner as in Example (28a) using 2-fluoro-4-methylphenol (5.0 g, 40 mmol), tert-butyl (5-chloro-2-nitrophenyl)methylcarbamate (10 g, 36 mmol), sodium hydride (> 56% in oil, 1.6 g, 40 mmol) and N,N-dimethylformamide (50 mL). The resulting yellow oil was directly used for the next reaction.

### (48b) tert-Butyl [2-amino-5-(2-fluoro-4-methylphenoxy)phenyl]methylcarbamate

The synthesis was carried out in the same manner as in Example (28b) using tert-butyl [5-(2-fluoro-4-methylphenoxy)-2-nitrophenyl]methylcarbamate produced in Example (48a) (14 g, 36 mmol), iron powder (10 g, 180 mmol), ammonium chloride (0.96 g, 18 mmol), ethanol (30 mL) and water (15 mL). The resulting brown oil was directly used for the next reaction.

### (48c) [6-(2-Fluoro-4-methylphenoxy)-1-methyl-1H-benzimidazol-2-yl]methanol

The reaction and post-treatment were carried out according to Example (34c) using tert-butyl [2-amino-5-(2-fluoro-4-methylphenoxy)phenyl]methylcarbamate produced in Example (48b) (13 g, 36 mmol), glycolic acid (3.0 g, 47 mmol), a 5 N hydrochloric acid solution (30 mL) and 1,4-dioxane (30 mL) to obtain the desired compound (9.0 g, yield: 87%) as a pale brown solid, which was directly used for the next reaction.

### (48d) Methyl 3-{[6-(2-fluoro-4-methylphenoxy)-1-methyl-1H-benzimidazol-2-yl]methoxy}benzoate

The reaction and post-treatment were carried out according to Example (28d) using [6-(2-fluoro-4-methylphenoxy)-1-methyl-1H-benzimidazol-2-yl]methanol produced in Example (48c) (0.35 g, 1.2 mmol), methyl 3-hydroxybenzoate (0.19 g, 1.3 mmol), tri-n-butylphosphine (0.58 mL, 2.3 mmol), 1,1'-(azodicarbonyl)dipiperidine (0.58 g, 2.3 mmol) and dichloromethane (4 mL) to obtain the desired compound (0.38 g, yield: 78%) as a white solid.

¹H-NMR (CDCl₃, 400 MHz) δ: 2.36 (3H, s), 3.80 (3H, s), 3.92 (3H, s), 5.38 (2H, s), 6.88-7.04 (5H, m), 7.29 (1H, s), 7.37 (1H, t, J = 8.0 Hz), 7.65-7.75 (3H, m).

MS (FAB) m/z: 421 (M+H)⁺.

### (48e) 3-{[6-(2-Fluoro-4-methylphenoxy)-1-methyl-1H-benzimidazol-2-yl]methoxy}benzoic acid

The reaction and post-treatment were carried out according to Example (28e) using methyl 3-{[6-(2-fluoro-4-methylphenoxy)-1-methyl-1H-benzimidazol-2-yl]methoxy}benzoate produced in Example (48d) (0.38 g, 0.90 mmol), a 1 N sodium hydroxide aqueous solution (1.4 mL, 1.4 mmol) and 1,4-dioxane (1.5 mL) to obtain the desired compound (0.41 g, yield: 100%) as a white solid.

¹H-NMR (DMSO-d₆, 400 MHz) δ: 2.31 (3H, s), 3.79 (3H, s), 5.45 (2H, s), 6.90 (1H, dd, J = 2.4, 9.0 Hz), 6.99-7.02 (2H, m), 7.21 (1H, t, J = 6.7 Hz), 7.21 (1H, s), 7.35-7.40 (1H, m), 7.45 (1H, t, J = 7.8 Hz), 7.55-7.59 (1 H, m), 7.63 (1H, d, J = 8.6 Hz), 7.63 (1H, dd, J = 1.6, 2.4 Hz), 13.03 (1H, br s).

MS (FAB) m/z: 407 (M+H)⁺.

Anal. calcd for C₂₃H₁₉FN₂O₄+0.20H₂O: C, 67.38; H, 4.77; F, 4.63; N, 6.83. Found C, 67.46; H, 4.72; F, 4.75; N, 6.89.

### (Example 49) 3-{[1-Methyl-6-(4-methylphenoxy)-1H-benzimidazol-2-yl]methoxy}benzoic acid (Compound No. 1-168)

### (49a) tert-Butyl methyl[5-(4-methylphenoxy)-2-nitrophenyl]carbamate

A crude product of the desired title compound was obtained as a yellow powder according to the method described in Example (28a) using 4-methylphenol (4.53 g, 41.9 mmol), tert-butyl (5-chloro-2-nitrophenyl)methylcarbamate (10.0 g, 34.9 mmol) and sodium hydride (63%, 1.59 g, 41.9 mmol). The crude product was directly used for the next reaction.

### (49b) tert-Butyl [2-amino-5-(4-methylphenoxy)phenyl]methylcarbamate

A crude product of the desired title compound was obtained as a brown powder according to the method described in Example (28b) using tert-butyl methyl[5-(4-methylphenoxy)-2-nitrophenyl]carbamate obtained in Example (49a) (12.5 g, 34.9 mmol), iron powder (9.74 g, 174 mmol) and ammonium chloride (0.933 g, 17.4 mmol). The crude product was directly used for the next reaction.

### (49c) [1-Methyl-6-(4-methylphenoxy)-1H-benzimidazol-2-yl]methanol

The desired title compound (8.31 g, yield: 89%) was obtained as a brown powder according to the method described in Example (28c) using tert-butyl [2-amino-5-(4-methylphenoxy)phenyl]methylcarbamate obtained in Example (49b) (11.5 g, 34.9 mmol) and glycolic acid (3.98 g, 52.3 mmol).

¹H NMR (CDCl₃, 400 MHz) δ: 2.34 (3H, s), 3.74 (3H, s), 4.86 (2H, s), 6.87-6.92 (3H, m), 6.95 (1H, dd, J = 2. 3, 8.6 Hz), 7.14 (2H, d, J = 7.8 Hz), 7.59 (1H, d, J = 8.6 Hz).

### (49d) Methyl 3-{[1-methyl-6-(4-methylphenoxy)-1H-benzimidazol-2-yl]methoxy}benzoate

The desired title compound (9.40 g, yield: 83%) was obtained as a white brown powder according to the method described in Example (28d) using [1-methyl-6-(4-methylphenoxy)-1H-benzimidazol-2-yl]methanol obtained in Example (49c) (7.50 g, 28.0 mmol), methyl 3-hydroxybenzoate (5.10 g, 33.5 mmol), tri-n-butylphosphine (8.38 mL, 33.5 mmol) and 1,1'-(azodicarbonyl)dipiperidine (8.46 g, 33.5 mmol).

¹H NMR (CDCl₃, 500 MHz) δ: 2.34 (3H, s), 3.80 (3H, s), 3.92 (3H, s), 5.39 (2H, s), 6.89-6.97 (3H, m), 7.01 (1H, dd, J = 2.4, 8.8 Hz), 7.14 (2H, d, J = 8.3 Hz), 7.27-7.31 (1H, m), 7.37 (1H, t, J = 8.1 Hz), 7.66-7.74 (3H, m).

### (49e) 3-{[1-Methyl-6-(4-methylphenoxy)-1H-benzimidazol-2-yl]methoxy}benzoic acid

The desired title compound (5.91 g, yield: 68%) was obtained as a white powder according to the method described in Example (28e) using methyl 3-{[1-methyl-6-(4-methylphenoxy)-1H-benzimidazol-2-yl]methoxy}benzoate (9.00 g, 22.4 mmol) obtained in Example (49d) and a 1 N sodium hydroxide aqueous solution (33.6 mL, 33.6 mmol).

¹H NMR (DMSO-d₆, 400 MHz): δ 2.28 (3H, s), 3.80 (3H, s), 5.46 (2H, s), 6.87-6.94 (3H, m), 7.17 (2H, d, J = 9.0 Hz), 7.26 (1H, d, J = 2.3 Hz), 7.36-7.41 (1H, m), 7.45 (1H, t, J = 8.0 Hz), 7.58 (1H, d, J = 7.8 Hz), 7.62-7.67 (2H, m), 13.05 (1H, s).

MS (FAB) m/z: 389 (M+H)⁺.

### (Example 50) 3-{[6-(5-Fluoro-2-methylphenoxy)-1-methyl-1H-benzimidazol-2-yl]methoxy}benzoic acid (Compound No. 1-185)

### (50a) tert-Butyl [5-(5-fluoro-2-methylphenoxy)-2-nitrophenyl]methylcarbamate

A crude product of the desired title compound was obtained as a brown oil according to the method described in Example (28a) using 5-fluoro-2-methylphenol (5.28 g, 41.9 mmol), tert-butyl (5-chloro-2-nitrophenyl)methylcarbamate (10.0 g, 34.9 mmol) and sodium hydride (63%, 1.59 g, 41.9 mmol). The crude product was directly used for the next reaction.

### (50b) tert-Butyl [2-amino-5-(5-fluoro-2-methylphenoxy)phenyl]methylcarbamate

A crude product of the desired title compound was obtained as a brown oil according to the method described in Example (28b) using tert-butyl [5-(5-fluoro-2-methylphenoxy)-2-nitrophenyl]methylcarbamate obtained in Example (50a) (13.1 g, 34.9 mmol), iron powder (9.74 g, 174 mmol) and ammonium chloride (0.933 g, 17.4 mmol). The crude product was directly used for the next reaction.

### (50c) [6-(5-Fluoro-2-methylphenoxy)-1-methyl-1H-benzimidazol-2-yl]methanol

The desired title compound (8.52 g, yield: 85%) was obtained as a brown powder according to the method described in Example (28c) using tert-butyl [2-amino-5-(5-fluoro-2-methylphenoxy)phenyl]methylcarbamate obtained in Example (50b) (12.1 g, 34.9 mmol) and glycolic acid (3.98 g, 52.3 mmol).

¹H NMR (CDCl₃, 500 MHz) δ: 2.26 (3H, s), 3.77 (3H, s), 4.88 (2H, s), 6.50 (1H, dd, J = 2.4, 10.3 Hz), 6.73 (1H, dt, J = 2.4, 8.3 Hz), 6.87 (1H, d, J = 2.4 Hz), 6.93 (1H, dd, J = 2.4, 8.8 Hz), 7.16-7.20 (1H, m), 7.63 (1H, d, J = 8.8 Hz).

### (50d) Methyl 3-{[6-(5-fluoro-2-methylphenoxy)-1-methyl-1H-benzimidazol-2-yl]methoxy}benzoate

The desired title compound (7.15 g, yield: 65%) was obtained as a white powder according to the method described in Example (28d) using [6-(5-fluoro-2-methylphenoxy)-1-methyl-1H-benzimidazol-2-yl]methanol obtained in Example (50c) (7.50 g, 26.2 mmol), methyl 3-hydroxybenzoate (4.78 g, 31.4 mmol), tri-n-butylphosphine (7.85 mL, 31.4 mmol) and 1,1'-(azodicarbonyl)dipiperidine (7.93 g, 31.4 mmol).

¹H NMR (CDCl₃, 400 MHz) δ: 2.27 (3H, s), 3.82 (3H, s), 3.92 (3H, s), 5.40 (2H, s), 6.53 (1H, dd, J = 2.7, 9.8 Hz), 6.74 4 (1H, dt, J = 2.7, 8.2 Hz), 6.91 (1H, d, J = 2.0 Hz), 6.98 (1H, dd, J = 2.0, 8.6 Hz), 7.17-7.22 (1H, m), 7.27-7.32 (1H, m), 7.38 (1H, t, J = 8.2 Hz), 7.67-7.76 (3H, m).

### (50e) 3-{[6-(5-Fluoro-2-methylphenoxy)-1-methyl-1H-benzimidazol-2-yl]methoxy}benzoic acid

The desired title compound (6.29 g, yield: 93%) was obtained as a white powder according to the method described in Example (28e) using methyl 3-{[6-(5-fluoro-2-methylphenoxy)-1-methyl-1H-benzimidazol-2-yl]methoxy}benzoate obtained in Example (50d) (7.00 g, 16.7 mmol) and a 1 N sodium hydroxide aqueous solution (25.0 mL, 25.0 mmol).

¹H NMR (DMSO-d₆, 400 MHz) δ: 2.24 4 (3H, s), 3.82 (3H, s), 5.47 (2H, s), 6.57 (1H, dd, J = 2.5, 10.4 Hz), 6.85-6.95 (2H, m), 7.29 (1H, d, J = 2.3 Hz), 7.31-7.41 (2H, m), 7.46 (1H, t, J = 7.8 Hz), 7.58 (1H, d, J = 7.8 Hz), 7.63-7.66 (1H, m), 7.67 (1H, d, J = 8.6 Hz), 13.04 (1H, s).

MS (FAB) m/z: 407 (M+H)⁺.

### (Example 51) 3-{[6-(2-Fluoro-5-methoxyphenoxy)-1-methyl-1H-benzimidazol-2-yl]methoxy}benzoic acid (Compound No. 1-191)

### (51a) tert-Butyl [5-(2-fluoro-5-methoxyphenoxy)-2-nitrophenyl]methylcarbamate

The reaction and post-treatment were carried out according to Example (27a) using known [Can. J. Chem., 1988, Vol. 66, p. 1479-1482] 2-fluoro-5-methoxyphenol (4.6 g, 32 mmol), tert-butyl (5-chloro-2-nitrophenyl)methylcarbamate (7 g, 36 mmol), sodium hydride (> 56% in oil, 1.3 g, 32 mmol) and N,N-dimethylformamide (60 mL) to obtain the desired compound (5.5 g, yield: 57%) as a yellow oil.

¹H-NMR (CDCl₃, 400 MHz) δ: 1.32 (6H, s), 1.50 (3H, s), 3.26 (3H, s), 3.80 (3H, s), 6.67-6.92 (4H, m), 7.15 (1H, t, J = 9.4 Hz), 7.94 (1H, d, J = 8.6 Hz).

### (51b) tert-Butyl [2-amino-5-(2-fluoro-5-methoxyphenoxy)phenyl]methylcarbamate

The synthesis was carried out in the same manner as in Example (28b) using tert-butyl [5-(2-fluoro-5-methoxyphenoxy)-2-nitrophenyl]methylcarbamate produced in Example (51a) (5.5 g, 14 mmol), iron powder (6.5 g, 120 mmol), ammonium chloride (0.65 g, 12 mmol), ethanol (30 mL) and water (15 mL). The resulting oil was directly used for the next reaction.

### (51c) [6-(2-Fluoro-5-methoxyphenoxy)-1-methyl-1H-benzimidazol-2-yl]methanol

The reaction and post-treatment were carried out according to Example (34c) using tert-butyl [2-amino-5-(2-fluoro-5-methoxyphenoxy)phenyl]methylcarbamate produced in Example (51b) (5.0 g, 14 mmol), glycolic acid (1.4 g, 18 mmol), a 5 N hydrochloric acid solution (30 mL) and 1,4-dioxane (30 mL) to obtain the desired compound (2.3 g, yield: 64%) as a pale brown solid.

¹H-NMR (CDCl₃, 400 MHz) δ: 3.72 (3H, s), 3.75 (3H, s), 4.90 (2H, d, J = 5.5 Hz), 6.54 (1H, dd, J = 2.9, 6.8 Hz), 6.57-6.63 (1H, m), 6.95 (1H, d, J = 2.4 Hz), 7.00 (1H, dd, J = 2.2, 8.8 Hz), 7.10 (1H, dd, J = 9.0, 10.6 Hz), 7.66 (1H, d, J = 8.6 Hz).

### (51d) Methyl 3-{[6-(2-fluoro-5-methoxyphenoxy)-1-methyl-1H-benzimidazol-2-yl]methoxy}benzoate

The reaction and post-treatment were carried out according to Example (28d) using [6-(2-fluoro-5-methoxyphenoxy)-1-methyl-1H-benzimidazol-2-yl]methanol produced in Example (51c) (2.7 g, 8.8 mmol), methyl 3-hydroxybenzoate (1.3 g, 8.8 mmol), tri-n-butylphosphine (4.4 mL, 18 mmol), 1,1'-(azodicarbonyl)dipiperidine (4.5 g, 18 mmol) and dichloromethane (4 mL) to obtain the desired compound (3.7 g, yield: 95%) as a white solid.

¹H-NMR (CDCl₃, 400 MHz) δ: 3.72 (3H, s), 3.82 (3H, s), 3.92 (3H, s), 5.39 (2H, s), 6.55 (1H, dd, J = 3.1, 6.7 Hz), 6.61 (1H, dt, J = 3.3, 9.0 Hz), 6.97 (1H, d, J = 2.4 Hz), 7.03 (1H, dd, J = 2.4, 8.6 Hz), 7.10 (1H, dd, J = 9.0, 10.2 Hz), 7.29 (1H, dd, J = 2.5, 9.2 Hz), 7.37 (1H, t, J = 8.0 Hz), 7.67-7.70 (1 H, m), 7.70-7.74 (2H, m).

MS (FAB) m/z: 437 (M+H)⁺.

### (51e) 3-{[6-(2-Fluoro-5-methoxyphenoxy)-1-methyl-1H-benzimidazol-2-yl]methoxy}benzoic acid

The reaction and post-treatment were carried out according to Example (28e) using methyl 3-{[6-(2-fluoro-5-methoxyphenoxy)-1-methyl-1H-benzimidazol-2-yl]methoxy}benzoate produced in Example (51d) (3.7 g, 8.4 mmol), a 1 N sodium hydroxide aqueous solution (13 mL, 13 mmol) and 1,4-dioxane (10 mL) to obtain the desired compound (2.9 g, yield: 81%) as a white solid.

¹H-NMR (DMSO-d₆, 400 MHz) δ: 3.68 (4H, s), 3.81 (3H, s), 5.43 (2H, s), 6.61 (1H, dd, J = 2.9, 6.8 Hz), 6.73 (1H, dt, J = 3.3, 9.1 Hz), 6.94 (1H, dd, J = 2.5, 8.8 Hz), 7.24-7.40 (4H, m), 7.54 (1 H, d, J = 7.4 Hz), 7.61 (1 H, br. s.), 7.65 (1 H, d, J = 9.0 Hz).

MS (FAB) m/z: 423 (M+H)⁺.

Anal. calcd for C₂₃H₁₉FN₂O₅+0.20H₂O: C, 64.85; H, 4.59; F, 4.46; N, 6.58. Found C, 64.74; H, 4.38; F, 4.63; N, 6.51.

### (Example 52) 3-({6-[3-(Dimethylamino)phenoxy]-1-methyl-1H-benzimidazol-2-yl}methoxy)benzoic acid (Compound No. 1-177)

### (52a) Methyl 3-({6-[3-(dimethylamino)phenoxy]-1-methyl-1H-benzimidazol-2-yl}methoxy)benzoate

The reaction and post-treatment were carried out according to Example (28d) using known [US6432993 B1] {6-[3-(dimethylamino)phenoxy]-1-methyl-1H-benzimidazol-2-yl}methanol (0.30 g, 1.0 mmol), methyl 3-hydroxybenzoate (0.15 g, 1.0 mmol), tri-n-butylphosphine (0.50 mL, 2.0 mmol), 1,1'-(azodicarbonyl)dipiperidine (0.51 g, 2.0 mmol) and dichloromethane (3.0 mL) to obtain the desired compound (0.37 g, yield: 84%) as a colorless oil.

¹H-NMR (CDCl₃, 400 MHz) δ: 2.93 (6H, s), 3.81 (3H, s), 3.92 (3H, s), 5.39 (2H, s), 6.27-6.35 (1H, m), 6.43 (1H, t, J = 2.4 Hz), 6.4-6.51 (1 H, m), 7.00 (1H, d, J = 1.6 Hz), 7.04 (1H, dd, J = 2.4, 8.6 Hz), 7.17 (1H, t, J = 8.2 Hz), 7.29 (1H, dd, J = 3.3, 8.8 Hz), 7.37 (1H, t, J = 7.8 Hz), 7.66-7.75 (3 H, m).

MS (FAB) m/z: 432 (M+H)⁺.

### (52b) 3-({6-[3-(Dimethylamino)phenoxy]-1-methyl-1H-benzimidazol-2-yl}methoxy)benzoic acid

The reaction and post-treatment were carried out according to Example (28e) using methyl 3-({6-[3-(dimethylamino)phenoxy]-1-methyl-1H-benzimidazol-2-yl}methoxy)benzoate produced in Example (52a) (0.44 g, 1.0 mmol), a 1 N sodium hydroxide aqueous solution (1.5 mL, 1.5 mmol) and 1,4-dioxane (1.0 mL) to obtain the desired compound (0.33 g, yield: 79%) as a white solid.

¹H-NMR (DMSO-d₆, 400 MHz) δ: 2.87 (6H, s), 3.81 (3 H, s), 5.46 (2H, s), 6.18 (1H, dd, J = 2.4, 7.4 Hz), 6.36 (1H, t, J = 2.4 Hz), 6.46 (1H, dd, J = 2.4, 7.8 Hz), 6.92 (1H, dd, J = 2.4, 9.0 Hz), 7.12 (1H, t, J = 8.2 Hz), 7.27 (1H, d, J = 2.0 Hz), 7.39 (1H, dd, J = 1.2, 2.7 Hz), 7.45 (1H, t, J = 8.0 Hz), 7.56-7.60 (1H, m), 7.62-7.65 (2H, m), 13.03 (1H, br s).

MS (FAB) m/z: 418 (M+H)⁺.

Anal. calcd for C₂₄H₂₃N₃O₄+0.20H₂O: C, 68.46; H, 5.60; N, 9.98. Found C, 68.33; H, 5.52; N, 9.98.

### (Example 53) 3-{[6-(3-Methoxy-5-methylphenoxy)-1-methyl-1H-benzimidazol-2-yl]methoxy}benzoic acid (Compound No. 1-206)

### (53a) tert-Butyl [5-(3-methoxy-5-methylphenoxy)-2-nitrophenyl]methylcarbamate

The synthesis was carried out in the same manner as in Example (28a) using 3-methoxy-5-methylphenol (4.9 g, 35 mmol), tert-butyl (5-chloro-2-nitrophenyl)methylcarbamate (8.1 g, 1.7 mmol), sodium hydride (> 56% in oil, 1.7 g, 42 mmol) and N,N-dimethylformamide (50 mL). The resulting yellow oil was directly used for the next reaction.

### (53b) tert-Butyl [2-amino-5-(3-methoxy-5-methylphenoxy)phenyl]methylcarbamate

The synthesis was carried out in the same manner as in Example (28b) using tert-butyl [5-(3-methoxy-5-methylphenoxy)-2-nitrophenyl]methylcarbamate produced in Example (53a) (11 g, 28 mmol), iron powder (7.6 g, 140 mmol), ammonium chloride (0.75 g, 14 mmol), ethanol (40 mL) and water (10 mL). The resulting oil was directly used for the next reaction.

### (53c) [6-(3-Methoxy-5-methylphenoxy)-1-methyl-1H-benzimidazol-2-yl]methanol

The synthesis was carried out in the same manner as in Example (34c) using tert-butyl [2-amino-5-(3-methoxy-5-methylphenoxy)phenyl]methylcarbamate produced in Example (53b) (10 g, 28 mmol), glycolic acid (2.8 g, 37 mmol), a 5 N hydrochloric acid solution (20 mL) and 1,4-dioxane (20 mL). The resulting pale brown solid was directly used for the next reaction.

### (53d) Methyl 3-{[6-(3-methoxy-5-methylphenoxy)-1-methyl-1H-benzimidazol-2-yl]methoxy}benzoate

The synthesis was carried out in the same manner as in Example (28d) using [6-(3-methoxy-5-methylphenoxy)-1-methyl-1H-benzimidazol-2-yl]methanol produced in Example (53c) (5.1 g, 17 mmol), methyl 3-hydroxybenzoate (2.6 g, 17 mmol), tri-n-butylphosphine (8.6 mL, 34 mmol), 1,1'-(azodicarbonyl)dipiperidine (8.6 g, 34 mmol) and dichloromethane (50 mL). The resulting pale brown solid was directly used for the next reaction.

### (53e) 3-{[6-(3-Methoxy-5-methylphenoxy)-1-methyl-1H-benzimidazol-2-yl]methoxy}benzoic acid

The reaction and post-treatment were carried out according to Example (28e) using methyl 3-{[6-(3-methoxy-5-methylphenoxy)-1-methyl-1H-benzimidazol-2-yl]methoxy}benzoate produced in Example (53d) (7.4 g, 17 mmol), a 1 N sodium hydroxide aqueous solution (26 mL, 26 mmol) and 1,4-dioxane (25 mL) to obtain the desired compound (5.2 g, yield: 70%) as a white solid.

¹H-NMR (DMSO-d₆, 400 MHz) δ: 2.22 (3H, s), 3.70 (3H, s), 3.82 (3H, s), 5.47 (2H, s), 6.32 (1H, s), 6.36 (1H, t, J = 2.4 Hz), 6.50 (1H, s), 6.93 (1H, dd, J = 2.4, 8.6 Hz), 7.32 (1H, d, J = 2.4 Hz), 7.36-7.42 (1H, m), 7.45 (1H, t, J = 7.8 Hz), 7.55-7.61 (1H, m), 7.66 (2H, d, J = 9.0 Hz), 13.04 (1H, s).

MS (FAB) m/z: 419 (M+H)⁺.

Anal. calcd for C₂₄H₂₂N₂O₅: C, 68.89; H, 5.30; N, 6.69. Found C, 68.64; H, 5.26; N, 6.59.

### (Example 54) 3-{[6-(3-Methoxy-4-methylphenoxy)-1-methyl-1H-benzimidazol-2-yl]methoxy}benzoic acid (Compound No. 1-204)

### (54a) tert-Butyl [5-(3-methoxy-4-methylphenoxy)-2-nitrophenyl]methylcarbamate

A crude product of the desired title compound was obtained as a brown oil according to the method described in Example (28a) using 5-fluoro-2-methylphenol (5.78 g, 41.9 mmol), tert-butyl (5-chloro-2-nitrophenyl)methylcarbamate (10.0 g, 34.9 mmol) and sodium hydride (63%, 1.59 g, 41.9 mmol). The crude product was directly used for the next reaction.

### (54b) tert-Butyl [2-amino-5-(3-methoxy-4-methylphenoxy)phenyl]methylcarbamate

A crude product of the desired title compound was obtained as a brown oil according to the method described in Example (28b) using tert-butyl [5-(3-methoxy-4-methylphenoxy)-2-nitrophenyl]methylcarbamate obtained in Example (54a) (13.6 g, 34.9 mmol), iron powder (9.74 g, 174 mmol) and ammonium chloride (0.933 g, 17.4 mmol). The crude product was directly used for the next reaction.

### (54c) [6-(3-Methoxy-4-methylphenoxy)-1-methyl-1H-benzimidazol-2-yl]methanol

The desired title compound (8.93 g, yield: 86%) was obtained as a brown powder according to the method described in Example (28c) using tert-butyl [2-amino-5-(3-methoxy-4-methylphenoxy)phenyl]methylcarbamate obtained in Example (54b) (12.5 g, 34.9 mmol) and glycolic acid (3.98 g, 52.3 mmol).

¹H NMR (CDCl₃, 400 MHz) δ: 2.19 (3H, s), 3.75 (3H, s), 3.77 (3H, s), 4.87 (2H, s), 5.10 (1H, s), 6.45 (1H, dd, J = 2.3, 8.2 Hz), 6.57 (1H, d, J = 2.3 Hz), 6.90 (1H, d, J = 2.0 Hz), 6.97 (1H, dd, J = 2.0, 8.6 Hz), 7.03-7.07 (1H, m), 7.60 (1H, d, J = 8.6 Hz).

### (54d) Methyl 3-{[6-(3-methoxy-4-methylphenoxy)-1-methyl-1H-benzimidazol-2-yl]methoxy}benzoate

The desired title compound (8.26 g, yield: 71%) was obtained as a white powder according to the method described in Example (28d) using [6-(3-methoxy-4-methylphenoxy)-1-methyl-1H-benzimidazol-2-yl]methanol obtained in Example (54c) (8.00 g, 26.8 mmol), methyl 3-hydroxybenzoate (4.90 g, 32.2 mmol), tri-n-butylphosphine (8.04 mL, 32.2 mmol) and 1,1'-(azodicarbonyl)dipiperidine (8.12 g, 32.2 mmol).

¹H NMR (CDCl₃, 500 MHz) δ: 2.19 (3H, s), 3.77 (3H, s), 3.81 (3H, s), 3.92 (3H, s), 5.39 (2H, s), 6.47 (1H, dd, J = 2.4, 8.3 Hz), 6.58 (1H, d, J = 2.4 Hz), 6.96 (1H, d, J = 2.4 Hz), 7.02 (1H, dd, J = 2.0, 8.8 Hz), 7.05 (1H, d, J = 8.3 Hz), 7.27-7.31 (1H, m), 7.37 (1H, t, J = 7.8 Hz), 7.67-7.73 (3H, m).

### (54e) 3-{[6-(3-Methoxy-4-methylphenoxy)-1-methyl-1H-benzimidazol-2-yl]methoxy}benzoic acid

The desired title compound (7.44 g, yield: 96%) was obtained as a white powder according to the method described in Example (28e) using methyl 3-{[6-(3-methoxy-4-methylphenoxy)-1-methyl-1H-benzimidazol-2-yl]methoxy}benzoate obtained in Example (54d) (8.00 g, 18.5 mmol) and a 1 N sodium hydroxide aqueous solution (27.8 mL, 27.8 mmol).

¹H NMR (DMSO-d₆, 400 MHz) δ: 2.11 (3H, s), 3.74 (3H, s), 3.81 (3H, s), 5.46 (2H, s), 6.40 (1H, dd, J = 2.3, 7.8 Hz), 6.67 (1H, d, J = 2.3 Hz), 6.94 (1H, dd, J = 2.3, 8.6 Hz), 7.08 (1H, d, J = 8.2 Hz), 7.27 (1H, d, J = 2.3 Hz), 7.36-7.42 (1H, m), 7.46 (1H, t, J = 8.0 Hz), 7.56-7.61 (1H, m), 7.65 (1H, d, J = 8.6 Hz), 7.63-7.65 (1H, m).

MS (EI) m/z: 418 M⁺.

### (Example 55) 3-{[6-(3,4-Dimethylphenoxy)-1-methyl-1H-benzimidazol-2-yl]methoxy}benzoic acid (Compound No. 1-198)

### (55a) tert-Butyl [5-(3,4-dimethylphenoxy)-2-nitrophenyl]methylcarbamate

A crude product of the desired title compound was obtained as a brown powder according to the method described in Example (28a) using 3,4-dimethylphenol (5.11 g, 41.9 mmol), tert-butyl (5-chloro-2-nitrophenyl)methylcarbamate (10.0 g, 34.9 mmol) and sodium hydride (63%, 1.59 g, 41.9 mmol). The crude product was directly used for the next reaction.

### (55b) tert-Butyl [2-amino-5-(3,4-dimethylphenoxy)phenyl]methylcarbamate

A crude product of the desired title compound was obtained as a brown oil according to the method described in Example (28b) using tert-butyl [5-(3,4-dimethylphenoxy)-2-nitrophenyl]methylcarbamate obtained in Example (55a) (13.0 g, 34.9 mmol), iron powder (9.74 g, 174 mmol) and ammonium chloride (0.933 g, 17.4 mmol). The crude product was directly used for the next reaction.

### (55c) [6-(3,4-Dimethylphenoxy)-1-methyl-1H-benzimidazol-2-yl]methanol

The desired title compound (7.98 g, yield: 81%) was obtained as a brown powder according to the method described in Example (28c) using tert-butyl [2-amino-5-(3,4-dimethylphenoxy)phenyl]methylcarbamate obtained in Example (55b) (11.9 g, 34.9 mmol) and glycolic acid (3.98 g, 52.3 mmol).

¹H NMR (CDCl₃, 400 MHz) δ: 2.23 (3H, s), 2.24 (3H, s), 3.74 (3H, s), 4.86 (2H, s), 4.94 (1H, s), 6.74 (1H, dd, J - 8.2, 2.3 Hz), 6.81 (1H, d, J = 2.3 Hz), 6.89 (1H, d, J - 2. 3 Hz), 6.96 (1H, dd, J = 8.6, 2.3 Hz), 7.08 (1H, d, J = 8.2 Hz), 7.60 (1H, d, J = 8.6 Hz).

### (55d) Methyl 3-{[6-(3,4-dimethylphenoxy)-1-methyl-1H-benzimidazol-2-yl]methoxy}benzoate

The desired title compound (8.36 g, yield: 81%) was obtained as a white powder according to the method described in Example (28d) using [6-(3,4-dimethylphenoxy)-1-methyl-1H-benzimidazol-2-yl]methanol obtained in Example (55c) (7.00 g, 24.8 mmol), methyl 3-hydroxybenzoate (4.53 g, 29.8 mmol), tri-n-butylphosphine (7.43 mL, 29.8 mmol) and 1,1'-(azodicarbonyl)dipiperidine (7.51 g, 29.8 mmol).

¹H NMR (CDCl₃, 500 MHz) δ: 2.23 (3H, s), 2.24 (3H, s), 3.80 (3H, s), 3.92 (3H, s), 5.38 (2H, s), 6.75 (1H, dd, J = 2.4, 8.3 Hz), 6.81 (1H, d, J = 2.4 Hz), 6.95 (1H, d, J = 2.4 Hz), 7.00 (1H, dd, J = 2.4, 8.8 Hz), 7.08 (1H, d, J = 8.3 Hz), 7.27-7.31 (1H, m), 7.37 (1H, t, J = 8.3 Hz), 7.66-7.73 (3H, m).

### (55e) 3-{[6-(3,4-Dimethylphenoxy)-1-methyl-1H-benzimidazol-2-yl]methoxy}benzoic acid

The desired title compound (6.21 g, yield: 80%) was obtained as a white powder according to the method described in Example (28e) using methyl 3-{[6-(3,4-dimethylphenoxy)-1-methyl-1H-benzimidazol-2-yl]methoxy}benzoate obtained in Example (55d) (8.00 g, 19.2 mmol) and a 1 N sodium hydroxide aqueous solution (28.8 mL, 28.8 mmol).

¹H NMR (DMSO-d₆, 400 MHz) δ: 2.18 (3H, s), 2.18 (3H, s), 3.80 (3H, s), 5.46 (2H, s), 6.71 (1 H, dd, J = 2.7, 7.8 Hz), 6.80 (1H, d, J = 2.7 Hz), 6.90 (1H, dd, J = 2.2, 8.8 Hz), 7.11 (1H, d, J = 8.2 Hz), 7.25 (1H, d, J = 2.3 Hz), 7.36-7.41 (1H, m), 7.45 (1H, t, J = 7.8 Hz), 7.56-7.60 (1H, m), 7.61-7.65 (2H, m).

MS (FAB) m/z: 402 M⁺.

### (Example 56) 3-{[6-(4-Ethylphenoxy)-1-methyl-1H-benzimidazol-2-yl]methoxy}benzoic acid (Compound No. 1-171)

### (56a) tert-Butyl [5-(4-ethylphenoxy)-2-nitrophenyl]methylcarbamate

The synthesis was carried out in the same manner as in Example (28a) using 4-ethylphenol (4.0 g, 33 mmol), tert-butyl (5-chloro-2-nitrophenyl)methylcarbamate (7.5 g, 26 mmol), sodium hydride (> 56% in oil, 2.0 g, 51 mmol) and N,N-dimethylformamide (50 mL). The resulting yellow oil was directly used for the next reaction.

### (56b) tert-Butyl [2-amino-5-(4-ethylphenoxy)phenyl]methylcarbamate

The synthesis was carried out in the same manner as in Example (28b) using tert-butyl [5-(4-ethylphenoxy)-2-nitrophenyl]methylcarbamate produced in Example (56a) (11 g, 26 mmol), iron powder (7.0 g, 130 mmol), ammonium chloride (0.70 g, 13 mmol), ethanol (30 mL) and water (15 mL). The resulting red oil was directly used for the next reaction.

### (56c) [6-(4-Ethylphenoxy)-1-methyl-1H-benzimidazol-2-yl]methanol

The reaction and post-treatment were carried out according to Example (34c) using tert-butyl [2-amino-5-(4-ethylphenoxy)phenyl]methylcarbamate produced in Example (56b) (9.0 g, 26 mmol), glycolic acid (2.6 g, 34 mmol), a 5 N hydrochloric acid solution (20 mL) and 1,4-dioxane (20 mL) to obtain the desired compound (5.8 g, yield: 78%) as a pale brown solid, which was directly used for the next reaction.

### (56d) Methyl 3-{[6-(4-ethylphenoxy)-1-methyl-1H-benzimidazol-2-yl]methoxy}benzoate

The reaction and post-treatment were carried out according to Example (28d) using [6-(4-ethylphenoxy)-1-methyl-1H-benzimidazol-2-yl]methanol produced in Example (56c) (5.3 g, 19 mmol), methyl 3-hydroxybenzoate (2.8 g, 19 mmol), tri-n-butylphosphine (9.3 mL, 37 mmol), 1,1'-(azodicarbonyl)dipiperidine (9.4 g, 37 mmol) and dichloromethane (50 mL) to obtain the desired compound (5.6 g, yield: 72%) as a pale red solid.

MS (FAB) m/z: 417 (M+H)⁺.

### (56e) 3-{[6-(4-Ethylphenoxy)-1-methyl-1H-benzimidazol-2-yl]methoxy}benzoic acid

The reaction and post-treatment were carried out according to Example (28e) using methyl 3-{[6-(4-ethylphenoxy)-1-methyl-1H-benzimidazol-2-yl]methoxy}benzoate produced in Example (56d) (5.6 g, 14 mmol), a 1 N sodium hydroxide aqueous solution (20 mL, 20 mmol) and 1,4-dioxane (25 mL) to obtain the desired compound (5.1 g, yield: 94%) as a white solid.

¹H-NMR (DMSO-d₆, 400 MHz) δ: 1.17 (3H, t, J = 7.6 Hz), 2.58 (2H, q, J = 7.3 Hz), 3.80 (3H, s), 5.46 (2H, s), 6.87-6.94 (3H, m), 7.19 (2H, d, J = 8.3 Hz), 7.28 (1H, d, J = 2.4 Hz), 7.38 (1H, dd, J = 2.7, 8.1 Hz), 7.45 (1H, t, J = 7.8 Hz), 7.58 (1H, d, J = 7.8 Hz), 7.62-7.67 (2H, m), 13.03 (1H, br s).

MS (FAB) m/z: 403 (M+H)⁺.

Anal. calcd for C₂₄H₂₂N₂O₄+0.20H₂O: C, 70.99; H, 5.56; N, 6.90. Found C, 70.82; H, 5.32; N, 6.88.

### (Example 57) 3-{[6-(2,3-Dihydro-1-benzofuran-6-yloxy)-1-methyl-1H-benzimidazol-2-yl]methoxy}benzoic acid (Compound No. 1-210)

### (57a) tert-Butyl [5-(2,3-dihydro-1-benzofuran-6-yloxy)-2-nitrophenyl]methylcarbamate

The synthesis was carried out in the same manner as in Example (28a) using known [J. Am. Chem. Soc., 1948, Vol. 70, p. 3619] 2,3-dihydro-1-benzofuran-6-ol (2.2 g, 16 mmol), tert-butyl (5-chloro-2-nitrophenyl)methylcarbamate (4.0 g, 14 mmol), sodium hydride (> 56% in oil, 0.66 g, 16 mmol) and N-methylpyrrolidinone (30 mL). The resulting yellow oil was directly used for the next reaction.

### (57b) tert-Butyl [2-amino-5-(2,3-dihydro-1-benzofuran-6-yloxy)phenyl]methylcarbamate

The synthesis was carried out in the same manner as in Example (28b) using tert-butyl [5-(2,3-dihydro-1-benzofuran-6-yloxy)-2-nitrophenyl]methylcarbamate produced in Example (57a) (5.4 g, 14 mmol), iron powder (3.7 g, 70 mmol), ammonium chloride (0.37 g, 7.0 mmol), ethanol (40 mL) and water (20 mL). The resulting oil was directly used for the next reaction.

### (57c) Methyl 3-[2-({2-[(tert-butoxycarbonyl)(methyl)amino]-4-(2,3-dihydro-1-benzofuran-6-yloxy)phenyl}amino)-2-oxoethoxy]benzoate

tert-Butyl [2-amino-5-(2,3-dihydro-1-benzofuran-6-yloxy)phenyl]methylcarbamate produced in Example (57b) (5.0 g, 14 mmol) and [3-(methoxycarbonyl)phenoxy]acetic acid (2.9 g, 14 mmol) were dissolved in dichloromethane (100 mL). 1-Ethyl-3-(3-dimethylaminopropyl)carbodiimide hydrochloride (3.2 g, 17 mmol) was added and the mixture was stirred at room temperature for 2.2 hours. A sodium bicarbonate aqueous solution was added, followed by extraction with dichloromethane twice. Then, the organic layers were washed with brine and dried over anhydrous magnesium sulfate. The solvent was evaporated under reduced pressure, and the resulting solid was directly used for the next reaction.

### (57d) Methyl 3-{[6-(2,3-dihydro-1-benzofuran-6-yloxy)-1-methyl-1H-benzimidazol-2-yl]methoxy}benzoate monohydrochloride

Methyl 3-[2-({2-[(tert-butoxycarbonyl)(methyl)amino]-4-(2,3-dihydro-1-benzofuran-6-yloxy)phenyl}amino)-2-oxoethoxy]benzoate produced in Example (57c) (7.7 g, 14 mmol) was dissolved in a 5 N hydrochloric acid-ethyl acetate solution (50 mL), and the mixture was heated under reflux for two hours. After cooling to room temperature, the precipitated pale red solid was collected by filtration to obtain the desired compound (7.9 g, yield: 100%).

¹H-NMR (DMSO-d₆, 500 MHz) δ: 3.15 (2H, t, J = 8.8 Hz), 3.88 (3H, s), 3.93 (3H, s), 4.57 (2H, t, J = 8.8 Hz), 5.69 (2H, s), 6.47 (2H, s), 7.16 (1H, d, J = 8.8 Hz), 7.21 (1H, d, J = 7.8 Hz), 7.47-7.56 (3H, m), 7.66 (1H, d, J = 8.3 Hz), 7.72 (1H, s), 7.78 (1H, d, J = 9.3 Hz).

MS (FAB) m/z: 431 (M+H)⁺.

### (57e) 3-{[6-(2,3-Dihydro-1-benzofuran-6-yloxy)-1-methyl-1H-benzimidazol-2-yl]methoxy}benzoic acid

The reaction and post-treatment were carried out according to Example (28e) using methyl 3-{[6-(2,3-dihydro-1-benzofuran-6-yloxy)-1-methyl-1H-benzimidazol-2-yl]methoxy}benzoate monohydrochloride produced in Example (57d) (6.5 g, 14 mmol), a 1 N sodium hydroxide aqueous solution (56 mL, 56 mmol) and 1,4-dioxane (60 mL) to obtain the desired compound (4.3 g, yield: 74%) as a white solid.

¹H-NMR (DMSO-d₆, 400 MHz) δ: 3.13 (2H, t, J = 8.4 Hz), 3.81 (3H, s), 4.55 (2H, t, J = 8.6 Hz), 5.46 (2H, s), 6.39-6.44 (2H, m), 6.91 (1H, dd, J = 2.4, 8.6 Hz), 7.16 (1H, d, J = 7.8 Hz), 7.28 (1H, d, J = 2.4 Hz), 7.36-7.40 (1H, m), 7.45 (1H, t, J = 7.8 Hz), 7.58 (1H, dt, J = 1.2, 1.4, 7.6 Hz), 7.62-7.66 (2H, m), 13.04 (1H, br s).

MS (FAB) m/z: 417 (M+H)⁺.

Anal. calcd for C₂₄H₂₀N₂O₅+0.33H₂O: C, 68.24; H, 4.93; N, 6.63. Found C, 68.34; H, 4.84; N, 6.79.

### (Example 58) 3-{[6-(1,3-benzodioxol-5-yloxy)-1-methyl-1H-benzimidazol-2-yl]methoxy}benzoic acid (Compound No. 1-211)

### (58a) tert-Butyl [5-(1,3-benzodioxol-5-yloxy)-2-nitrophenyl]methylcarbamate

The synthesis was carried out in the same manner as in Example (28a) using sesamol (2.9 g, 21 mmol), tert-butyl (5-chloro-2-nitrophenyl)methylcarbamate (5.0 g, 17 mmol), sodium hydride (> 56% in oil, 0.82 g, 21 mmol) and N-methylpyrrolidinone (50 mL). The resulting brown oil was directly used for the next reaction.

### (58b) tert-Butyl [2-amino-5-(1,3-benzodioxol-5-yloxy)phenyl]methylcarbamate

The synthesis was carried out in the same manner as in Example (28b) using tert-butyl [5-(1,3-benzodioxol-5-yloxy)-2-nitrophenyl]methylcarbamate produced in Example (58a) (6.8 g, 17 mmol), iron powder (4.7 g, 87 mmol), ammonium chloride (0.47 g, 8.7 mmol), ethanol (40 mL) and water (20 mL). The resulting brown oil was directly used for the next reaction.

### (58c) Methyl 3-[2-({4-(1,3-benzodioxol-5-yloxy)-2-[(tert-butoxycarbonyl)(methyl)amino]phenyl}amino)-2-oxoethoxy]benzoate

The reaction and post-treatment were carried out according to Example (57c) using tert-butyl [2-amino-5-(1,3-benzodioxol-5-yloxy)phenyl]methylcarbamate produced in Example (58b) (6.7 g, 17 mmol), [3-(methoxycarbonyl)phenoxy]acetic acid (3.7 g, 17 mmol), 1-ethyl-3-(3-dimethylaminopropyl)carbodiimide hydrochloride (4.0 g, 21 mmol) and dichloromethane (70 mL) to obtain a solid, which was directly used for the next reaction.

### (58d) Methyl 3-{[6-(1,3-benzodioxol-5-yloxy)-1-methyl-1H-benzimidazol-2-yl]methoxy}benzoate monohydrochloride

The reaction and post-treatment were carried out according to Example (57d) using methyl 3-[2-({4-(1,3-benzodioxol-5-yloxy)-2-[(tert-butoxycarbonyl)(methyl)amino]phenyl}amino)-2-oxoethoxy]benzoate produced in Example (58c) (9.6 g, 17 mmol) and a 5 N hydrochloric acid-ethyl acetate solution (50 mL) to obtain the desired compound (7.3 g, yield: 89%) as a white solid.

¹H-NMR (CDCl₃, 400 MHz) δ: 3.11 (3H, br. s.), 3.93 (3H, s), 4.68 (2H, s), 5.99 (2H, s), 6.49 (1H, dd, J = 2.4, 8.2 Hz), 6.58 (1H, d, J = 2.4 Hz), 6.76 (1H, d, J = 8.2 Hz), 6.82 (1H, br s), 6.86-6.93 (1H, m), 7.20 (1H, dd, J = 2.2, 8.0 Hz), 7.42 (1H, t, J = 8.0 Hz), 7.63 (1H, br. s.), 7.74 (1H, d, J = 7.8 Hz).

### (58e) 3-{[6-(1,3-Benzodioxol-5-yloxy)-1-methyl-1H-benzimidazol-2-yl]methoxy}benzoic acid

The reaction and post-treatment were carried out according to Example (28e) using methyl 3-{[6-(1,3-benzodioxol-5-yloxy)-1-methyl-1H-benzimidazol-2-yl]methoxy}benzoate monohydrochloride produced in Example (58d) (7.3 g, 15 mmol), a 2 N sodium hydroxide aqueous solution (31 mL, 62 mmol) and 1,4-dioxane (60 mL) to obtain the desired compound (6.3 g, yield: 98%) as a white solid.

¹H-NMR (DMSO-d₆, 400 MHz) δ: 3.80 (3H, s), 5.46 (2H, s), 6.03 (2H, s), 6.45 (1H, dd, J = 2.5, 8.4 Hz), 6.71 (1H, d, J = 2.4 Hz), 6.86-6.93 (2H, m), 7.23 (1H, d, J = 2.0 Hz), 7.36-7.41 (1H, m), 7.45 (1H, t, J = 7.8 Hz), 7.56-7.60 (1H, m), 7.61-7.66 (2H, m), 13.07 (1H, br s).

MS (FAB) m/z: 419 (M+H)⁺.

Anal. calcd for C₂₃H₁₈N₂O₆+0.25H₂O: C, 65.32; H, 4.41; N, 6.62. Found C, 65.54; H, 4.71; N, 6.65.

### (Example 59) 3-{[6-(4-Chloro-3-fluorophenoxy)-1-methyl-1H-benzimidazol-2-yl]methoxy}-5-fluorobenzoic acid (Compound No. 1-220)

### (59a) Methyl 3-{[6-(4-chloro-3-fluorophenoxy)-1-methyl-1H-benzimidazol-2-yl]methoxy}-5-fluorobenzoate

The desired title compound (362 mg, yield: 81%) was obtained as a white powder according to the method described in Example (28d) using [6-(4-chloro-3-fluorophenoxy)-1-methyl-1H-benzimidazol-2-yl]methanol obtained in Example (9b) (300 mg, 0.978 mmol), methyl 5-fluoro-3-hydroxybenzoate (200 mg, 1.17 mmol), tri-n-butylphosphine (0.366 mL, 1.47 mmol) and 1,1'-(azodicarbonyl)dipiperidine (370 mg, 1.47 mmol).

¹H NMR (CDCl₃, 400 MHz) δ: 3.84 (3H, s), 3.93 (3H, s), 5.40 (2H, s), 6.71-6.76 (1H, m), 6.78 (1H, dd, J = 2.7, 10.2 Hz), 7.00-7.06 (3H, m), 7.31 (1H, dd, J = 8.6, 8.6 Hz), 7.36-7.41 (1H, m), 7.53-7.56 (1H, m), 7.77 (1H, dd, J = 0.6, 8.6 Hz).

### (59b) 3-{[6-(4-Chloro-3-fluorophenoxy)-1-methyl-1H-benzimidazol-2-yl]methoxy}-5-fluorobenzoic acid

The desired title compound (292 mg, yield: 85%) was obtained as a white powder according to the method described in Example (28e) using methyl 3-{[6-(4-chloro-3-fluorophenoxy)-1-methyl-1H-benzimidazol-2-yl]methoxy}-5-fluorobenzoate obtained in Example (59a) (354 mg, 0.772 mmol) and a 1 N sodium hydroxide aqueous solution (1.16 mL, 1.16 mmol).

¹H NMR (DMSO-d₆, 400 MHz) δ: 3.83 (3H, s), 5.52 (2H, s), 6.83 (1H, ddd, J = 1.2, 2.7, 9.0 Hz), 7.01 (1H, dd, J = 2.3, 8.6 Hz), 7.09 (1H, dd, J = 2.7, 10.6 Hz), 7.28-7.33 (1H, m), 7.35 (1H, ddd, J = 2.3, 2.3, 10.6 Hz), 7.45 (1H, d, J = 2.0 Hz), 7.47-7.51 (1H, m), 7.54 (1H, dd, J = 8.6, 8.6 Hz), 7.71 (1H, d, J = 8.6 Hz).

MS (FAB) m/z: 445 (M+H)⁺.

### (Example 60) 3-Fluoro-5-{[6-(3-methoxyphenoxy)-1-methyl-1H-benzimidazol-2-yl]methoxy}benzoic acid (Compound No. 1-218)

### (60a) Methyl 3-fluoro-5-{[6-(3-methoxyphenoxy)-1-methyl-1H-benzimidazol-2-yl]methoxy}benzoate

The desired title compound (387 mg, yield: 84%) was obtained as a white powder according to the method described in Example (28d) using [6-(3-methoxyphenoxy)-1-methyl-1H-benzimidazol-2-yl]methanol obtained in Example (29c) (300 mg, 1.06 mmol), methyl 5-fluoro-3-hydroxybenzoate (197 mg, 1.16 mmol), tri-n-butylphosphine (0.395 mL, 1.58 mmol) and 1,1'-(azodicarbonyl)dipiperidine (399 mg, 1.58 mmol).

¹H NMR (CDCl₃, 400 MHz) δ: 3.78 (3H, s), 3.81 (3H, s), 3.93 (3H, s), 5.39 (2H, s), 6.56-6.60 (2H, m), 6.63-6.67 (1H, m), 7.00-7.07 (3H, m), 7.19-7.26 (1H, m), 7.36-7.40 (1H, m), 7.53-7.56 (1H, m), 7.74 (1H, d, J = 9.0 Hz).

### (60b) 3-Fluoro-5-{[6-(3-methoxyphenoxy)-1-methyl-1H-benzimidazol-2-yl]methoxy}benzoic acid

The desired title compound (303 mg, yield: 83%) was obtained as a white powder according to the method described in Example (28e) using methyl 3-fluoro-5-{[6-(3-methoxyphenoxy)-1-methyl-1H-benzimidazol-2-yl]methoxy}benzoate obtained in Example (60a) (376 mg, 0.862 mmol) and a 1 N sodium hydroxide aqueous solution (1.29 mL, 1.29 mmol).

¹H NMR (DMSO-d₆, 500 MHz) δ: 3.72 (3H, s), 3.82 (3H, s), 5.50 (2H, s), 6.51 (1H, dd, J = 2.2, 8.1 Hz), 6.56 (1H, t, J = 2.2 Hz), 6.68 (1H, dd, J = 2.4, 8.3 Hz), 6.95 (1H, dd, J = 2.4, 8.8 Hz), 7.25 (1H, t, J = 8.3 Hz), 7.28-7.33 (1H, m), 7.33-7.38 (2H, m), 7.48-7.51 (1H, m), 7.67 (1H, d, J = 8.8 Hz).

MS (FAB) m/z: 423 (M+H)⁺.

### (Example 61) 3-Fluoro-5-{[6-(3-fluorophenoxy)-1-methyl-1H-benzimidazol-2-yl]methoxy}benzoic acid (Compound No. 1-212)

### (61a) Methyl 3-fluoro-5-{[6-(3-fluorophenoxy)-1-methyl-1H-benzimidazol-2-yl]methoxy}benzoate

The desired title compound (426 mg, yield: 91%) was obtained as a pink powder according to the method described in Example (28d) using [6-(3-fluorophenoxy)-1-methyl-1H-benzimidazol-2-yl]methanol obtained in Example (7c) (300 mg, 1.10 mmol), methyl 5-fluoro-3-hydroxybenzoate (206 mg, 1.21 mmol), tri-n-butylphosphine (0.413 mL, 1.65 mmol) and 1,1'-(azodicarbonyl)dipiperidine (417 mg, 1.65 mmol).

¹H NMR (CDCl₃, 400 MHz) δ: 3.84 (3H, s), 3.93 (3H, s), 5.40 (2H, s), 6.66-6.72 (1H, m), 6.75-6.81 (2H, m), 7.01-7.06 (3H, m), 7.23-7.30 (1H, m), 7.36-7.40 (1H, m), 7.54-7.56 (1H, m), 7.75-7.78 (1H, m).

### (61b) 3-Fluoro-5-{[6-(3-fluorophenoxy)-1-methyl-1H-benzimidazol-2-yl]methoxy}benzoic acid

The desired title compound (304 mg, yield: 80%) was obtained as a white powder according to the method described in Example (28e) using methyl 3-fluoro-5-{[6-(3-fluorophenoxy)-1-methyl-1H-benzimidazol-2-yl]methoxy}benzoate obtained in Example (61a) (395 mg, 0.931 mmol) and a 1 N sodium hydroxide aqueous solution (1.40 mL, 1.40 mmol).

¹H NMR (DMSO-d₆, 500 MHz) δ: 3.83 (3H, s), 5.52 (2H, s), 6.77-6.85 (2H, m), 6.89-6.95 (1H, m), 6.99 (1H, dd, J = 2.4, 8.8 Hz), 7.28-7.44 (4H, m), 7.49 (1H, s), 7.70 (1H, d, J = 8.3 Hz), 13.37 (1H, s).

MS (FAB) m/z: 411 (M+H)⁺.

### (Example 62) 3-Fluoro-5-{[6-(4-methoxyphenoxy)-1-methyl-1H-benzimidazol-2-yl]methoxy}benzoic acid (Compound No. 1-219)

### (62a) Methyl 3-fluoro-5-{[6-(4-methoxyphenoxy)-1-methyl-1H-benzimidazol-2-yl]methoxy}benzoate

The desired title compound (432 mg, yield: 94%) was obtained as a yellow oil according to the method described in Example (28d) using [6-(4-methoxyphenoxy)-1-methyl-1H-benzimidazol-2-yl]methanol obtained in Example (30c) (300 mg, 1.06 mmol), methyl 5-fluoro-3-hydroxybenzoate (197 mg, 1.16 mmol), tri-n-butylphosphine (0.395 mL, 1.58 mmol) and 1,1'-(azodicarbonyl)dipiperidine (399 mg, 1.58 mmol).

¹H NMR (CDCl₃, 400 MHz) δ: 3.79 (3H, s), 3.82 (3H, s), 3.93 (3H, s), 5.37 (2H, s), 6.87-6.93 (3H, m), 6.97-7.05 (4H, m), 7.35-7.40 (1H, m), 7.52-7.55 (1H, m), 7.70 (1H, d, J = 8.6 Hz).

### (62b) 3-Fluoro-5-{[6-(4-methoxyphenoxy)-1-methyl-1H-benzimidazol-2-yl]methoxy}benzoic acid

The desired title compound (332 mg, yield: 82%) was obtained as a white powder according to the method described in Example (28e) using methyl 3-fluoro-5-{[6-(4-methoxyphenoxy)-1-methyl-1H-benzimidazol-2-yl]methoxy}benzoate obtained in Example (62a) (420 mg, 0.962 mmol) and a 1 N sodium hydroxide aqueous solution (1.44 mL, 1.44 mmol).

¹H NMR (DMSO-d₆, 400 MHz) δ: 3.74 (3H, s), 3.79 (3H, s), 5.49 (2H, s), 6.89 (1H, dd, J = 2.3, 8.6 Hz), 6.92-7.01 (4H, m), 7.20 (1H, d, J = 2.3 Hz), 7.27-7.37 (2H, m), 7.47-7.50 (1H, m), 7.63 (1H, d, J = 8.6 Hz), 13.37 (1H, s).

MS (FAB) m/z: 423 (M+H)⁺.

### (Example 63) 3-Fluoro-5-{[6-(4-fluorophenoxy)-1-methyl-1H-benzimidazol-2-yl]methoxy}benzoic acid (Compound No. 1-213)

### (63a) Methyl 3-fluoro-5-{[6-(4-fluorophenoxy)-1-methyl-1H-benzimidazol-2-yl]methoxy}benzoate

The desired title compound (263 mg, yield: 56%) was obtained as a yellow powder according to the method described in Example (28d) using [6-(4-fluorophenoxy)-1-methyl-1H-benzimidazol-2-yl]methanol obtained in Example (27c) (300 mg, 1.10 mmol), methyl 5-fluoro-3-hydroxybenzoate (206 mg, 1.21 mmol), tri-n-butylphosphine (0.413 mL, 1.65 mmol) and 1,1'-(azodicarbonyl)dipiperidine (417 mg, 1.65 mmol).

¹H NMR (CDCl₃, 400 MHz) δ: 3.81 (3H, s), 3.93 (3H, s), 5.38 (2H, s), 6.92-7.09 (7H, m), 7.35-7.41 (1H, m), 7.52-7.56 (1H, m), 7.73 (1H, d, J = 9.0 Hz).

### (63b) 3-Fluoro-5-{[6-(4-fluorophenoxy)-1-methyl-1H-benzimidazol-2-yl]methoxy}benzoic acid

The desired title compound (197 mg, yield: 80%) was obtained as a white powder according to the method described in Example (28e) using methyl 3-fluoro-5-{[6-(4-fluorophenoxy)-1-methyl-1H-benzimidazol-2-yl]methoxy}benzoate obtained in Example (63a) (255 mg, 0.601 mmol) and a 1 N sodium hydroxide aqueous solution (0.900 mL, 0.900 mmol).

¹H NMR (DMSO-d₆, 400 MHz) δ: 3.81 (3H, s), 5.50 (2H, s), 6.94 (1H, dd, J = 2.3, 8.6 Hz), 7.00-7.06 (2H, m), 7.16-7.24 (2H, m), 7.28-7.32 (2H, m), 7.35 (1H, dd, J = 2.3, 10.6 Hz), 7.47-7.50 (1H, m), 7.67 (1H, d, J = 9.0 Hz), 13.37 (1H, s).

MS (FAB) m/z: 411 (M+H)⁺.

### (Example 64) 3-{[1-Methyl-(6-tetrahydro-2H-pyran-4-yloxy)-1H-benzimidazol-2-yl]methoxy}benzoic acid (Compound No. 1-208)

### (64a) tert-Butyl [5-(tetrahydro-2H-pyran-4-yloxy)-2-nitrophenyl]methylcarbamate

The title substance (1.59 g, yield: 60%) was obtained as a yellow oil by synthesis in the same manner as in Example (28a) using tetrahydro-4-pyranol (780 mg, 7.5 mmol), tert-butyl (5-chloro-2-nitrophenyl)methylcarbamate (2.15 g, 7.5 mmol), sodium hydride (> 56% in oil, 290 mg, 7.5 mmol) and N,N-dimethylformamide (30 mL) and purification using an automatic purification system (Isco, 15% ethyl acetate-hexane).

¹H-NMR (CDCl₃, 400 MHz) δ: 1. 33 (6H, s), 1. 50 (3H, s), 3.26 (3H, s), 6.81 (1H, dd, J = 2.7, 9.0 Hz), 6.85 (1H, br s), 7.07-7.17 (4H, m), 7.93-7.97 (1H, m).

### (64b) tert-Butyl [2-amino-5-(6-tetrahydro-2H-pyran-4-yloxy)phenyl]methylcarbamate

The synthesis was carried out in the same manner as in Example (28b) using tert-butyl [5-(6-tetrahydro-2H-pyran-4-yloxy)-2-nitrophenyl]methylcarbamate produced in Example (64a) (3.2 g, 8.8 mmol), iron powder (2.4 g, 12 mmol), ammonium chloride (0.24 g, 1.2 mmol), ethanol (40 mL) and water (20 mL). The resulting oil was directly used for the next reaction.

### (64c) [6-(6-Tetrahydro-2H-pyran-4-yloxy)-1-methyl-1H-benzimidazol-2-yl]methanol

The synthesis was carried out in the same manner as in Example (28c) using tert-butyl [2-amino-5-(6-tetrahydro-2H-pyran-4-yloxy)phenyl]methylcarbamate produced in Example (64b) (2.9 g, 8.8 mmol), glycolic acid (1.0 g, 13 mmol) and a 4 N hydrochloric acid-1,4-dioxane solution (40 mL). The resulting dark brown oil was directly used for the next reaction.

### (64d) Methyl 3-{[1-methyl-(6-tetrahydro-2H-pyran-4-yloxy)-1H-benzimidazol-2-yl]methoxy}benzoate

The reaction and post-treatment were carried out according to Example (28d) using [6-(6-tetrahydro-2H-pyran-4-yloxy)-1-methyl-1H-benzimidazol-2-yl]methanol produced in Example (64c) (0.30 g, 1.1 mmol), methyl 3-hydroxybenzoate (0.25 g, 1.7 mmol), tri-n-butylphosphine (0.55 mL, 2.2 mmol), 1,1'-(azodicarbonyl)dipiperidine (0.56 g, 2.2 mmol) and dichloromethane (6.0 mL) to obtain the desired compound (0.36 g, yield: 81%).

¹H-NMR (CDCl₃, 500 MHz) δ: 3.82 (3H, s), 3.92 (3H, s), 5.39 (2H, s), 6.94-7.05 (5H, m), 7.29 (1H, br s), 7.38 (1 H, t, J = 7.82 Hz), 7.69 (1H, d, J = 7.82 Hz), 7.71-7.74 (2H, m).

MS (FAB) m/z: 407 (M+H)⁺.

### (64e) 3-{[1-Methyl-(6-tetrahydro-2H-pyran-4-yloxy)-1H-benzimidazol-2-yl]methoxy}benzoic acid

The reaction and post-treatment were carried out according to Example (28e) using methyl 3-{[1-methyl-(6-tetrahydro-2H-pyran-4-yloxy)-1H-benzimidazol-2-yl]methoxy}benzoate produced in Example (64d) (0.34 g, 0.84 mmol), a 1 N sodium hydroxide aqueous solution (1.3 mL, 1.3 mmol) and 1,4-dioxane to obtain the desired compound (0.10 g, yield: 37%) as a white solid.

¹H-NMR (DMSO-d₆, 500 MHz) δ: 3.81 (3H, s), 5.46 (2H, s), 6.93 (1H, dd, J = 2.44, 8.79 Hz), 7.01-7.04 (2H, m), 7.19 (2H, t, J = 8.79 Hz), 7.30 (1H, d, J = 2.44 Hz), 7.37-7.39 (1H, m), 7.45 (1H, t, J = 7.81 Hz), 7.57 (2H, d, J = 7.81 Hz), 7.66 (1H, d, J = 8.79 Hz), 7.63 (1H, s), 13.03 (1H, br. s).

MS (FAB) m/z: 393 (M+H)⁺.

Anal. calcd for C₂₄H₂₂N₂O₅+0.14H₂O: C, 66.91; H, 4.41; N, 7.09; F, 4.81. Found C, 66.85; H, 4.46; N, 7.21; F, 4.81.

### (Example 66) 3-{[1-Methyl-(6-tetrahydrofuran-3-yloxy)-1H-benzimidazol-2-yl]methoxy}benzoic acid (Compound No. 1-209)

### (66a) tert-Butyl [5-(tetrahydrofuran-3-yloxy)-2-nitrophenyl]methylcarbamate

The synthesis was carried out in the same manner as in Example (28a) using 3-hydroxytetrahydrofuran (881 mg, 10 mmol), tert-butyl (5-chloro-2-nitrophenyl)methylcarbamate (2.87 g, 10 mmol), sodium hydride (> 56% in oil, 380 mg, 10 mmol) and N,N-dimethylformamide (40 mL). The resulting oil (2.93 g) was directly used for the next reaction.

### (66b) tert-Butyl [2-amino-5-(tetrahydrofuran-3-yloxy)phenyl]methylcarbamate

The synthesis was carried out in the same manner as in Example (28b) using tert-butyl [5-(tetrahydrofuran-3-yloxy)-2-nitrophenyl]methylcarbamate produced in Example (66a) (2.93 g, 8.7 mmol), iron powder (2.3 g, 43 mmol), ammonium chloride (0.23 g, 4.3 mmol), ethanol (40 mL) and water (20 mL). The resulting oil (2.67 g) was directly used for the next reaction.

### (66c) [6-(Tetrahydrofuran-3-yloxy)-1-methyl-1H-benzimidazol-2-yl]methanol

The synthesis was carried out in the same manner as in Example (28c) using tert-butyl [2-amino-5-(tetrahydrofuran-3-yloxy)phenyl]methylcarbamate produced in Example (66b) (2.67 g, 8.7 mmol), glycolic acid (1.0 g, 13.0 mmol), a 5 N hydrochloric acid solution (25 ml) and dioxane (25 mL). The resulting oil (1.39 g, yield: 65%) was directly used for the next reaction.

### (66d) Methyl 3-{[6-(tetrahydrofuran-3-yloxy)-1-methyl-1H-benzimidazol-2-yl]methoxy}benzoate

The reaction and post-treatment were carried out according to Example (28d) using [6-(tetrahydrofuran-3-yloxy)-1-methyl-1H-benzimidazol-2-yl]methanol produced in Example (66c) (1.39 g, 5.6 mmol), methyl 3-hydroxybenzoate (2.83 g, 11.2 mmol), tri-n-butylphosphine (2.8 mL, 11.2 mmol), 1,1'-(azodicarbonyl)dipiperidine (2.83 g, 11.2 mmol) and dichloromethane (56 mL) to obtain the desired compound (1.28 g, yield: 60%) as a colorless solid.

¹H-NMR (CDCl₃, 400 MHz) δ: 2.23-2.27 (2H, m), 3.86 (3H, s), 3.94-3.98 (1H, m), 3.95 (3H, s), 4.03-4.08 (3H, m), 5.01-5.05 (1H, m), 5.41 (2H, s), 6.80-6.82 (1H, m), 6.91-6.94 (1H, m), 7.30-7.34 (3H, m), 7.38-7.42 (1H, m), 7.68-7.72 (2H, m), 7.73-7.75 (1H, m).

MS (EI) m/z: 383 (M+H)⁺.

### (66e) 3-{[6-(Tetrahydrofuran-3-yloxy)-1-methyl-1H-benzimidazol-2-yl]methoxy}benzoic acid

The reaction and post-treatment were carried out according to Example (28e) using methyl 3-{[6-(tetrahydrofuran-3-yloxy)-1-methyl-1H-benzimidazol-2-y1]methoxy}benzoate produced in Example (66d) (1.28 g, 3.35 mmol), a 1 N sodium hydroxide aqueous solution (5.0 mL, 5.0 mmol) and 1,4-dioxane (5.0 mL) to obtain the desired compound (0.55 g, yield: 45%) as a colorless solid.

¹H-NMR (DMSO-D₆, 400 MHz) δ: 1.92-2.01 (1H, m), 2.16-2.25 (1H, m), 3.71-3.91 (4H, m), 3.78 (3H, s), 5.03-5.08 (1H, m), 5.38 (2H, s), 6.76-6.81 (1H, m), 7.07-7.10 (1H, m), 7.31-7.36 (1H, m), 7.38-7.43 (1H, m), 7.47-7.53 (2H, m), 7.57-7.60 (1H, m), 13.00 (1H, br s).

MS (FAB) m/z: 391 (M+Na)⁺.

Anal. calcd for C₂₀H₂₀N₂O₅+0.2H₂O: C, 64.58; H, 5.53; N, 7.53. Found C, 64.55; H, 5.43; N, 7.43.

### (Example 68) 3-{[5-(3-Fluoro-4-methylphenoxy)-3-methyl-3H-imidazo[4,5-b]pyridin-2-yl]methoxy}benzoic acid (Compound No. 1-227)

### (68a) 6-(3-Fluoro-4-methylphenoxy)-N-methyl-3-nitropyridin-2-amine

The desired title compound (1.94 g, yield: 70%) was obtained as a yellow powder according to the method described in Example (31a) using 6-chloro-N-methyl-3-nitropyridin-2-amine (J. Med. Chem., 43, 3052, 2000, 1.88 g, 10 mmol), 3-fluoro-4-methylphenol (1.51 g, 12 mmol) and sodium hydride (56%, 0.46 g, 12.0 mmol).

¹H-NMR (DMSO-d₆, 400 MHz) δ: 2.25 (3H, s), 2.76 (3H, d, J = 4.7 Hz), 6.30 (1H, d, J = 9.0 Hz), 7.02 (1H, dd, J = 2.4, 8.6 Hz), 7.18 (1H, dd, J = 2.0, 10.6 Hz), 7.35 (1H, t, J = 8.6 Hz), 8.44 (1H, d, J = 9.0 Hz), 8.75 (1H, br s).

### (68b) 6-(3-Fluoro-4-methylphenoxy)-N²-methylpyridine-2,3-diamine

The desired title compound (1.73 g, yield: 99%) was obtained as a brown oil according to the method described in Example (31b) using 6-(3-fluoro-4-methylphenoxy)-N-methyl-3-nitropyridin-2-amine obtained in Example (68a) (1.94 g, 7.0 mmol) and iron powder (1.95 g, 35.0 mmol).

¹H-NMR (CDCl₃, 400 MHz) δ: 2.24 (3H, s), 2.96 (3H, d, J = 4.7 Hz), 4.41 (1H, brs), 5.95 (1H, d, J = 7.8 Hz), 6.80 (1H, s), 6.82 (1H, s), 6.88 (1H, d, J = 7.8 Hz), 7.11 (1H, t, J = 8.2 Hz).

### (68c) [3-(Methoxycarbonyl)phenoxy]acetic acid

A solution of t-butyl bromoacetate (506 g, 2.6 mol), methyl 3-hydroxybenzoate (395 g, 2.60 mol) and potassium carbonate (789 g, 5.71 mol) in DMF (2 L) was stirred at room temperature for four hours. The reaction mixture was concentrated under reduced pressure and ethyl acetate (2 L) was added. The mixture was washed with water (1 L) twice, dried over anhydrous sodium sulfate and concentrated under reduced pressure to obtain crude t-butyl [3-(methoxycarbonyl)phenoxy]acetate. Anisole (100 mL) and trifluoroacetic acid (680 mL) were added to a solution of the ester in methylene chloride (1 L), and the mixture was stirred at room temperature for three days. The reaction mixture was concentrated under reduced pressure. The residue was crystallized from diisopropyl ether to obtain the title compound (476 g, 87%) as a white solid.

¹H-NMR (CDCl₃, 400 MHz) δ: 3.93 (3H, s), 4.76 (2H, s), 7.18 (1H, dd, J = 2.7, 8.2 Hz), 7.40 (1H, t, J = 8.2 Hz), 7.58 (1H, dd, J = 1.6, 2.7 Hz), 7.73 (1H, d, J = 7.4 Hz).

### (68d) Methyl 3-(2-{[6-(3-fluoro-4-methylphenoxy)-2-(methylamino)pyridin-3-yl]amino}-2-oxoethoxy)benzoate

A solution of 6-(3-fluoro-4-methylphenoxy)-N²-methylpyridine-2,3-diamine obtained in Example (68b) (1.11 g, 4.49 mmol), [3-(methoxycarbonyl)phenoxy]acetic acid obtained in Example (68c) (0.94 g, 4.49 mmol), WSC· HCl (0.86 g, 4.49 mmol) and HOBt (0.61 g, 4.49 mmol) in methylene chloride (100 mL) was stirred at room temperature for one day. The reaction mixture was concentrated under reduced pressure. Then, the residue was purified by silica gel chromatography (hexane:ethyl acetate, 1:1) to obtain the title compound (1.97 g, yield: 79%) as a brown oil.

¹H-NMR (CDCl₃, 400 MHz) δ: 2.27 (3H, d, J = 2.0 Hz), 2.86 (3H, s), 3.95 (3H, s), 4.53 (1H, br s), 4.73 (2H, s), 6.05 (1H, d, J = 8.2 Hz), 6.85-6.90 (2H, m), 7.15 (1H, t, J = 9.0 Hz), 7.21 (1H, ddd, J = 0.8, 2.7, 8.2 Hz), 7.36 (1H, d, J = 7.8 Hz), 7.46 (1H, t, J = 7.8 Hz), 7.67 (1H, dd, J = 1.1, 2.4 Hz), 7.74 (1H, s), 7.78 (1H, dt, J = 1.1, 7.8 Hz).

### (68e) Methyl 3-{[5-(3-fluoro-4-methylphenoxy)-3-methyl-3H-imidazo[4,5-b]pyridin-2-yl]methoxy}benzoate

Methyl 3-(2-{[6-(3-fluoro-4-methylphenoxy)-2-(methylamino)pyridin-3-yl]amino}-2-oxoethoxy)benzoate obtained in Example (68d) (1.56 g, 3.55 mmol) and acetic acid (20 mL) were stirred at 80°C for one day. After leaving to cool, water (100 mL) was added to the reaction mixture, followed by extraction with ethyl acetate (100 mL). Then, the organic layer was washed with saturated sodium bicarbonate (100 mL) twice and dried over anhydrous sodium sulfate. After concentration under reduced pressure, the residue was purified by silica gel chromatography (hexane:ethyl acetate, 1:1) to obtain the title compound (1.13 g, yield: 76%) as a white solid.

¹H-NMR (CDCl₃, 400 MHz): δ 2.29 (3H, d, J = 1.6 Hz), 3.84 (3H, s), 3.93 (3H, s), 5.38 (2H, s), 6.83 (1H, d, J = 8.6 Hz), 6.86-6.91 (2H, m), 7.18 (1H, t, J = 8.6 Hz), 7.26-7.29 (1H, m), 7.38 (1H, t, J = 7.8 Hz), 7.69-7.72 (2H, m), 8.03 (1H, d, J = 8.6 Hz).

### (68f) 3-{[5-(3-Fluoro-4-methylphenoxy)-3-methyl-3H-imidazo[4,5-b]pyridin-2-yl]methoxy}benzoic acid

The desired title compound (0.79 g, yield: 72%) was obtained as a white powder according to the method described in Example (33e) using methyl 3-{[5-(3-fluoro-4-methylphenoxy)-3-methyl-3H-imidazo[4,5-b]pyridin-2-yl]methoxy}benzoate obtained in Example (68e) (1.13 g, 2.68 mmol).

¹H-NMR (DMSO-d₆, 400 MHz) δ: 2.23 (3H, s), 3.70 (3H, s), 5.41 (2H, s), 6.91 (1H, d, J = 8.2 Hz), 6.91-.6.94 (1H, m), 7.05 (1H, dd, J = 2.4, 11.0 Hz), 7.15 (1H, d, J = 9.0 Hz), 7.28 (1H, d, J = 7.8 Hz), 7.32 (1H, d, J = 8.2 Hz), 7.51 (1H, d, J = 7.4 Hz), 7.57 (1H, dd, J = 1.2, 2.4 Hz), 8.14 (1H, d, J = 8.6 Hz).

MS (FAB+) m/z: 408 (M+H)⁺.

Mp: 205-207°C.

### (Example 69) 3-{[3-Methyl-5-(4-methylphenoxy)-3H-imidazo[4,5-b]pyridin-2-yl]methoxy}benzoic acid (Compound No. 1-221)

### (69a) N-Methyl-6-(4-methylphenoxy)-3-nitropyridin-2-amine

The synthesis was carried out in the same manner as in Example (28a) using 4-methylphenol (5.0 g, 46 mmol), 6-chloro-N-methyl-3-nitropyridin-2-amine (7.4 g, 39 mmol), sodium hydride (> 56% in oil, 2.0 g, 51 mmol) and N,N-dimethylformamide (50 mL). The resulting dark brown solid was directly used for the next reaction.

### (69b) 3-Amino-2-N-methylamino-6-(4-methylphenoxy)pyridine

The synthesis was carried out in the same manner as in Example (28b) using N-methyl-6-(4-methylphenoxy)-3-nitropyridin-2-amine produced in the Example (69a) (10 g, 39 mmol), iron powder (11 g, 200 mmol), ammonium chloride (1.1 g, 20 mmol), ethanol (30 mL) and water (15 mL). The resulting oil was purified by silica gel column chromatography (elution solvent: hexane/ethyl acetate = 6/1 -> 3/2). The desired compound (2.2 g, yield: 25%) was obtained as a dark brown oil by drying under reduced pressure.

¹H-NMR (CDCl₃, 400 MHz) δ: 2.33 (3H, s), 2.91 (2H, br s), 2.97 (3H, d, J = 5.1 Hz), 4.41 (1H, br. s.), 5.84 (1H, d, J = 7.8 Hz), 6.85 (1H, d, J = 7.8 Hz), 7.01 (2H, d, J = 8.6 Hz), 7.13 (2H, d, J = 8.2 Hz).

### (69c) Methyl 3-(2-{[2-(methylamino)-6-(4-methylphenoxy)pyridin-3-yl]amino}-2-oxoethoxy)benzoate

The synthesis was carried out in the same manner as in Example (28c) using 3-amino-2-N-methylamino-6-(4-methylphenoxy)pyridine (2.2 g, 9.7 mmol) produced in Example (69b), [3-(methoxycarbonyl)phenoxy]acetic acid (2.0 g, 9.7 mmol), 1-ethyl-3-(3-dimethylaminopropyl)carbodiimide hydrochloride (2.2 g, 12 mmol), 1-hydroxybenzotriazole monohydrate (1.8 g, 12 mmol) and dichloromethane (40 mL). The resulting crude product was purified by silica gel column chromatography (elution solvent: hexane/ethyl acetate = 3/1 -> 6/5). The desired compound (3.7 g, yield: 90%) was obtained as a dark green oil by drying under reduced pressure.

¹H-NMR (CDCl₃, 400 MHz) δ: 2.35 (3H, s), 2.88 (3H, s), 3.94 (3H, s), 4.72 (2H, s), 5.96 (1H, d, J = 8.2 Hz), 7.02-7.08 (2H, m), 7.16 (2H, d, J = 8.2 Hz), 7.20 (1H, ddd, J = 1.2, 2.7, 8.2 Hz), 7.32 (1H, d, J = 8.2 Hz), 7.45 (1H, t, J = 8.0 Hz), 7.66 (1H, dd, J = 1.4, 2.5 Hz), 7.73 (1H, s), 7.77 (1H, dt, J = 1.0, 1.2, 7. 6 Hz).

MS (FAB) m/z: 422 (M+H)⁺.

### (69d) Methyl 3-{[3-methyl-5-(4-methylphenoxy)-3H-imidazo[4,5-b]pyridin-2-yl]methoxy}benzoate monohydrochloride

Methyl 3-(2-{[3-methyl-5-(4-methylphenoxy)-3H-imidazo[4,5-b]pyridin-2-yl]amino}-2-oxoethoxy)benzoate produced in Example (69c) (3.7 g, 8.7 mmol) was dissolved in acetic acid (40 mL), and the solution was heated under reflux for 3.5 hours. After cooling to room temperature, a sodium bicarbonate aqueous solution (100 mL) was added to the concentrated reaction solution, followed by extraction with ethyl acetate (120 mL x 2). The resulting organic layers were washed with water (100 mL) and brine (80 mL) and then dried over anhydrous magnesium sulfate. The solvent was evaporated under reduced pressure, and the resulting pale brown oil was directly used for the next reaction.

### (69e) 3-{[3-Methyl-5-(4-methylphenoxy)-3H-imidazo[4,5-b]pyridin-2-yl]methoxy}benzoic acid

The reaction and post-treatment were carried out according to Example (28e) using methyl 3-{[3-methyl-5-(4-methylphenoxy)-3H-imidazo[4,5-b]pyridin-2-yl]methoxy}benzoate produced in Example (69d) (3.5 g, 8.3 mmol), a 1 N sodium hydroxide aqueous solution (18 mL, 18 mmol) and 1,4-dioxane (20 mL) to obtain the desired compound (2.8 g, yield: 82%) as a white solid.

¹H-NMR (DMSO-d₆, 400 MHz) δ: 2.32 (3H, s), 3.70 (3H, s), 5.47 (2H, s), 6.86 (1H, d, J = 8.6 Hz), 7.06 (2H, d, J = 8.2 Hz), 7.22 (2H, d, J = 7.8 Hz), 7.35-7.39 (1H, m), 7.45 (1H, t, J = 7.8 Hz), 7.62 (1H, dd, J = 1.4, 2.5 Hz), 7.58 (1H, dt, J = 1.3, 7.5 Hz), 8.12 (1H, d, J = 8.6 Hz), 13.05 (1H, br s).

MS (FAB) m/z: 389 (M+H)⁺.

Anal. calcd for C₂₂H₁₉N₃O₄: C, 67.86; H, 4.92; N, 10.79. Found C, 67.69; H, 4.71; N, 10.72.

### (Example 70) 3-{[5-(3,4-Dimethylphenoxy)-3-methyl-3H-imidazo[4,5-b]pyridin-2-yl]methoxy}benzoic acid (Compound No. 1-223)

### (70a) 6-(3,4-Dimethylphenoxy)-N-methyl-3-nitropyridin-2-amine

A crude product of the desired title compound was obtained as a yellow powder according to the method described in Example (28a) using 3,4-dimethylphenol (5.47 g, 44.8 mmol), 6-chloro-N-methyl-3-nitropyridin-2-amine (7.00 g, 37.3 mmol) and sodium hydride (63%, 1.71 g, 44.8 mmol). The crude product was directly used for the next reaction.

### (70b) 6-(3,4-Dimethylphenoxy)-N²-methylpyridine-2,3-diamine

The desired title compound (2.83 g, yield: 31%) was obtained as a brown oil according to the method described in Example (28b) using 6-(3,4-dimethylphenoxy)-N-methyl-3-nitropyridin-2-amine obtained in Example (70a) (10.2 g, 37.3 mmol), iron powder (10.4 g, 187 mmol) and ammonium chloride (1.00 g, 18.7 mmol).

¹H NMR (CDCl₃, 400 MHz) δ: 2.05 (3H, s), 2.23 (3H, s), 2.98 (3H, d, J = 5.1 Hz), 5.82 (1H, d, J = 7.8 Hz), 6.84 (2H, s), 6.90 (1H, d, J = 2.7 Hz), 7.07 (1H, d, J = 8.2 Hz).

### (70c) Methyl 3-(2-{[6-(3,4-dimethylphenoxy)-2-(methylamino)pyridin-3-yl]amino}-2-oxoethoxy)benzoate

The desired title compound (4.45 g, yield: 88%) was obtained as a brown powder according to the method described in Example (28c) using 6-(3,4-dimethylphenoxy)-N²-methylpyridine-2,3-diamine obtained in Example (70b) (2.83 g, 11.6 mmol), [3-(methoxycarbonyl)phenoxy]acetic acid (2.44 g, 11.6 mmol), 1-ethyl-3-(3-dimethylaminopropyl)carbodiimide hydrochloride (2.23 g, 11.6 mmol) and 1-hydroxybenzotriazole monohydrate (1.57 g, 11.6 mmol).

¹H NMR (CDCl₃, 400 MHz) δ: 2.25 (3H, s), 2.25 (3H, s), 2.89 (3H, s), 3.94 (3H, s), 4.72 (2H, s), 5.93 (1H, d, J = 8.2 Hz), 6.89 (1H, dd, J = 2.7, 8.2 Hz), 6.94 (1H, d, J = 2.3 Hz), 7.11 (1H, d, J = 7.8 Hz), 7.19 (1H, dd, J = 2.7, 8.2 Hz), 7.31 (1H, d, J = 8.2 Hz), 7.44 (1H, t, J = 8.0 Hz), 7.64-7.67 (1H, m), 7.73-7.80 (2H, m).

### (70d) Methyl 3-{[5-(3,4-dimethylphenoxy)-3-methyl-3H-imidazo[4,5-b]pyridin-2-yl]methoxy}benzoate

The desired title compound (3.21 g, yield: 75%) was obtained as a white powder according to the method described in Example (28d) using methyl 3-(2-{[6-(3,4-dimethylphenoxy)-2-(methylamino)pyridin-3-yl]amino}-2-oxoethoxy)benzoate obtained in Example (70c) (4.45 g, 10.2 mmol) and acetic acid (51.0 mL).

¹H NMR (CDCl₃, 400 MHz) δ: 2.27 (3H, s), 2.27 (3H, s), 3.85 (3H, s), 3.92 (3H, s), 5.37 (2H, s), 6.75 (1H, d, J = 8.6 Hz), 6.90 (1H, dd, J = 2.3, 7.8 Hz), 6.95 (1H, d, J = 2.3 Hz), 7.14 (1H, d, J = 8.6 Hz), 7.25-7.29 (2H, m), 7.38 (1H, t, J = 7.8 Hz), 7.67-7.73 (2H, m).

### (70e) 3-{[5-(3,4-Dimethylphenoxy)-3-methyl-3H-imidazo[4,5-b]pyridin-2-yl]methoxy}benzoic acid

The desired title compound (2.90 g, yield: 95%) was obtained as a white powder according to the method described in Example (28e) using methyl 3-{[5-(3,4-dimethylphenoxy)-3-methyl-3H-imidazo[4,5-b]pyridin-2-yl]methoxy}benzoate obtained in Example (70d) (3.16 g, 7.57 mmol) and a 1 N sodium hydroxide aqueous solution (11.4 mL, 11.4 mmol).

¹H NMR (DMSO-d₆, 400 MHz) δ: 2.22 (3H, s), 2.22 (3H, s), 3.71 (3H, s), 5.47 (2H, s), 6.82 (1H, d, J = 8.6 Hz), 6.88 (1H, dd, J = 2.7, 8.2 Hz), 6.96 (1H, d, J = 2.7 Hz), 7.16 (1H, d, J = 7.8 Hz), 7.37 (1H, ddd, J = 1.2, 2.7, 8.2 Hz), 7.45 (1H, t, J = 7.8 Hz), 7.56-7.59 (1H, m), 7.62-7.64 (1H, m), 8.10 (1H, d, J = 8.2 Hz), 13.03 (1H, s).

MS (FAB) m/z: 404 (M+H)⁺.

### (Example 71) 3-{[5-(3,5-Dimethylphenoxy)-3-methyl-3H-imidazo[4,5-b]pyridin-2-yl]methoxy}benzoic acid (Compound No. 1-225)

### (71a) 6-(3,5-Dimethylphenoxy)-N-methyl-3-nitropyridin-2-amine

The synthesis was carried out in the same manner as in Example (28a) using 3,5-dimethylphenol (5.0 g, 45 mmol), 6-chloro-N-methyl-3-nitropyridin-2-amine (7.1 g, 38 mmol), sodium hydride (> 56% in oil, 2.0 g, 49 mmol) and N,N-dimethylformamide (50 mL). The resulting yellow solid was directly used for the next reaction.

### (71b) 3-Amino-6-(3,5-dimethylphenoxy)-2-N-methylaminopyridine

6-(3,5-Dimethylphenoxy)-N-methyl-3-nitropyridin-2-amine produced in Example (71a) (10 g, 37 mmol) was dissolved in ethanol (100 mL) in a nitrogen atmosphere and 10% palladium/carbon (2.0 g) was added. The atmosphere was replaced with hydrogen and the mixture was vigorously stirred at room temperature for 1.2 hours. The atmosphere was replaced again with nitrogen and then the catalyst was filtered off through celite. The filtrate was dried under reduced pressure to obtain the desired compound as a red purple oil, which was directly used for the next reaction.

### (71c) Methyl 3-(2-{[6-(3,5-dimethylphenoxy)-2-(methylamino)pyridin-3-yl]amino}-2-oxoethoxy)benzoate

The synthesis was carried out in the same manner as in Example (57c) using 3-amino-6-(3,5-dimethylphenoxy)-2-N-methylaminopyridine (12 g, 37 mmol) produced in Example (71b), [3-(methoxycarbonyl)phenoxy]acetic acid (7.7 g, 37 mmol), 1-ethyl-3-(3-dimethylaminopropyl)carbodiimide hydrochloride (8.4 g, 44 mmol) and dichloromethane (150 mL). The resulting desired compound as a gray amorphous compound was directly used for the next reaction.

### (71d) Methyl 3-{[5-(3,5-dimethylphenoxy)-3-methyl-3H-imidazo[4,5-b]pyridin-2-yl]methoxy}benzoate

Methyl 3-(2-{[6-(3,5-dimethylphenoxy)-2-(methylamino)pyridin-3-yl]amino}-2-oxoethoxy)benzoate produced in Example (71c) (16 g, 37 mmol) was dissolved in acetic acid (150 mL), and the mixture was heated under reflux for 2.5 hours. The reaction solution was concentrated. Ethyl acetate (5 mL) and a saturated sodium bicarbonate aqueous solution were added and the mixture was ultrasonically treated. The precipitated pale brown solid was collected by filtration to obtain the desired compound (13 g, yield: 86%).

¹H-NMR (DMSO-d₆, 400 MHz) δ: 2.32 (6H, s), 3.86 (3H, s), 3.93 (3H, s), 5.38 (2H, s), 6.71-6.81 (3H, m), 6.84 (1H, s), 7.29 (1H, dd, J = 1.0, 2.5 Hz), 7.38 (1H, t, J = 7.8 Hz), 7.67-7.71 (1H, m), 7.72 (1H, dd, J = 1.4, 2.5 Hz), 7.99 (1H, d, J = 8.6 Hz).

MS (FAB) m/z: 418 (M+H)⁺.

### (71e) 3-{[5-(3,5-Dimethylphenoxy)-3-methyl-3H-imidazo[4,5-b]pyridin-2-yl]methoxy}benzoic acid

The reaction and post-treatment were carried out according to Example (28e) using methyl 3-{[5-(3,5-dimethylphenoxy)-3-methyl-3H-imidazo[4,5-b]pyridin-2-yl]methoxy}benzoate produced in Example (71d) (13 g, 32 mmol), a 1 N sodium hydroxide aqueous solution (47 mL, 47 mmol), and 1,4-dioxane (50 mL) to obtain the desired compound (11 g, yield: 85%) as a white solid.

¹H-NMR (DMSO-d₆, 400 MHz) δ: 2.27 (6H, s), 3.72 (3H, s), 5.48 (2H, s), 6.75 (2H, s), 6.84 (2H, s), 7.34-7.41 (1H, m), 7.45 (1H, t, J = 7.8 Hz), 7.58 (1H, d, J = 7.4 Hz), 7.63 (1H, s), 8.12 (1H, d, J = 8.6 Hz).

MS (FAB) m/z: 404 (M+H)⁺.

Anal. calcd for C₂₃H₂₁N₃O₄+0.33H₂O: C, 67.47; H, 5.33; N, 10.26. Found C, 67.69; H, 5.30; N, 10.28.

### (Example 72) 3-Fluoro-5-{[3-methyl-5-(4-methylphenoxy)-3H-imidazo[4,5-b]pyridin-2-yl]methoxy}benzoic acid (Compound No. 1-222)

### (72a) Methyl 3-fluoro-5-(2-{[2-(methylamino)-6-(4-methylphenoxy)pyridin-3-yl]amino}-2-oxoethoxy)benzoate

The desired title compound (2.65 g, yield: 73%) was obtained as a brown powder according to the method described in Example (68d) using 6-(4-methylphenoxy)-N²-methylpyridine-2,3-diamine obtained in Example (69b) (1.88 g, 8.22 mmol), [3-fluoro-5-(methoxycarbonyl)phenoxy]acetic acid (1.88 g, 8.22 mmol), 1-ethyl-3-(3-dimethylaminopropyl)carbodiimide hydrochloride (1.58 g, 8.22 mmol) and 1-hydroxybenzotriazole monohydrate (1.11 g, 8.22 mmol).

¹H NMR (CDCl₃, 400 MHz) δ: 2.36 (3H, s), 2.90 (3H, s), 3.94 (3H, s), 4.71 (2H, s), 5.96 (1H, d, J = 8.2 Hz), 6.93 (1H, td, J = 2.3, 9.4 Hz), 7.05 (2H, d, J = 8.6 Hz), 7.17 (2H, d, J = 8.6 Hz), 7.31 (1H, d, J = 8.2 Hz), 7.44-7.49 (2H, m), 7.71 (1H, s).

### (72b) Methyl 3-fluoro-5-{[3-methyl-5-(4-methylphenoxy)-3H-imidazo[4,5-b]pyridin-2-yl]methoxy}benzoate

The desired title compound (2.17 g, yield: 85%) was obtained as a white powder according to the method described in Example (68e) using methyl 3-fluoro-5-(2-{[2-(methylamino)-6-(4-methylphenoxy)pyridin-3-yl]amino}-2-oxoethoxy)benzoate obtained in Example (72a) (2.65 g, 6.09 mmol) and acetic acid (30.0 mL).

¹H NMR (CDCl₃, 500 MHz) δ: 2.37 (3H, s), 3.82 (3H, s), 3.92 (3H, s), 5.36 (2H, s), 6.78 (1H, d, J = 8.8 Hz), 6.98-7.03 (1H, m), 7.06 (2H, d, J = 8.3 Hz), 7.19 (2H, d, J = 8.8 Hz), 7.36-7.40 (1H, m), 7.51-7.54 (1H, m), 7.99 (1H, d, J = 8.3 Hz).

### (72c) 3-Fluoro-5-{[3-methyl-5-(4-methylphenoxy)-3H-imidazo[4,5-b]pyridin-2-yl]methoxy}benzoic acid

The desired title compound (1.52 g, yield: 72%) was obtained as a white powder according to the method described in Example (28e) using methyl 3-fluoro-5-{[3-methyl-5-(4-methylphenoxy)-3H-imidazo[4,5-b]pyridin-2-yl]methoxy}benzoate obtained in Example (72b) (2.17 g, 5.15 mmol) and a 1 N sodium hydroxide aqueous solution (7.72 mL, 7.72 mmol).

¹H NMR (DMSO-d₆, 400 MHz) δ: 2.32 (3H, s), 3.69 (3H, s), 5.50 (2H, s), 6.86 (1H, d, J = 8.6 Hz), 7.03-7.09 (2H, m), 7.20-7.25 (2H, m), 7.28-7.36 (2H, m), 7.46-7.49 (1H, m), 8.12 (1H, d, J = 8.6 Hz), 13.38 (1H, s).

MS (FAB) m/z: 408 (M+H)⁺.

### Test Example 1: Hypoglycemic effect

Six-week-old male KK mice were purchased from CLEA Japan, Inc. and then were fed until 15 to 20 weeks old to develope diabetes. The mice were individually fed during the adaptation period and the test period, and water and feed (FR2, Funabashi Farm) were freely ingested.

At the start of the experiment, after body weight measurement, blood was collected from the tail vein of the mice into a heparin-coated glass tube and centrifuged, and then plasma was separated. The glucose level in the plasma was measured by Glucoloader GXT (A&T Corp.), and individuals having a plasma glucose level of about 350 mg/dl or more were selected. The mice were grouped, each group having 3 to 4 mice, to make the average body weight and the average plasma glucose level similar. Each compound was administered to a compound group with a diet admixture containing 0.03% of the compound. A separate group in which the mice were fed only with diet was a control group.

The experiment period (drug administration period) was three days. The grouping day was the 0th day. On the 3rd day, the body weight was measured and blood was collected from the tail vein to measure the plasma glucose level.

The hypoglycemic rate was determined by the following formula.

Hypoglycemic rate = [(Control group blood glucose level - Compound-administered group blood glucose level)/Control group blood glucose level] x 100

The results obtained are shown in Table 2.

**(Table 2)**

| Example | Hypoglycemic rate (%) |
|---|---|
| 2 | 37 |
| 8 | 29 |
| 10 | 35 |
| 12 | 30 |
| 14 | 33 |
| 16 | 60 |
| 28 | 61 |
| 29 | 60 |
| 31 | 47 |
| 33 | 42 |
| 41 | 51 |
| 42 | 29 |
| 49 | 48 |
| 54 | 32 |
| 55 | 44 |
| 59 | 47 |
| | |
| 69 | 43 |
| 70 | 38 |
| 72 | 42 |

As is clear from Table 2, the compound of the present invention has an excellent hypoglycemic effect. Accordingly, the compound of the present invention is assumed to be useful as a therapeutic agent for diabetes (especially a therapeutic agent for type II diabetes).

In the following Test Examples 2 to 5, each operation was carried out according to the method described in the literature (Sambrook, J., Fritsch, E. F. and Maniatis, T., "Molecular Cloning", Cold Spring Harbor Laboratory Press, 1989) unless otherwise noted. Commercially available reagents and kits were used according to the attached instructions.

### Test Example 2: Evaluation of PPARγ modulator activity

### (Procedure 1) Chemical synthesis of DNA oligomer as polymerase chain reaction primer

Polymerase chain reaction (hereinafter "PCR") primers were designed based on the human PPARγ2 gene sequence (GenBANK accession No. D83233). The recognition sequences for restriction enzyme BglII were added to upstream and downstream regions of a gene encoding human PPARγ2 protein, in which the recognition sequences were necessary for inserting the gene into the restriction site BamHI of the expression plasmid pSG5 (Stratagene) for the gene. Two polynucleotides represented by SEQ ID NOS: 1 and 2 in the later-described Sequence Listing (hereinafter "S1" and "AS1", respectively) were used as PCR primers.

### (Procedure 2) Chemical synthesis of DNA oligomer containing PPARγ response gene sequence

Two polynucleotides represented by SEQ ID NOS: 3 and 4 in the later-described Sequence Listing (hereinafter "S2" and "AS2") were used for constructing a reporter plasmid having a PPAR response sequence to measure the ability of transcriptional activation through PPARγ. The DNA fragment to be inserted was designed based on the gene sequence in the promoter region of rat acyl-CoA oxidase (J. D. Tugwood, EMBO J, 1992, Vol. 11, No. 2, p. 433-439). The recognition sequence for restriction enzyme NheI was added to S2 and the recognition sequence for restriction enzyme XhoI was added to AS2 for insertion into the reporter plasmid pGV-P2 (Toyo Ink Mfg. Co., Ltd.).

### (Procedure 3) Construction of human PPARγ expression plasmid

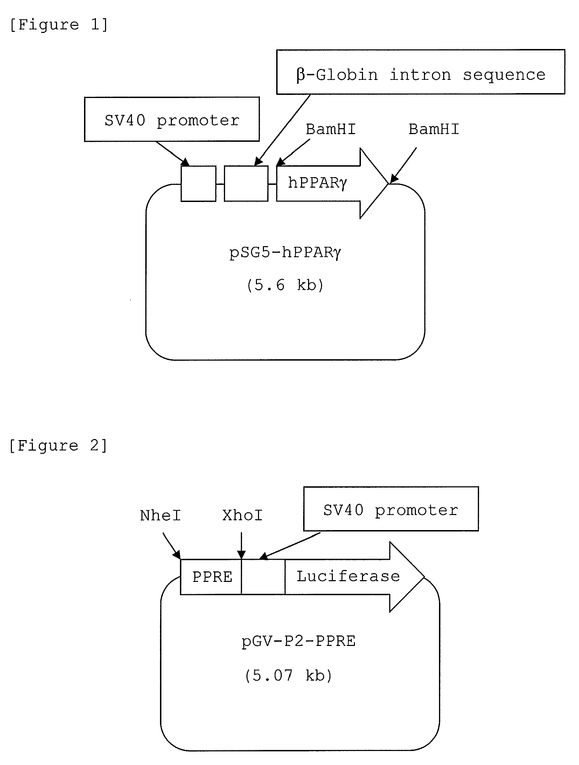

Figure 1 shows a schematic diagram of a PPARγ expression plasmid.

PCR was carried out using thermostable DNA polymerase Ex-Taq (Takara Shuzo Co., Ltd.) with a human adipose tissue-derived cDNA library (Clontech) as a template and the DNA oligomers S1 and AS1 obtained in Procedure 1 as PCR primers. As a result, a DNA fragment of about 1500 base pairs (hereinafter bp) was amplified. A cycle was repeated 30 times consisting of incubation at 94°C for one minute, incubation at 55°C for 30 seconds and incubation at 72°C for 30 seconds. The resulting DNA fragment of about 1500 bp was partially cleaved by restriction enzyme BglII and inserted into the restriction site BamHI of pSG5 to obtain a human PPARγ expression plasmid pSG5-hPPARγ. The DNA base sequence of the inserted DNA fragment was confirmed to be human PPARγ2 according to the dideoxynucleotide chain termination method.

### (Procedure 4) Construction of reporter plasmid

Figure 2 shows a schematic diagram of a PPRE reporter plasmid.

A vector pGV-P2 digest was prepared by digestion with restriction enzymes NheI and XhoI and purification by 1.0% agarose gel electrophoresis. The DNA oligomers S2 and AS2 obtained in Procedure 2 were mixed, incubated in a hot water bath at 94°C for one minute, and then incubated at 25°C for one hour to form a double-stranded DNA with S2 and AS2 annealed. Thereafter, the terminals of the double-stranded DNA were phosphorylated using DNA polynucleotide kinase (Toyobo Co., Ltd.) and then ligated to the previously prepared pGV-P2 digest using the restriction sites NheI and XhoI to obtain a reporter plasmid pGV-P2-PPRE having a PPAR response sequence.

### (Procedure 5) Gene transfer to animal cells

E. coli HB-101 was transformed by a conventional method using the plasmids obtained in Procedures 3 and 4. The HB-101 having the plasmids was cultured in L-broth medium containing 100 µg/ml ampicillin (containing 10 g of Tryptone (Difco), 5 g of yeast extract (Difco) and 5 g of sodium chloride in a 1 L solution, respectively) at 37°C for 17 hours. Thereafter, the respective plasmids were purified by the alkali-SDS method and used for gene transfer to animal cells. pSG5-hPPARγ, pGV-P2-PPRE and LipofectAMINE reagent (Invitrogen Cat. No. 18324-020) were mixed according to the manual attached to the LipofectAMINE reagent. The gene was transiently transferred to the human osteosarcoma cell line MG63, and then cells were harvested. The harvested cells were seeded into each well of a 96-well plate (COSTAR 3917) at 30000 to 40000 cells/well using α-MEM medium (GIBCO BRL Cat. No. 12571-048) mixed with 10% fetal bovine serum (MOREGATE BATCH: 474030) at 10% (v/v) and Penicillin-Streptomycin, Liquid (GIBCO BRL Cat. No. 15140-122) at 1% (v/v) (hereinafter abbreviated as 10% α-MEM). The cells were cultured in a CO₂ incubator (NAPCO) under the conditions of 37°C, 5% CO₂ and 95%-RH for 24 hours.

### (Procedure 6) Reagent addition method for evaluation of promoting effect of transcriptional activity

The medium was removed from the culture plate prepared in Procedure 5. 10% α-MEM was added to the control group at 95 µl/well. The following Compound A (Compound A is illustrated as an example and the PPARγ agonist is not limited thereto) prepared as a 10 µM solution in DMSO was diluted 1000-fold with 10% α-MEM; this was added to the positive control group at 95 µl/well. Thereafter, DMSO diluted 20-fold with 10% α-MEM was added to the control group and the positive control group at 5 µl/well. 10% α-MEM was added to the test compound-added group at 95 µl/well. Thereafter, the test compound diluted to various concentrations with DMSO was diluted 20-fold with 10% α-MEM; this was added to the test compound-added group at 5 µl/well.

### (Compound A and production process thereof)

### Compound A: N-[4-[2-[4-(2,4-dioxothiazolidin-5-ylmethyl)phenoxymethyl]-1-methyl-1H-benzimidazol-6-yloxy]phenyl]benzamide

0.36 ml of triethylamine and 0.10 ml of benzoyl chloride were added dropwise to a solution of 400 mg of 5-[4-[6-(4-aminophenoxy)-1-methyl-1H-benzimidazol-2-ylmethoxy]benzyl]thiazolidine-2,4-dione dihydrochloride in 8 ml of anhydrous N,N-dimethylformamide. The reaction solution was stirred at room temperature for one hour. Then, the solvent was evaporated under reduced pressure and water was added to the residue, followed by extraction with ethyl acetate. The organic layer was washed with brine and dried over anhydrous sodium sulfate. Ethyl acetate was evaporated. The residue was purified by silica gel column chromatography (ethyl acetate:n-hexane = 1:1 -> 2:1 -> 3:1 -> 4:1) to obtain 247 mg of the desired compound as a white powder.

Melting point: 200-204°C.

### (Procedure 7) Reagent addition method for evaluation of inhibitory effect of transcriptional activity

The medium was removed from the culture plate prepared in Procedure 5. 10% α-MEM was added to the control group at 95 µl/well. The above Compound A prepared as a 10 µM solution in DMSO was diluted 1000-fold with 10% α-MEM; this was added to the positive control group at 95 µl/well. Thereafter, DMSO was diluted 20-fold with 10% α-MEM; this was added to the control group and the positive control group at 5 µl/well. Compound A prepared as a 10 µM solution in DMSO was diluted 1000-fold with 10% α-MEM; this was added to the test compound-added group at 95 µl/well. Thereafter, the test compound diluted to various concentrations with DMSO was diluted 20-fold with 10% α-MEM; this was added to the test compound-added group at 5 µl/well.

### (Procedure 8) Method for measuring luciferase activity

The cells prepared in Procedures 6 and 7 were cultured for 24 hours and then the medium was removed. Dulbecco's phosphate-buffered saline (GIBCO BRL Cat. No. 14040-117 or SIGMA CHEMICAL CO. Cat. No. D8662) was added to an equal amount of luciferase luminescent substrate LT 2.0 (Wako Pure Chemical Industries, Ltd., Cat. No. 309-05884); this was added at 50 µl/well. The mixture was left to stand at room temperature for about 10 minutes and then stirred with a micromixer (TAITEC E-36). The luciferase activity was measured using Analyst (Molecular Devices) and a dose-dependent curve was drawn.

### (Procedure 9) Method for calculating IC₅₀ and EC₅₀

The IC₅₀, Imax, EC₅₀ and Emax of the test compound to be determined are defined as follows. Figure 3 shows a conceptual diagram.

When the luciferase activity of the positive control group is 100% and the luciferase activity of the control group is 0%, the maximum luciferase activity exhibited by the test compound alone is defined as Emax (%) and the maximum inhibition of luciferase activity by the test compound in the presence of Compound A is defined as Imax (%). Here, the concentration of the test compound representing Emax/2 is calculated as EC₅₀. The concentration of the test compound representing (100 - Imax) /2 is calculated as IC₅₀. The IC₅₀ and EC₅₀ calculated in this manner were used for evaluating the PPARγ modulator activity.

The measurement results are shown in Table 3.

**(Table 3)**

| Example | EC₅₀ (M) | Emax (%) | IC₅₀ (M) | Imax (%) |
|---|---|---|---|---|
| 2 | 3.28 × 10⁻⁹ | 70 | 5.61 × 10⁻⁸ | -44 |
| 8 | 7.72 × 10⁻⁹ | 39 | 7.33 × 10⁻⁸ | -34 |
| 10 | 2.06 × 10⁻⁹ | 42 | 3.32 × 10⁻⁸ | -50 |
| 12 | 2.34 × 10⁻⁹ | 42 | 4.04 × 10⁻⁸ | -43 |
| 14 | 5.17 × 10⁻⁸ | 54 | 6.37 × 10⁻⁸ | -35 |
| 16 | 2.64 × 10⁻⁸ | 46 | 4.02 × 10⁻⁷ | -44 |

As shown in Table 3, the compounds of the present invention have PPARγ modulator activity and are useful as therapeutic agents or prophylactic agents for a disease based on dyslipidemia, arteriosclerosis, hyperlipidemia, diabetes, involutional osteoporosis, adiposis, cancer, or the like.

### Test Example 3: Adipocyte differentiation inhibition test

Rat white adipocytes included in a white adipocyte culture kit purchased from Primary Cell Co., Ltd. were subjected to this test. A medium included in the white adipocyte culture kit purchased from Primary Cell Co., Ltd. was used as a growth medium or a differentiation-inducing medium. In this test, cells were all cultured in a CO₂ incubator (37°C, 95% humidity, 5% CO₂).

The whole of the transportation medium was extracted immediately after the arrival of the purchased cells. The growth medium was added in an amount of 5 ml(/25cm²-flask), and the cells were cultured for one day. Thereafter, a cell suspension (83,000 cells/mL) was prepared using the growth medium. The cell suspension was dispensed to a 96-well type-I collagen-coated microplate (Becton, Dickinson and Company) at 5,000 to 6,000 cells/well (60 µL/well). A well to which only the growth medium not containing cells was dispensed (blank well) was provided as a blank group in each plate.

On the following day, the whole of the growth medium was removed and the differentiation-inducing medium was added at 147 µL/well. Further, 1) for the test compound-added group, a 100 µM solution of the test compound in DMSO was diluted 20-fold with the differentiation-inducing medium; this was added at 3 µL/well (final test compound concentration: 100 nM, final DMSO concentration: 0.1% (v/v)) to the wells into which the cells were seeded, and the above Compound A was added at a final concentration of 3.3 nM to the wells (here, the final DMSO concentration was 0.01% and therefore negligible). 2) For the positive control group, DMSO diluted 20-fold with the differentiation-inducing medium was added at 3 µL/well (final DMSO concentration: 0.1% (v/v)) to the wells into which the cells were seeded, and Compound A was added at a final concentration of 3.3 nM to the wells. 3) For the negative control group, DMSO diluted 20-fold with the differentiation-inducing medium was added at 3 µL/well (final DMSO concentration: 0.1% (v/v)) to the wells into which the cells were seeded.

After culturing for five days, the whole of the differentiation-inducing medium was removed from each well, and 60 µL of a 10% (v/v) formaldehyde solution (fixative solution) was added to each well. The cells were incubated at room temperature for 20 minutes. The whole of the fixative solution was removed and 60 µL of a 0.2% (v/v) Triton X-100 solution (Sigma) was dispensed to each well. The cells were incubated at room temperature for five minutes. The whole of the Triton X-100 solution was removed. A fat stain was prepared by dissolving Oil Red O (Sigma) in a 60% (v/v) isopropanol solution at 0.3% (w/v), and 60 µL of the fat stain was dispensed to each well. The cells were incubated at room temperature for 10 minutes. The whole of the fat stain was removed, and then 60 µL of a 60% (v/v) isopropanol solution was dispensed and removed. Thus, each well was washed twice. Thereafter, DMSO was added to each well at 100 µL per well, followed by stirring at room temperature for five minutes. The absorbance at 550 nm (ABS550) was measured with a multiplate reader (Bio-Tek Instruments Inc.) or the like, and the amount of staining with Oil Red O was measured. The degree of adipocyte differentiation (%) of the test compound-added group was calculated assuming that the measured ABS550 of the positive control group was 100% and the measured ABS550 of the negative control group was 0.

The results are shown in Table 4. N.D. denotes not calculable.

**(Table 4)**

| Example | IC₅₀ (M) | Imax (%) |
|---|---|---|
| 2 | 1.2 × 10⁻⁸ | -33 |
| 8 | 5.3 × 10⁻⁸ | -29 |
| 10 | 4.5 × 10⁻⁸ | -29 |
| 12 | N.D. | N.D. |
| 14 | N.D. | N.D. |
| 16 | N.D. | N.D. |

As shown in Table 4, the compounds of the present invention inhibit differentiation into adipocytes and are useful as antiobesity agents.

### Test Example 4: Adipocyte differentiation promotion test

Rat white adipocytes included in a white adipocyte culture kit purchased from Primary Cell Co., Ltd. were subjected to this test. A medium included in the white adipocyte culture kit purchased from Primary Cell Co., Ltd. was used as a growth medium or a differentiation-inducing medium. In this test, cells were all cultured in a CO₂ incubator (37°C, 95% humidity, 5% CO₂).

The whole of the transportation medium was extracted immediately after the arrival of the purchased cells. The growth medium was added in an amount of 5 ml(/25cm²-flask), and the cells were cultured for one day. Thereafter, a cell suspension (83,000 cells/mL) was prepared using the growth medium. The cell suspension was dispensed to a 96-well type-I collagen-coated microplate (SUMITOMO BAKELITE Co., Ltd.) at 5,000 cells/well (60 µL/well). A well to which only the growth medium not containing cells was dispensed (blank well) was provided as a blank group in each plate.

On the following day, the whole of the growth medium was removed and the differentiation-inducing medium was added at 147 µL/well. Further, 1) for the test compound-added group, a 100 µM solution of the test compound in DMSO was diluted 20-fold with the differentiation-inducing medium; this was added at 3 µL/well (final test compound concentration: 100 nM, final DMSO concentration: 0.1% (v/v)) to the wells into which the cells were seeded. 2) For the positive control group, DMSO diluted 20-fold with the differentiation-inducing medium was added at 3 µL/well (final DMSO concentration: 0.1% (v/v)) to the wells into which the cells were seeded, and Compound A was added at a final concentration of 3.3 nM to the wells (here, the final DMSO concentration was 0.01% and therefore negligible). 3) For the negative control group, DMSO diluted 20-fold with the differentiation-inducing medium was added at 3 µL/well (final DMSO concentration: 0.1% (v/v)) to the wells into which the cells were seeded.

After culturing for five days, the whole of the differentiation-inducing medium was removed from each well, and 60 µL of a 10% (v/v) formaldehyde solution (fixative solution) was added to each well. The cells were incubated at room temperature for 20 minutes. The whole of the fixative solution was removed and 60 µL of a 0.2% (v/v) Triton X-100 solution (Sigma) was dispensed to each well. The cells were incubated at room temperature for five minutes. The whole of the Triton X-100 solution was removed. A fat stain was prepared by dissolving Oil Red 0 (Sigma) in a 60% (v/v) isopropanol solution at 0.3% (w/v), and 60 µL of the fat stain was dispensed to each well. The cells were incubated at room temperature for 10 minutes. The whole of the fat stain was removed, and then 60 µL of a 60% (v/v) isopropanol solution was dispensed and removed. Thus, each well was washed twice. Thereafter, DMSO was added to each well at 100 µL per well, followed by stirring at room temperature for five minutes. The absorbance at 550 nm (ABS550) was measured with a multiplate reader (Bio-Tek Instruments Inc.), and the amount of staining with Oil Red O was measured. The degree of adipocyte differentiation (%) of the test compound-added group was calculated assuming that the measured ABS550 of the positive control group was 100% and the measured ABS550 of the negative control group was 0.

The results are shown in Table 5.

**(Table 5)**

| Example | EC₅₀ (M) | Emax (%) |
|---|---|---|
| 2 | 4.2 × 10⁻⁹ | 73 |
| 8 | 7.4 × 10⁻⁹ | 75 |
| 10 | 6.6 × 10⁻⁹ | 78 |
| 12 | 5.2 × 10⁻⁹ | 82 |
| 14 | 2.0 × 10⁻⁸ | 56 |
| 16 | 6.4 × 10^{-g} | 71 |

As shown in Table 5, the compounds of the present invention are assumed to partially promote differentiation into adipocytes as a result of enhanced insulin sensitivity and are useful as antidiabetic agents.

### Test Example 5: Measurement of PPARγ activation effect/modulator activity

Rosiglitazone used in the Examples is a commercially available PPARγ activator and is a compound described in U.S. Patent No. 5,002,953, and can be produced according to the method described therein.

A test was carried out according to the reporter assay method with reference to a report by Kliewer et al. (Journal of Biological Chemistry, 1995, Vol. 270 (22), p. 12953-12956) as a method for measuring the ability of a compound to activate PPARγ (hereinafter PPARγ activation effect/modulator activity). The details will be shown below.

### (1) Preparation of GAL4-PPARγ chimeric receptor expression plasmid

The ligand-binding domain of human PPARγ (corresponding to about 300 amino acids at the carboxy end) was bound to the DNA-binding domain of the yeast transcription factor GAL4 (corresponding to 147 amino acids at the amino end) with reference to the report by Kliewer et al. to prepare a gene expressing a GAL4-PPARγ receptor.

The base sequence of the human PPARγ gene is described in the gene database GenBank under Accession No. X90563.

### (1-1) Extraction of total RNA from cell line HepG2

The cell line HepG2 (American Type Culture Collection HB-8065) was purchased from Dainippon Pharmaceutical Co., Ltd. and cultured in a tissue culture flask having a culture area of 75 cm² (manufactured by BD Biosciences). Dulbecco's modified Eagle's medium (Gibco D-MEM, manufactured by Invitrogen Corporation) supplemented with fetal bovine serum (manufactured by HyClone) at a volume ratio of 10% and an antibiotic solution [Antibiotic Antimycotic Solution, stabilized (100 x), manufactured by Sigma] at a volume ratio of 1% was used as a medium.

The cells were cultured in a carbon dioxide incubator at 37°C under 95% carbon dioxide for three days. When the cells were grown to an approximately semiconfluent state, the medium in the flask was removed by aspiration. The cells were washed by adding 10 ml of ice-cooled phosphate-buffered saline (Gibco Dulbecco's Phosphate-Buffered Saline, manufactured by Invitrogen Corporation), and then the saline was removed by aspiration. Thereafter, 7.5 ml of Trizol reagent (Gibco TRIZOL reagent, manufactured by Invitrogen Corporation) was added to the cells in the flask, and repeatedly pipetted. The cells were lysed by incubating at room temperature for about five minutes.

The cell lysate was subjected to precipitation with isopropyl alcohol according to the instructions of the Trizol reagent. The resulting RNA precipitate was dissolved in pure water and stored in a freezer at about -20°C. Here, the volume of the RNA solution was 0.22 ml. A sample obtained by diluting a part of the RNA solution 100-fold with pure water had an absorbance at 260 nm of 0.562. The yield of the total RNA was calculated to be 0.562 × 100 × 39.5 × 0.22 = 488 µg assuming that 39.5 µg/ml of RNA was present when the absorbance was 1.

### (1-2) Cloning of cDNA of PPARγ ligand-binding domain

The following two deoxyoligonucleotides (primer No. 3 and primer No. 4) designed based on the gene sequence of human PPARγ were chemically synthesized as primers for amplification by reverse transcript polymerase chain reaction (hereinafter RT-PCR) of cDNA of the PPARγ ligand-binding domain using Beckman Oligo 1000 (manufactured by Beckman).

cDNA of PPARγ was amplified by RT-PCR using Ready-To-Go RT-PCR Beads (manufactured by Amersham Pharmacia Biotech, Inc.) with the HepG2 total RNA previously obtained as a template and the primers No. 3 and No. 4 as primers. The reaction product was subjected to 1.5% agarose electrophoresis. The amplified band of about 900 base pairs was cut out, purified, and cloned to the plasmid pCRII (manufactured by Invitrogen Corporation). The amplified DNA fragment is assumed to have the nucleotide sequence represented by SEQ ID NO: 7 of the Sequence Listing which includes a sequence encoding the the ligand-binding domain, specifically, amino acids 175 to 475, of human PPARγ, and to which a restriction enzyme BamHI cleavage site and a restriction enzyme HindIII site are added on the 5'-terminal and 3'- terminal, respectively. The plasmid clone correctly containing the sequence represented by SEQ ID NO: 7 was selected by confirming the nucleotide sequence.

### (1-3) Production of plasmid pM-PPARγ

Next, the selected plasmid was treated with restriction enzymes BamHI and HindIII to obtain a 900-base-pair fragment containing the gene of the PPARγ ligand-binding domain. This was inserted into the BamHI-HindIII site of the plasmid pM having the gene of the DNA-binding domain of the yeast transcription factor GAL4 (manufactured by Clontech Laboratories, Inc.) and cloned.

The plasmid pM-PPARγ obtained by the above operation includes the nucleotide sequence represented by SEQ ID NO: 8 of the Sequence Listing and encodes an amino acid sequence represented by SEQ ID NO: 9 of the Sequence Listing containing amino acids 1 to 147 of the yeast transcription factor GAL4 at the amino end and containing amino acids 175 to 475 of human PPARγ and a stop codon at the carboxy end. The plasmid is a gene that can express a GAL4-PPARγ chimeric receptor in mammalian cells.

### (2) Measurement of PPARγ activation ability

The previously acquired plasmid pM-PPARγ and the plasmid pFR-Luc purchased from Stratagene Cloning Systems, Inc. were dissolved in deionised water at a concentration of 1 mg/mL each.

The monkey kidney-derived cell line COS-7 (American Type Culture Collection CRL-1651) was seeded into a 75 cm² culture flask and cultured using Dulbecco's modified Eagle's medium containing 10% fetal bovine serum (hereinafter medium) under the conditions of 37°C and 5% carbon dioxide gas until an approximately 80% confluent state was obtained.

COS-7 cells were transfected with 4.8 micrograms per flask of the plasmid pM-PPARγ and 19.2 µg per flask of the plasmid pFR-Luc using Lipofectamine 2000 transfection reagent (manufactured by Invitrogen Corporation), and the cells were cultured overnight.

On the following day, the cells were harvested by trypsin treatment, suspended in phenol red-free Dulbecco's modified Eagle's medium containing 75 mL of 10% fetal bovine serum, seeded into a white 96-well plate (manufactured by Costar) using the medium in a volume of 95 µL per well, and cultured overnight.

The test compound was dissolved in dimethyl sulfoxide at a concentration of 30 mM. The solution was serially diluted 6-fold with dimethyl sulfoxide to prepare solutions of the compound at concentrations up to 18 nM. Dimethyl sulfoxide was prepared for the control group. Rosiglitazone dissolved in dimethyl sulfoxide at a concentration of 30 mM was prepared for the positive control group. They were diluted 150-fold with the medium, and 5 µL of the dilution was added to the wells in which the cells were grown. The concentrations of the test compound treating the cells ranged from 10 µM to 0.006 nM. After the addition, the cells were cultured overnight.

On the following day, the medium was removed, and Luc Lite (manufactured by PerkinElmer Inc.) was prepared according to the attached document and added at 50 microliters per well. The plates with cells in the Luc Lite was stirred for about 30 minutes. The amount of luminescence in each well was measured as luciferase activity using Analyst (Molecular Devices) for 0.5 second. A dose-dependent curve was drawn.

When the luciferase activity of the positive control group was 100% and the luciferase activity of the control group was 0%, the maximum luciferase activity exhibited by the test compound alone was calculated as Emax (%) and the concentration of the test compound represented by Emax/2 was calculated as EC50.

The results obtained are shown in Table 6.

**(Table 6)**

| Example | EC₅₀ (microM) | Emax (%) |
|---|---|---|
| 2 | 0.2987 | 73.3 |
| 8 | 4.7426 | 91.0 |
| 10 | 0.0670 | 46.5 |
| 12 | 0.0397 | 60.2 |
| 14 | 0.8446 | 89.6 |
| 16 | 0.5497 | 60.9 |

As shown in Table 6, the compounds of the present invention have PPARγ activation effect/modulator activity and are useful as therapeutic agents or prophylactic agents for a disease based on dyslipidemia, arteriosclerosis, hyperlipidemia, diabetes, involutional osteoporosis, adiposis, cancer, or the like.

**Preparation Example 1: Capsules**

| | |
|---|---|
| Compound of Example 16 | 50 mg |
| Lactose | 128 mg |
| Corn starch | 70 mg |
| Magnesium stearate | 2 mg |
| | 250 mg |

The above-formulated powder is mixed and allowed to pass through a 60-mesh sieve. Then, the powder is put in 250 mg gelatin capsules No. 3 to prepare capsules.

**Preparation Example 2: Tablets**

| | |
|---|---|
| Compound of Example 16 | 50 mg |
| Lactose | 126 mg |
| Corn starch | 23 mg |
| Magnesium stearate | 1 mg |
| | 200 mg |

The above-formulated powder is mixed, wet granulated using corn starch paste, dried, and then tableted using a tableting machine to prepare tablets each having a weight of 200 mg. The tablets may be sugar-coated as necessary.

### Industrial Applicability

The fused bicyclic heteroaryl derivatives or pharmacologically acceptable salts thereof having the general formula (I) according to the present invention have excellent hypoglycemic effects and are useful as therapeutic agents and/or prophylactic agents for metabolic syndrome, specifically, a disease such as diabetes, hyperglycemia, hyperlipidemia, adiposity, impaired glucose tolerance (IGT), insulin resistance, impaired fasting glucose (IFG), hypertension, fatty liver, nonalcoholic steatohepatitis (NASH), diabetic complications (such as retinopathy, nephropathy or neuropathy), arteriosclerosis, gestational diabetes mellitus (GDM) or polycystic ovary syndrome (PCOS), inflammatory disease (such as osteoarthritis, pain or inflammatory enteritis), acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease (such as systemic lupus erythematosus, chronic rheumatism, Sjogren's syndrome, systemic sclerosis, mixed connective tissue disease, Hashimoto's disease, Crohn's disease, ulcerative colitis, idiopathic Addison's disease, male sterility, Goodpasture's syndrome, rapidly progressive glomerulonephritis, myasthenia gravis, polymyositis, multiple sclerosis, autoimmune hemolytic anemia, idiopathic thrombocytopenic purpura, Behcet's disease or CREST syndrome), pancreatitis, cachexia, cancer (such as gastric cancer, lung cancer, breast cancer, colon cancer, prostate cancer, pancreatic cancer or liver cancer), leukemia, sarcoma (such as liposarcoma), osteoporosis, involutional osteoporosis, neurodegenerative disease, Alzheimer's disease, hyperuricemia, or dry eyes.

### Sequence Listing Free Text

SEQ ID NO: 1: PCR primer S1
SEQ ID NO: 2: PCR primer AS1
SEQ ID NO: 3: PCR primer S2
SEQ ID NO: 4: PCR primer AS2
SEQ ID NO: 5: PCR sense primer
SEQ ID NO: 6: PCR antisense primer
SEQ ID NO: 7: Nucleotide sequence of synthetic human PPARγ cDNA
SEQ ID NO: 8: Nucleotide sequence of GAL4 chimeric PPARγ receptor gene
SEQ ID NO: 9: Amino acid sequence of GAL4 chimeric PPARγ receptor

Sequence Listing

## Claims

1. A compound having general formula (I): 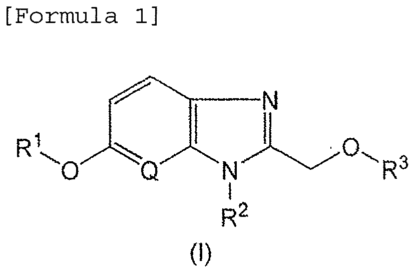 [wherein
R¹ represents a C₆-C₁₀ aryl group which may be substituted with 1 to 5 group(s) independently selected from Substituent Group a, or a heterocyclic group which may be substituted with 1 to 3 group(s) independently selected from Substituent Group a,
R² represents a C₁-C₆ alkyl group,
R³ represents a C₆-C₁₀ aryl group which may be substituted with 1 to 5 group(s) independently selected from Substituent Group a,
Q represents a group represented by the formula =CH-or a nitrogen atom, and
Substituent Group a represents a group consisting of a halogen atom, a C₁-C₆ alkyl group, a C₁-C₆ hydroxyalkyl group, a C₁-C₆ halogenated alkyl group, a carboxyl group, a carbamoyl group, a C₂-C₇ alkylcarbonyl group, a C₂-C₇ alkoxycarbonyl group, a hydroxy group, a C₁-C₆ alkoxy group, a C₁-C₆ halogenated alkoxy group, a C₂-C₇ alkylcarbonyloxy group, a C₂-C₇ alkoxycarbonyloxy group, an amino group, a C₂-C₇ alkylcarbonylamino group, a C₂-C₇ alkoxycarbonylamino group, a C₁-C₆ alkylsulfonylamino group, a 4-morpholinyl group and a di-(C₁-C₆ alkyl)amino group]
or a pharmacologically acceptable salt thereof.

2. The compound or pharmacologically acceptable salt thereof according to claim 1, wherein R¹ is a heterocyclic group and is a pyridyl group, a morpholinyl group, a tetrahydro-2H-pyran group, a tetrahydrofuranyl group, a 2,3-dihydro-1-benzofuran group or a 1,3-benzodioxole group.

3. The compound or pharmacologically acceptable salt thereof according to claim 1, wherein R¹ is a phenyl group which may be substituted with 1 to 3 group(s) independently selected from a halogen atom, a C₁-C₆ alkyl group, a C₁-C₆ alkoxy group, a C₁-C₆ halogenated alkoxy group and an amino group, or a 2,3-dihydro-1-benzofuran-6-yl group.

4. The compound or pharmacologically acceptable salt thereof according to claim 1, wherein R¹ is a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group, a 3-ethylphenyl group, a 3,4-dimethylphenyl group, a 3-trifluoromethoxyphenyl group, a 3-methoxyphenyl group, a 3-methoxy-4-methylphenyl group, a 4-amino-3,5-dimethylphenyl group or a 2,3-dihydro-1-benzofuran-6-yl group.

5. The compound or pharmacologically acceptable salt thereof according to claim 1, wherein R¹ is a 2-fluorophenyl group, a 3-fluorophenyl group, a 3-chlorophenyl group, a 2,5-difluorophenyl group, a 4-chloro-3-fluorophenyl group, a 3-chloro-4-fluorophenyl group, a 4-methylphenyl group or a 2,3-dihydro-1-benzofuran-6-yl group.

6. The compound or pharmacologically acceptable salt thereof according to any one of claims 1 to 5, wherein R² is a methyl group and Q is a group represented by the formula =CH-.

7. The compound or pharmacologically acceptable salt thereof according to any one of claims 1 to 6, wherein R³ is a phenyl group substituted with 1 to 3 fluorine atom(s) and/or carboxyl group(s).

8. The compound or pharmacologically acceptable salt thereof according to any one of claims 1 to 6, wherein R³ is a 3-carboxylphenyl group or a 3-carboxyl-5-fluorophenyl group.

9. The compound or pharmacologically acceptable salt thereof according to claim 1, wherein the compound having the general formula (I) is
3-{[6-(3-fluorophenoxy)-1-methyl-1H-benzimidazol-2-yl]methoxy}benzoic acid,
3-[6-(3-chlorophenoxy)-1-methyl-1H-benzimidazol-2-ylmethoxy]benzoic acid,
3-{[6-(4-chloro-3-fluorophenoxy)-1-methyl-1H-benzimidazol-2-yl]methoxy}benzoic acid,
3-{[6-(3-chloro-4-fluorophenoxy)-1-methyl-1H-benzimidazol-2-yl]methoxy}benzoic acid,
3-{[6-(2-fluorophenoxy)-1-methyl-1H-benzimidazol-2-yl]methoxy}benzoic acid,
3-{[1-methyl-6-(4-methylphenoxy)-1H-benzimidazol-2-yl]methoxy}benzoic acid,
3-{[6-(2,5-difluorophenoxy)-1-methyl-1H-benzimidazol-2-yl]methoxy}benzoic acid or
3-{[6-(2,3-dihydro-1-benzofuran-6-yloxy)-1-methyl-1H-benzimidazol-2-yl]methoxy}benzoic acid.

10. A pharmaceutical composition comprising the compound according to any one of claims 1 to 9 or pharmacologically acceptable salt thereof as an active ingredient.

11. The pharmaceutical composition according to claim 10 for lowering blood glucose.

12. The pharmaceutical composition according to claim 10 for the treatment and/or prevention of diabetes.

13. The pharmaceutical composition according to claim 10 for the treatment and/or prevention of type II diabetes.

14. The pharmaceutical composition according to claim 10 for activating PPARγ.

15. The pharmaceutical composition according to claim 10 for improving carbohydrate or lipid metabolism, for improving insulin resistance, for inhibiting inflammation or for inhibiting the growth of cancer cells.

16. The pharmaceutical composition according to claim 10 for the treatment and/or prevention of a disease caused by metabolic syndrome.

17. The pharmaceutical composition according to claim 10 for the treatment and/or prevention of hyperglycemia, hyperlipidemia, adiposity, impaired glucose tolerance, insulin resistance, impaired fasting glucose, hypertension, fatty liver, nonalcoholic steatohepatitis, diabetic complications, arteriosclerosis, atherosclerosis, gestational diabetes mellitus or polycystic ovary syndrome.

18. The pharmaceutical composition according to claim 10 for the treatment and/or prevention of inflammatory disease, cancer, osteoporosis, involutional osteoporosis, neurodegenerative disease, Alzheimer's disease or hyperuricemia.

19. The pharmaceutical composition according to claim 10 for the treatment and/or prevention of acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease, pancreatitis, cachexia, leukemia, sarcoma or dry eyes.

20. A PPARγ activator/modulator comprising the compound according to any one of claims 1 to 9 or pharmacologically acceptable salt thereof as an active ingredient.

21. Use of the compound according to any one of claims 1 to 9 or pharmacologically acceptable salt thereof for producing a pharmaceutical composition.

22. The use according to claim 21, wherein the pharmaceutical composition is a composition for lowering blood glucose.

23. The use according to claim 21, wherein the pharmaceutical composition is a composition for the treatment and/or prevention of diabetes.

24. The use according to claim 21, wherein the pharmaceutical composition is a composition for the treatment and/or prevention of type II diabetes.

25. The use according to claim 21, wherein the pharmaceutical composition is a composition for activating PPARγ.

26. The use according to claim 21, wherein the pharmaceutical composition is a composition for improving carbohydrate or lipid metabolism, for improving insulin resistance, for inhibiting inflammation or for inhibiting the growth of cancer cells.

27. The use according to claim 21, wherein the pharmaceutical composition is a composition for the treatment and/or prevention of a disease caused by metabolic syndrome.

28. The use according to claim 21, wherein the pharmaceutical composition is a composition for the treatment and/or prevention of hyperglycemia, hyperlipidemia, adiposity, impaired glucose tolerance, insulin resistance, impaired fasting glucose, hypertension, fatty liver, nonalcoholic steatohepatitis, diabetic complications, arteriosclerosis, atherosclerosis, gestational diabetes mellitus or polycystic ovary syndrome.

29. The use according to claim 21, wherein the pharmaceutical composition is a composition for the treatment and/or prevention of inflammatory disease, cancer, osteoporosis, involutional osteoporosis, neurodegenerative disease, Alzheimer's disease or hyperuricemia.

30. The use according to claim 21, wherein the pharmaceutical composition is a composition for the treatment and/or prevention of acne, sunburn, psoriasis, eczema, allergic disease, asthma, peptic ulcer, ulcerative colitis, Crohn's disease, coronary artery disease, arteriosclerosis, atherosclerosis, diabetic retinopathy, diabetic maculopathy, macular edema, diabetic nephropathy, ischemic heart disease, cerebrovascular disorder, peripheral circulatory disturbance, autoimmune disease, pancreatitis, cachexia, leukemia, sarcoma or dry eyes.

31. The use according to claim 21, wherein the pharmaceutical composition is a PPARγ activator/modulator.

## Patentansprüche

1. Verbindung mit der allgemeinen Formel (I): 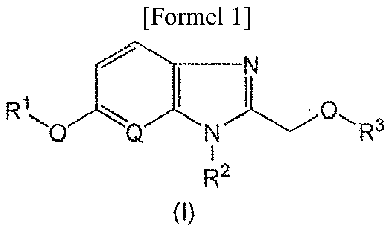 [wobei
R¹ eine C₆-C₁₀-Arylgruppe darstellt, die mit 1 bis 5 unabhängig aus Substituentengruppe a ausgewählten Gruppen substituiert sein kann, oder eine heterocyclische Gruppe, die mit 1 bis 3 unabhängig aus Substituentengruppe a ausgewählten Gruppen substituiert sein kann,
R² eine C₁-C₆-Alkylgruppe darstellt,
R³ eine C₆-C₁₀-Arylgruppe darstellt, die mit 1 bis 5 unabhängig aus Substituentengruppe a ausgewählten Gruppen substituiert sein,
Q eine Gruppe darstellt, die durch die Formel =CH- oder ein Stickstoffatom dargestellt ist, und
Substituentengruppe a eine Gruppe darstellt, die besteht aus einem Halogenatom, einer C₁-C₆-Alkylgruppe, einer C₁-C₆-Hydroxyalkylgruppe, einer halogenierten C₁-C₆-Alkylgruppe, einer Carboxylgruppe, einer Carbamoylgruppe, einer C₂-C₇-Alkylcarbonylgruppe, einer C₂-C₇-Alkoxycarbonylgruppe, einer Hydroxygruppe, einer C₁-C₆-Alkoxygruppe, einer halogenierten C₁-C₆-Alkoxygruppe, einer C₂-C₇-Alkylcarbonyloxygruppe, einer C₂-C₇-Alkoxycarbonyloxygruppe, einer Aminogruppe, einer C₂-C₇-Alkylcarbonylaminogruppe, einer C₂-C₇-Alkoxycarbonylaminogruppe, einer C₁-C₆-Alkylsulfonylaminogruppe, einer 4-Morpholinylgruppe und einer Di-(C₁-C₆-alkyl)aminogruppe]
oder ein pharmakologisch verträgliches Salz davon.

2. Verbindung oder pharmakologisch verträgliches Salz davon nach Anspruch 1, wobei R¹ eine heterocyclische Gruppe ist und eine Pyridylgruppe, eine Morpholinylgruppe, eine Tetrahydro-2H-pyrangruppe, eine Tetrahydrofuranylgruppe, eine 2,3-Dihydro-1-benzofurangruppe oder eine 1,3-Benzodioxolgruppe ist.

3. Verbindung oder pharmakologisch verträgliches Salz davon nach Anspruch 1, wobei R¹ eine Phenylgruppe, die mit 1 bis 3 Gruppen, unabhängig ausgewählt aus einem Halogenatom, einer C₁-C₆-Alkylgruppe, einer C₁-C₆-Alkoxygruppe, einer halogenierten C₁-C₆-Alkoxygruppe und einer Aminogruppe, substituiert sein kann, oder eine 2,3-Dihydro-1-benzofuran-6-yl-Gruppe ist.

4. Verbindung oder pharmakologisch verträgliches Salz davon nach Anspruch 1, wobei R¹ eine 2-Fluorphenylgruppe, eine 3-Fluorphenylgruppe, eine 3-Chlorphenylgruppe, eine 2,5-Difluorphenylgruppe, eine 4-Chlor-3-fluorphenylgruppe, eine 3-Chlor-4-fluorphenylgruppe, eine 4-Methylphenylgruppe, eine 3-Ethylphenylgruppe, eine 3,4-Dimethylphenylgruppe, eine 3-Trifluormethoxyphenylgruppe, eine 3-Methoxyphenylgruppe, eine 3-Methoxy-4-methylphenylgruppe, eine 4-Amino-3,5-dimethylphenylgruppe oder eine 2,3-Dihydro-1-benzofuran-6-yl-Gruppe ist.

5. Verbindung oder pharmakologisch verträgliches Salz davon nach Anspruch 1, wobei R¹ eine 2-Fluorphenylgruppe, eine 3-Fluorphenylgruppe, eine 3-Chlorphenylgruppe, eine 2,5-Difluorphenylgruppe, eine 4-Chlor-3-fluorphenylgruppe, eine 3-Chlor-4-fluorphenylgruppe, eine 4-Methylphenylgruppe oder eine 2,3-Dihydro-1-benzofuran-6-yl-Gruppe ist.

6. Verbindung oder pharmakologisch verträgliches Salz davon nach einem der Ansprüche 1 bis 5, wobei R² eine Methylgruppe ist und Q eine durch die Formel =CHdargestellte Gruppe ist.

7. Verbindung oder pharmakologisch verträgliches Salz davon nach einem der Ansprüche 1 bis 6, wobei R³ eine mit 1 bis 3 Fluoratomen und/oder Carboxylgruppen substituierte Phenylgruppe ist.

8. Verbindung oder pharmakologisch verträgliches Salz davon nach einem der Ansprüche 1 bis 6, wobei R³ eine 3-Carboxylphenylgruppe oder eine 3-Carboxyl-5-fluorphenylgruppe ist.

9. Verbindung oder pharmakologisch verträgliches Salz davon nach Anspruch 1, wobei die Verbindung mit der allgemeinen Formel (I) lautet
3-{[6-(3-Fluorphenoxy)-1-methyl-1H-benzimidazol-2-yl]methoxy}benzoesäure,
3-[6-(3-Chlorphenoxy)-1-methyl-1H-benzimidazol-2-ylmethoxy]benzoesäure,
3-{[6-(4-Chlor-3-fluorphenoxy)-1-methyl-1H-benzimidazol-2-yl]methoxy}benzoesäure,
3-{[6-(3-Chlor-4-fluorphenoxy)-1-methyl-1H-benzimidazol-2-yl]methoxy}benzoesäure,
3-{[6-(2-Fluorphenoxy)-1-methyl-1H-benzimidazol-2-yl]methoxy}benzoesäure,
3-{[1-Methyl-6-(4-methylphenoxy)-1H-benzimidazol-2-yl]methoxy}benzoesäure,
3-{[6-(2,5-Difluorphenoxy)-1-methyl-1H-benzimidazol-2-yl]methoxy}benzoesäure oder
3-{[6-(2,3-Dihydro-1-benzofuran-6-yloxy)-1-methyl-1H-benzimidazol-2-yl]methoxy}benzoesäure.

10. Pharmazeutische Zusammensetzung, umfassend die Verbindung nach einem der Ansprüche 1 bis 9 oder ein pharmakologisch verträgliches Salz davon als Wirkstoff.

11. Pharmazeutische Zusammensetzung nach Anspruch 10 zur Blutzuckersenkung.

12. Pharmazeutische Zusammensetzung nach Anspruch 10 für die Behandlung und/oder Verhütung von Diabetes.

13. Pharmazeutische Zusammensetzung nach Anspruch 10 für die Behandlung und/oder Verhütung von Typ-II-Diabetes.

14. Pharmazeutische Zusammensetzung nach Anspruch 10 zur Aktivierung von PPARγ.

15. Pharmazeutische Zusammensetzung nach Anspruch 10 zur Verbesserung von Kohlenhydrat- oder Lipidstoffwechsel, zur Verbesserung von Insulinresistenz, zur Entzündungshemmung oder zur Hemmung des Wachstums von Krebszellen.

16. Pharmazeutische Zusammensetzung nach Anspruch 10 für die Behandlung und/oder Verhütung einer Erkrankung, die durch das Metabolische Syndrom verursacht wird.

17. Pharmazeutische Zusammensetzung nach Anspruch 10 für die Behandlung und/oder Verhütung von Hyperglykämie, Hyperlipidämie, Adipositas, gestörter Glukosetoleranz, Insulinresistenz, abnormer Nüchternglukose, Bluthochdruck, Fettleber, nicht alkoholischer Steatohepatitis, diabetischen Komplikationen, Arteriosklerose, Atherosklerose, Schwangerschaftsdiabetes oder polyzystischem Ovarialsyndrom.

18. Pharmazeutische Zusammensetzung nach Anspruch 10 für die Behandlung und/oder Verhütung von entzündlicher Erkrankung, Krebs, Osteoporose, Involutionsosteoporose, neurodegenerativer Erkrankung, Morbus Alzheimer oder Hyperurikämie.

19. Pharmazeutische Zusammensetzung nach Anspruch 10 für die Behandlung und/oder Verhütung von Akne, Sonnenbrand, Psoriasis, Ekzem, allergischer Erkrankung, Asthma, peptischem Ulkus, Colitis ulcerosa, Morbus Crohn, koronarer Herzerkrankung, Arteriosklerose, Atherosklerose, diabetischer Retinopathie, diabetischer Makulopathie, Makulaödem, diabetischer Nephropathie, ischämischer Herzkrankheit, zerebrovaskulärer Störung, peripherer Durchblutungsstörung, Autoimmunerkrankung, Bauchspeicheldrüsenentzündung, Kachexie, Leukämie, Sarkom oder Xerophthalmie.

20. PPARγ-Aktivator/Modulator, umfassend die Verbindung nach einem der Ansprüche 1 bis 9 oder ein pharmakologisch verträgliches Salz davon als Wirkstoff.

21. Verwendung der Verbindung nach einem der Ansprüche 1 bis 9 oder eines pharmakologisch verträglichen Salzes davon zwecks Herstellung einer pharmazeutischen Zusammensetzung.

22. Verwendung nach Anspruch 21, wobei die pharmazeutische Zusammensetzung eine Zusammensetzung zur Blutzuckersenkung ist.

23. Verwendung nach Anspruch 21, wobei die pharmazeutische Zusammensetzung eine Zusammensetzung für die Behandlung und/oder Verhütung von Diabetes ist.

24. Verwendung nach Anspruch 21, wobei die pharmazeutische Zusammensetzung eine Zusammensetzung für die Behandlung und/oder Verhütung von Typ-II-Diabetes ist.

25. Verwendung nach Anspruch 21, wobei die pharmazeutische Zusammensetzung eine Zusammensetzung zur Aktivierung von PPARγ ist.

26. Verwendung nach Anspruch 21, wobei die pharmazeutische Zusammensetzung eine Zusammensetzung zur Verbesserung von Kohlenhydrat- oder Lipidstoffwechsel, zur Verbesserung von Insulinresistenz, zur Entzündungshemmung oder zur Hemmung des Wachstums von Krebszellen ist.

27. Verwendung nach Anspruch 21, wobei die pharmazeutische Zusammensetzung eine Zusammensetzung für die Behandlung und/oder Verhütung einer Erkrankung ist, die durch das Metabolische Syndrom verursacht wird.

28. Verwendung nach Anspruch 21, wobei die pharmazeutische Zusammensetzung eine Zusammensetzung ist für die Behandlung und/oder Verhütung von Hyperglykämie, Hyperlipidämie, Adipositas, gestörter Glukosetoleranz, Insulinresistenz, abnormer Nüchternglukose, Bluthochdruck, Fettleber, nicht alkoholischer Steatohepatitis, diabetischen Komplikationen, Arteriosklerose, Atherosklerose, Schwangerschaftsdiabetes oder polyzystischem Ovarialsyndrom.

29. Verwendung nach Anspruch 21, wobei die pharmazeutische Zusammensetzung eine Zusammensetzung ist für die Behandlung und/oder Verhütung von entzündlicher Erkrankung, Krebs, Osteoporose, Involutionsosteoporose, neurodegenerativer Erkrankung, Morbus Alzheimer oder Hyperurikämie.

30. Verwendung nach Anspruch 21, wobei die pharmazeutische Zusammensetzung eine Zusammensetzung ist für die Behandlung und/oder Verhütung von Akne, Sonnenbrand, Psoriasis, Ekzem, allergischer Erkrankung, Asthma, peptischem Ulkus, Colitis ulcerosa, Morbus Crohn, koronarer Herzerkrankung, Arteriosklerose, Atherosklerose, diabetischer Retinopathie, diabetischer Makulopathie, Makulaödem, diabetischer Nephropathie, ischämischer Herzkrankheit, zerebrovaskulärer Störung, peripherer Durchblutungsstörung, Autoimmunerkrankung, Bauchspeicheldrüsenentzündung, Kachexie, Leukämie, Sarkom oder Xerophthalmie.

31. Verwendung nach Anspruch 21, wobei die pharmazeutische Zusammensetzung ein PPARγ-Aktivator/Modulator ist.

## Revendications

1. Composé ayant la formule générale (I): 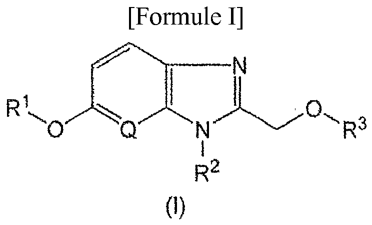 [dans laquelle
R¹ représente un groupe C₆-C₁₀ aryle qui peut être substitué par 1 à 5 groupe(s) indépendamment choisi(s) dans le Groupe substituant a, ou un groupe hétérocyclique qui peut être substitué par 1 à 3 groupe(s) indépendamment choisi(s) dans le Groupe substituant a,
R² représente un groupe C₁-C₆ alkyle,
R³ représente un groupe C₆-C₁₀ aryle qui peut être substitué par 1 à 5 groupe(s) indépendamment choisi(s) dans le Groupe substituant a,
Q représente un groupe représenté par la formule =CH- ou un atome d'azote, et
le Groupe substituant a représente un groupe consistant en un atome d'halogène, un groupe C₁-C₆ alkyle, un groupe C₁-C₆ hydroxyalkyle, un groupe C₁-C₆ alkyle halogéné, un groupe carboxyle, un groupe carbamoyle, un groupe C₂-C₇ alkylcarbonyle, un groupe C₂-C₇ alcoxycarbonyle, un groupe hydroxy, un groupe C₁-C₆ alcoxy, un groupe C₁-C₆ alcoxy halogéné, un groupe C₂-C₇ alkylcarbonyloxy, un groupe C₂-C₇ alcoxycarbonyloxy, un groupe amino, un groupe C₂-C₇ alkylcarbonylamino, un groupe C₂-C₇ alcoxycarbonylamino, un groupe C₁-C₆ alkylsulfonylamino, un groupe 4-morpholinyle et un groupe di-(C₁-C₆ alkyl)amino]
ou sel pharmacologiquement acceptable de celui-ci.

2. Composé ou sel pharmacologiquement acceptable de celui-ci selon la revendication 1, dans lequel R¹ est un groupe hétérocyclique et est un groupe pyridyle, un groupe morpholinyle, un groupe tétrahydro-2H-pyrane, un groupe tétrahydrofuranyle, un groupe 2,3-dihydro-1-benzofurane ou un groupe 1,3-benzodioxole.

3. Composé ou sel pharmacologiquement acceptable de celui-ci selon la revendication 1, dans lequel R¹ est un groupe phényle qui peut être substitué par 1 à 3 groupe(s) indépendamment choisi(s) parmi un atome d'halogène, un groupe C₁-C₆ alkyle, un groupe C₁-C₆ alcoxy, un groupe C₁-C₆ alcoxy halogéné et un groupe amino, ou un groupe 2,3-dihydro-1-benzofuran-6-yl.

4. Composé ou sel pharmacologiquement acceptable de celui-ci selon la revendication 1, dans lequel R¹ est un groupe 2-fluorophényle, un groupe 3-fluorophényle, un groupe 3-chlorophényle, un groupe 2,5-difluorophényle, un groupe 4-chloro-3-fluorophényle, un groupe 3-chloro-4-fluorophényle, un groupe 4-méthylphényle, un groupe 3-éthylphényle, un groupe 3,4-diméthylphényle, un groupe 3-trifluorométhoxyphényle, un groupe 3-méthoxyphényle, un groupe 3-méthoxy-4-méthylphényle, un groupe 4-amino-3,5-diméthylphényle ou un groupe 2,3-dihydro-1-benzofuran-6-yle.

5. Composé ou sel pharmacologiquement acceptable de celui-ci selon la revendication 1, dans lequel R¹ est un groupe 2-fluorophényle, un groupe 3-fluorophényle, un groupe 3-chlorophényle, un groupe 2,5-difluorophényle, un groupe 4-chloro-3-fluorophényle, un groupe 3-chloro-4-fluorophényle, un groupe 4-méthylphényle ou un groupe 2,3-dihydro-1-benzofuran-6-yle.

6. Composé ou sel pharmacologiquement acceptable de celui-ci selon l'une quelconque des revendications 1 à 5, dans lequel R² est un groupe méthyle et Q est un groupe représenté par la formule =CH-.

7. Composé ou sel pharmacologiquement acceptable de celui-ci selon l'une quelconque des revendications 1 à 6, dans lequel R³ est un groupe phényle substitué par 1 à 3 atome(s) de fluor et/ou groupe(s) carboxyle(s).

8. Composé ou sel pharmacologiquement acceptable de celui-ci selon l'une quelconque des revendications 1 à 6, dans lequel R³ est un groupe 3-carboxylphényle ou un groupe 3-carboxyl-5-fluorophényle.

9. Composé ou sel pharmacologiquement acceptable de celui-ci selon la revendication 1, lequel composé ayant la formule générale (I) est
acide 3-{[6-(3-fluorophénoxy)-1-méthyl-1H-benzimidazol-2-yl]méthoxy}benzoïque,
acide 3-[6-(3-chlorophénoxy)-1-méthyl-1H-benzimidazol-2-ylméthoxy]benzoïque,
acide 3-{[6-(4-chloro-3-fluorophénoxy)-1-méthyl-1H-benzimidazol-2-yl]méthoxy} benzoïque,
acide 3-{[6-(3-chloro-4-fluorophénoxy)-1-méthyl-1H-benzimidazol-2-yl]méthoxy}benzoïque,
acide 3-{[6-(2-fluorophénoxy)-1-méthyl-1H-benzimidazol-2-yl]méthoxy}benzoïque,
acide 3-{[1-méthyl-6-(4-méthylphénoxy)-1H-benzimidazol-2-yl]méthoxy}benzoïque,
acide 3-{[6-(2,5-difluorophénoxy)-1-méthyl-1H-benzimidazol-2-yl]méthoxy}benzoïque ou
acide 3-{[6-(2,3-dihydro-1-benzofuran-6-yloxy)-1-méthyl-1H-benzimidazol-2-yl]méthoxy} benzoïque.

10. Composition pharmaceutique comprenant le composé selon l'une quelconque des revendications 1 à 9 ou un sel pharmacologiquement acceptable de celui-ci en tant qu'ingrédient actif.

11. Composition pharmaceutique selon la revendication 10 pour abaisser la glycémie.

12. Composition pharmaceutique selon la revendication 10 pour le traitement et/ou la prévention d'un diabète.

13. Composition pharmaceutique selon la revendication 10 pour le traitement et/ou la prévention d'un diabète de type II.

14. Composition pharmaceutique selon la revendication 10 pour activer PPARγ.

15. Composition pharmaceutique selon la revendication 10 pour améliorer le métabolisme des glucides ou lipides, pour améliorer la résistance à l'insuline, pour inhiber une inflammation ou pour inhiber la croissance de cellules cancéreuses.

16. Composition pharmaceutique selon la revendication 10 pour le traitement et/ou la prévention d'une maladie provoquée par un syndrome métabolique.

17. Composition pharmaceutique selon la revendication 10 pour le traitement et/ou la prévention d'une hyperglycémie, d'une hyperlipidémie, d'une adiposité, d'une tolérance au glucose détériorée, d'une résistance à l'insuline, d'une glycémie à jeun détériorée, d'une hypertension, d'un foie gras, d'une stéatohépatite non alcoolique, de complications diabétiques, d'une artériosclérose, d'une athérosclérose, d'un diabète sucré gestationnel ou d'un syndrome des ovaires polykystiques.

18. Composition pharmaceutique selon la revendication 10 pour le traitement et/ou la prévention d'une maladie inflammatoire, d'un cancer, d'une ostéoporose, d'une ostéoporose involutive, d'une maladie neurodégénérative, d'une maladie d'Alzheimer ou d'une hyperuricémie.

19. Composition pharmaceutique selon la revendication 10 pour le traitement et/ou la prévention d'une acné, d'un coup de soleil, d'un psoriasis, d'un eczéma, d'une maladie allergique, d'un asthme, d'un ulcère peptique, d'une recto-colite hémorragique, d'une maladie de Crohn, d'une maladie artérielle coronaire, d'une artériosclérose, d'une athérosclérose, d'une rétinopathie diabétique, d'une maculopathie diabétique, d'un oedème maculaire, d'une néphropathie diabétique, d'une maladie cardiaque ischémique, d'un trouble cérébrovasculaire, d'un trouble circulatoire périphérique, d'une maladie auto-immune, d'une pancréatite, d'une cachexie, d'une leucémie, d'un sarcome ou d'une sécheresse oculaire.

20. Activateur/modulateur de PPARγ comprenant le composé selon l'une quelconque des revendications 1 à 9 ou un sel pharmacologiquement acceptable de celui-ci en tant qu'ingrédient actif.

21. Utilisation d'un composé selon l'une quelconque des revendications 1 à 9 ou d'un sel pharmacologiquement acceptable de celui-ci pour produire une composition pharmaceutique.

22. Utilisation selon la revendication 21, dans laquelle la composition pharmaceutique est une composition pour abaisser la glycémie.

23. Utilisation selon la revendication 21, dans laquelle la composition pharmaceutique est une composition pour le traitement et/ou la prévention d'un diabète.

24. Utilisation selon la revendication 21, dans laquelle la composition pharmaceutique est une composition pour le traitement et/ou la prévention d'un diabète de type II.

25. Utilisation selon la revendication 21, dans laquelle la composition pharmaceutique est une composition pour activer PPARγ.

26. Utilisation selon la revendication 21, dans laquelle la composition pharmaceutique est une composition pour améliorer le métabolisme des glucides ou lipides, pour améliorer la résistance à l'insuline, pour inhiber une inflammation ou pour inhiber la croissance de cellules cancéreuses.

27. Utilisation selon la revendication 21, dans laquelle la composition pharmaceutique est une composition pour le traitement et/ou la prévention d'une maladie provoquée par un syndrome métabolique.

28. Utilisation selon la revendication 21, dans laquelle la composition pharmaceutique est une composition pour le traitement et/ou la prévention d'une hyperglycémie, d'une hyperlipidémie, d'une adiposité, d'une tolérance au glucose détériorée, d'une résistance à l'insuline, d'une glycémie à jeun détériorée, d'une hypertension, d'un foie gras, d'une stéatohépatite non alcoolique, de complications diabétiques, d'une artériosclérose, d'une athérosclérose, d'un diabète sucré gestationnel ou d'un syndrome des ovaires polykystiques.

29. Utilisation selon la revendication 21, dans laquelle la composition pharmaceutique est une composition pour le traitement et/ou la prévention d'une maladie inflammatoire, d'un cancer, d'une ostéoporose, d'une ostéoporose involutive, d'une maladie neurodégénérative, d'une maladie d'Alzheimer ou d'une hyperuricémie.

30. Utilisation selon la revendication 21, dans laquelle la composition pharmaceutique est une composition pour le traitement et/ou la prévention d'une acné, d'un coup de soleil, d'un psoriasis, d'un eczéma, d'une maladie allergique, d'un asthme, d'un ulcère peptique, d'une recto-colite hémorragique, d'une maladie de Crohn, d'une maladie artérielle coronaire, d'une artériosclérose, d'une athérosclérose, d'une rétinopathie diabétique, d'une maculopathie diabétique, d'un oedème maculaire, d'une néphropathie diabétique, d'une maladie cardiaque ischémique, d'un trouble cérébrovasculaire, d'un trouble circulatoire périphérique, d'une maladie auto-immune, d'une pancréatite, d'une cachexie, d'une leucémie, d'un sarcome ou d'une sécheresse oculaire.

31. Utilisation selon la revendication 21, dans laquelle la composition pharmaceutique est un activateur/modulateur de PPARγ.
